# EUROPEAN PATENT APPLICATION

(11) **EP 3 981 436 A1**
(43) Date of publication of application: **13.04.2022**
(21) Application number: 20819505.7
(22) Date of filing: 04.06.2020
(51) Int. Cl.: A61K 48/00, A61P 43/00, C07H 21/02, A61K 31/7115, A61K 31/7125

(54) **STABLE TARGET-EDITING GUIDE RNA HAVING CHEMICALLY MODIFIED NUCLEIC ACID INTRODUCED THEREINTO**

(30) Priority: 05.06.2019 JP 2019105532; 13.08.2019 JP 2019148463
(71) Applicant: Fukuoka University, Fukuoka-shi, Fukuoka 814-0180 (JP); Daiichi Sankyo Company, Limited, Tokyo 103-8426 (JP)
(72) Inventor: FUKUDA, Masatora, Fukuoka-shi, Fukuoka 814-0180 (JP); KOIZUMI, Makoto, Tokyo 103-8426 (JP); IWASHITA, Shinzo, Tokyo 103-8426 (JP)
(74) Representative: Vossius & Partner Patentanwälte Rechtsanwälte mbB
(86) International application number: PCT/JP2020/022200
(87) International publication number: WO 2020/246560

(57) **Abstract**

An oligonucleotide that induces a site-specific editing for a target RNA, the oligonucleotide including a first oligonucleotide that specifies the target RNA, a second oligonucleotide that is linked to the 3'-side of the first oligonucleotide, a third oligonucleotide that is capable of forming a complementary pair together with the second oligonucleotide, and a first linking portion that links the second oligonucleotide and the third oligonucleotide. The first oligonucleotide is composed of a target-corresponding nucleotide residue that corresponds to an adenosine residue in the target RNA; an oligonucleotide of 10 to 30 residues linked to the 5'- side of the target-corresponding nucleotide residue and having a base sequence complementary to the target RNA; and an oligonucleotide of 3-6 residues linked to the 3'-side of the target-corresponding nucleotide residue and having a base sequence complementary to the target RNA. The second oligonucleotide consists of 5 to 8 residues and the third oligonucleotide consists of 5 to 8 residues. At least one residue selected from a counter region composed of the target-corresponding nucleotide residue and two respective residues on the 3'- and 5'-sides thereof is a nucleotide residue other than a natural ribonucleotide residue.

SEQUENCE LISTING

<110>  FUKUOKA UNIVERSITY
       DAIICHI SANKYO COMPANY, LIMITED

<120>  Stable target editing guide RNA with chemically modified nucleic
       acid

<130>  675571

<160>  44

<170>  PatentIn version 3.5

<210>  1
<211>  35
<212>  RNA
<213>  Artificial Sequence

<220>
<223>  sgRNA

<400>  1
uucagcagcu cgaaccaagg ggugguucgc caccu                                35

<210>  2
<211>  19
<212>  RNA
<213>  Artificial Sequence

<220>
<223>  ASR

<400>  2
acacgaaagc aaugccauc                                                  19

<210>  3
<211>  19
<212>  RNA
<213>  Artificial Sequence

<220>
<223>  ASR

<400>  3
   ccucuucaag ggguccaca                                                   19

<210>  4
<211>  19
<212>  RNA
<213>  Artificial Sequence

<220>
<223>  ASR

<400>  4
gccucauacu gcgugcgga                                                  19

<210>  5

<211>  17
<212>  RNA
<213>  Artificial Sequence

<220>
<223>  ASR

<400>  5
cucauacugc gugcgga                                                    17


<210>  6
<211>  31
<212>  DNA
<213>  Artificial Sequence

<220>
<223>  sgRNA

<400>  6
cgcgcgtttt cgcgcgucga accaagcggu g                                    31


<210>  7
<211>  910
<212>  RNA
<213>  Artificial Sequence

<220>
<223>  Rluc

<400>  7
accauggcuu ccaaggugua cgaccccgag caacgcaaac gcaugaucac ugggccucag      60

uggugggcuc gcugcaagca aaugaacgug cuggacuccu ucaucaacua cuaugauucc     120

gagaagcacg ccgagaacgc cgugauuuuu cugcauggua acgcugccuc cagcuaccug     180

uggaggcacg ucgugccuca caucgagccc guggcuagau gcaucauccc ugaucugauc     240

ggaaugggua aguccggcaa gagcgggaau ggcucauauc gccuccugga ucacuacaag     300

uaccucaccg cuugguucga gcugcugaac cuuccaaaga aaaucaucuu ugugggccac     360

gacugggggg cuugucuggc cuuucacuac uccuacgagc accaagacaa gaucaaggcc     420

aucguccaug cugagagugu cguggacgug aucgaguccu gggacgagug gccugacauc     480

gaggaggaua ucgcccugau caagagcgaa gagggcgaga aaauggugcu ugagaauaac     540

uucuucgucg agaccaugcu cccaagcaag aucaugcgga aacuggagcc ugaggaguuc     600

gcugccuacc uggagccauu caaggagaag ggcgagguua gacggccuac ccucuccugg     660

ccucgcgaga ucccucucgu uaagggaggc aagcccgacg ucguccagau uguccgcaac     720

uacaacgccu accuucgggc cagcgacgau cugccuaaga uguucaucga guccgacccu     780

ggguucuuuu ccaacgcuau ugucgaggga gcuaagaagu ucccuaacac cgaguucgug     840

aaggugaagg gccuccacuu cagccaggag gacgcuccag augaaauggg uaaguacauc     900

aagagcuucg                                                            910

<210>  8
<211>  42
<212>  DNA
<213>  Artificial Sequence

<220>
<223>  primer R

<400>  8
gaaggatcct tacaggtcct cctctgagat cagcttctgc tc                          42


<210>  9
<211>  33
<212>  DNA
<213>  Artificial Sequence

<220>
<223>  primer F

<400>  9
gcagaattca ccatggcttc caaggtgtac gac                                    33


<210>  10
<211>  33
<212>  DNA
<213>  Artificial Sequence

<220>
<223>  primer F

<400>  10
gacgctagca ccatggcttc caaggtgtac gac                                    33


<210>  11
<211>  21
<212>  DNA
<213>  Artificial Sequence

<220>
<223>  primer R

<400>  11
cagctcgaac taagcggtga g                                                 21


<210>  12
<211>  42
<212>  DNA
<213>  Artificial Sequence

<220>
<223>  primer R

<400>  12
gaactcgagt tacaggtcct cctctgagat cagcttctgc tc                          42


<210>  13

<211>  21
<212>  DNA
<213>  Artificial Sequence

<220>
<223>  primer F

<400>  13
ctcaccgctt agttcgagct g                                                21


<210>  14
<211>  39
<212>  DNA
<213>  Artificial Sequence

<220>
<223>  primer F

<400>  14
ctaatacgac tcactatagg gctggactcc ttcatcaac                             39


<210>  15
<211>  19
<212>  DNA
<213>  Artificial Sequence

<220>
<223>  primer R

<400>  15
ccagtcgtgg cccacaaag                                                   19


<210>  16
<211>  936
<212>  DNA
<213>  Artificial Sequence

<220>
<223>  cDNA

<400>  16
atggcttcca aggtgtacga ccccgagcaa cgcaaacgca tgatcactgg gcctcagtgg      60

tgggctcgct gcaagcaaat gaacgtgctg gactccttca tcaactacta tgattccgag     120

aggcacgccg agaacgccgt gatttttctg catggtaacg ctgcctccag ctacctgtgg     180

aggcacgtcg tgcctcacat cgagcccgtg gctagatgca tcatccctga tctgatcgga     240

atgggtaagt ccggcaagag cgggaatggc tcatatcgcc tcctggatca ctacaagtac     300

ctcaccgctt agttcgagct gctgaacctt ccaaagaaaa tcatctttgt gggccacgac     360

tgggggcctt gtctggcctt tcactactcc tacgagcacc aagacaagat caaggccatc     420

gtccatgctg agagtgtcgt ggacgtgatc gagtcctggg acgagtggcc tgacatcgag     480

gaggatatcg ccctgatcaa gagcgaagag ggcgagaaaa tggtgcttga gaataacttc     540

ttcgtcgaga ccatgctccc aagcaagatc atgcggaaac tggagcctga ggagttcgct     600

gcctacctgg agccattcaa ggagaagggc gaggttagac ggcctaccct ctcctggcct 660

cgcgagatcc ctctcgttaa gggaggcaag cccgacgtcg tccagattgt ccgcaactac 720

aacgcctacc ttcgggccag cgacgatctg cctaagatgt tcatcgagtc cgaccctggg 780

ttcttttcca acgctattgt cgagggagct aagaagttcc ctaacaccga gttcgtgaag 840

gtgaagggcc tccacttcag ccaggaggac gctccagatg aaatgggtaa gtacatcaag 900

agcttcgtgg agcgcgtgct gaagaacgag cagtaa 936

<210> 17
<211> 279
<212> RNA
<213> Artificial Sequence

<220>
<223> target sRNA

<400> 17
gggcuggacu ccuucaucaa cuacuaugau uccgagaggc acgccgagaa cgccgugauu 60

uuucugcaug guaacgcugc cuccagcuac cuguggaggc acgucgugcc ucacaucgag 120

cccguggcua gaugcaucau cccugaucug aucggaaugg guaaguccgg caagagcggg 180

aauggcucau aucgccuccu ggaucacuac aaguaccuca ccgcuuaguu cgagcugcug 240

aaccuuccaa agaaaaucau cuuugugggc cacgacugg 279

<210> 18
<211> 19
<212> DNA
<213> Artificial Sequence

<220>
<223> primer F

<400> 18
gctggactcc ttcatcaac 19

<210> 19
<211> 37
<212> DNA
<213> Artificial Sequence

<220>
<223> primer

<400> 19
ggccacgcgt cgactagtac tttttttttt ttttttt 37

<210> 20
<211> 42
<212> DNA
<213> Artificial Sequence

<220>
<223>  primer F

<400>  20
ctaatacgac tcactatagg gaccatggct tccaaggtgt ac                          42


<210>  21
<211>  20
<212>  DNA
<213>  Artificial Sequence

<220>
<223>  primer

<400>  21
ggccacgcgt cgactagtac                                                   20


<210>  22
<211>  22
<212>  DNA
<213>  Artificial Sequence

<220>
<223>  primer R

<400>  22
ttactgctcg ttcttcagca cg                                                22


<210>  23
<211>  57
<212>  DNA
<213>  Artificial Sequence

<220>
<223>  primer F

<400>  23
aattccccaa gtccacacag gggaggtgat agcattgctt tcgtgtaaat tatgtaa          57


<210>  24
<211>  57
<212>  DNA
<213>  Artificial Sequence

<220>
<223>  primer R

<400>  24
agctttacat aatttacacg aaagcaatgc tatcacctcc cctgtgtgga cttgggg          57


<210>  25
<211>  21
<212>  DNA
<213>  Artificial Sequence

<220>
<223>  primer F

<400>  25
caactgttgg gaagggcgat c                                                21


<210>  26
<211>  23
<212>  DNA
<213>  Artificial Sequence

<220>
<223>  primer R

<400>  26
cgacaggttt cccgactgga aag                                              23


<210>  27
<211>  41
<212>  DNA
<213>  Artificial Sequence

<220>
<223>  primer F

<400>  27
ctaatacgac tcactatagg gcctcttcgc tattacgcca g                          41


<210>  28
<211>  24
<212>  DNA
<213>  Artificial Sequence

<220>
<223>  primer R

<400>  28
ggaaacagct atgaccatga ttac                                             24


<210>  29
<211>  21
<212>  DNA
<213>  Artificial Sequence

<220>
<223>  primer

<400>  29
ctaatacgac tcactatagg g                                                21


<210>  30
<211>  57
<212>  DNA
<213>  Artificial Sequence

<220>
<223>  primer F

<400>  30
aattctctcc cctcctcaca gttgccatgt agaccccttg aagaggggag gggccta         57

<210>  31
<211>  57
<212>  DNA
<213>  Artificial Sequence

<220>
<223>  primer R

<400>  31
agcttaggcc cctcccctct tcaaggggtc tacatggcaa ctgtgaggag gggagag          57


<210>  32
<211>  57
<212>  DNA
<213>  Artificial Sequence

<220>
<223>  primer F

<400>  32
aattcctcac cgcagccctg aaagagatcc acacgcagta tgaggcaatg gcgtcca          57


<210>  33
<211>  57
<212>  DNA
<213>  Artificial Sequence

<220>
<223>  primer R

<400>  33
agcttggacg ccattgcctc atactgcgtg tggatctctt tcagggctgc ggtgagg          57


<210>  34
<211>  54
<212>  DNA
<213>  Artificial Sequence

<220>
<223>  primer R

<400>  34
aggtggcgaa ccaccctccg cacgcagtat gaggcctata gtgagtcgta ttag          54


<210>  35
<211>  61
<212>  DNA
<213>  Artificial Sequence

<220>
<223>  primer F

<400>  35
ctaatacgac tcactatagg cctcatactg cgtgcggagg gtggaatagt ataacaatat          60

g                                                                       61

<210>  36
<211>  49
<212>  DNA
<213>  Artificial Sequence

<220>
<223>  primer R

<400>  36
aggtgggata ctataacaac atttagcata ttgttatact attccaccc                    49


<210>  37
<211>  69
<212>  RNA
<213>  Artificial Sequence

<220>
<223>  sgRNA

<400>  37
ggccucauac ugcgugcgga ggguggaaua guauaacaau augcuaaaug uuguuauagu        60

aucccaccu                                                                69


<210>  38
<211>  36
<212>  RNA
<213>  Artificial Sequence

<220>
<223>  gRNA

<400>  38
ggccucauac ugcgugcgga gggugguucg ccaccu                                  36


<210>  39
<211>  21
<212>  RNA
<213>  Homo sapiens

<400>  39
ccucuucaag ggguccacau g                                                  21


<210>  40
<211>  23
<212>  RNA
<213>  Homo sapiens

<400>  40
   ccccucuuca aggggtccac aug                                               23


<210>  41
<211>  25
<212>  RNA
<213>  Homo sapiens

<400>  41
cuccccucuu caaggggucc acaug                                              25

<210>  42
<211>  27
<212>  RNA
<213>  Homo sapiens

<400>  42
cccuccccuc uucaaggggu ccacaug                                          27

<210>  43
<211>  19
<212>  RNA
<213>  Homo sapiens

<400>  43
ccucuucaag ggguccaca                                                   19

<210>  44
<211>  21
<212>  RNA
<213>  Homo sapiens

<400>  44
ccucuucaag ggguccacau g                                                21

## Description

### TECHNICAL FIELD

The present invention relates to a stable target-editing guide RNA with a chemically modified nucleic acid introduced thereinto.

### BACKGROUND ART

The development of genome editing techniques has triggered the use of a method of controlling biological phenomena by modifying genetic information that is a design drawing of living organisms, i.e., intracellular DNA information, in the medical and drug discovery fields as a disease treatment approach. Since DNA is an intracellularly constitutive and invariant molecule, a DNA modification effect permanently remains in an object cell or object organism. On the other hand, RNA is a nucleic acid molecule to which DNA information is copied, and, unlike DNA, is a transient genetic information molecule in which synthesis and degradation are repeated. Therefore, modification of RNA information can give the object organism a non-permanent temporary genetic information modification effect. Thus, although an RNA modification technique is a gene modification technique like DNA modification, the characteristics thereof are significantly different.

As an RNA modification technique, for example, Patent Document 1 describes an oligonucleotide construct for site-specific editing of nucleotides in a target RNA sequence, comprising: a target-directing portion containing an antisense sequence complementary to a portion of a target RNA; and a recruitment moiety capable of binding and recruiting an RNA editing entity existing in a cell and capable of editing nucleotides. For example, Patent Document 2 describes a site-specific RNA mutation introduction method in which double-stranded specific adenosine deaminase (ADAR) is allowed to act on a complex of a target RNA and a target-editing guide RNA. Non-Patent Document 1 describes introducing a modified nucleic acid into an antisense oligonucleotide that recruits ADAR.

### PRIOR ART DOCUMENT

### PATENT LITERATURE

Patent Literature 1: WO 2016/0972212
Patent Literature 2: WO 2017/010556

### NON-PATENT LITERATURE

Non-patent Literature 1: Nature Biotechnology 37,133-138 (2019)

### SUMMARY OF THE INVENTION

### PROBLEM TO BE SOLVED BY THE INVENTION

The oligonucleotide construct described in Patent Document 1 has a stem-loop structure having a specific repetitive sequence as a recruitment portion in addition to a target-directing site and requires 16 or more oligonucleotides in the recruitment portion. The target-editing guide RNA described in Patent Document 2 requires an ADAR binding region having a stem-loop structure composed of a specific sequence of 40 or 49 residues in addition to an antisense region. All oligonucleotides applied to the RNA modification technique described in Patent Document 1 or 2 essentially require an intramolecular double-stranded region having a certain length for binding to ADAR in addition to a site forming a duplex with the target RNA and tends to inevitably have a long total length as an oligonucleotide.

An object of an aspect of the present invention is to provide a target-editing guide RNA having a small number of nucleotides added to a target recognition site, having excellent stability in vivo, and capable of inducing site-specific editing in a cell.

### MEANS FOR SOLVING PROBLEM

Specific means for solving the problems are as follows, and the present invention includes the following aspects.
(1-1) An oligonucleotide, or a pharmaceutically acceptable salt thereof, comprising: a first oligonucleotide identifying a target RNA; a second oligonucleotide linked to the 3'-side of the first oligonucleotide; a third oligonucleotide capable of forming a complementary pair with the second oligonucleotide; and a first linking portion linking the second oligonucleotide and the third oligonucleotide, wherein the first oligonucleotide is composed of a target-corresponding nucleotide residue corresponding to an adenosine residue in the target RNA, an oligonucleotide of 10 to 30 residues linked to the 5'-side of the target-corresponding nucleotide residue and having a base sequence complementary to the target RNA, and an oligonucleotide of 3 to 6 residues linked to the 3'-side of the target-corresponding nucleotide residue and having a base sequence complementary to the target RNA, wherein the number of residues of the second oligonucleotide is 5 to 8, wherein the number of residues of the third oligonucleotide is 5 to 8, wherein at least one residue selected from a counter region composed of the target-corresponding nucleotide residue and two respective residues on the 3'- and 5'-sides thereof is a nucleotide residue other than a natural ribonucleotide residue, and wherein the oligonucleotide or a pharmaceutically acceptable salt thereof induces site-specific editing for the target RNA.
(1-2) The oligonucleotide, or a pharmaceutically acceptable salt thereof, according to (1-1), wherein the first linking portion contains at least one selected from the group consisting of an oligonucleotide of 4 or 5 residues and a polyalkyleneoxy group consisting of 1 to 8 alkyleneoxy units.
(1-3) The oligonucleotide, or a pharmaceutically acceptable salt thereof, according to (1-1) or (1-2), comprising a second linking portion containing an alkyleneoxy unit between the first oligonucleotide and the second oligonucleotide.
(1-4) The oligonucleotide, or a pharmaceutically acceptable salt thereof, according to any one of (1-1) to (1-3), wherein the first oligonucleotide contains a phosphorothioate bond.
(1-5) The oligonucleotide, or a pharmaceutically acceptable salt thereof, according to any one of (1-1) to (1-4), wherein the first oligonucleotide contains at least one modified nucleotide residue selected from the group consisting of a 2'-O-alkylribonucleotide residue, a 2'-deoxy-2'-fluororibonucleotide residue, a bridged nucleotide residue, and a 2'-deoxyribonucleotide residue.
(1-6) The oligonucleotide, or a pharmaceutically acceptable salt thereof, according to any one of (1-1) to (1-5), wherein the counter region contains a phosphorothioate bond.
(1-7) The oligonucleotide, or a pharmaceutically acceptable salt thereof, according to any one of (1-1) to (1-6), wherein the target-corresponding nucleotide residue contains a phosphorothioate bond.
(1-8) The oligonucleotide, or a pharmaceutically acceptable salt thereof, according to any one of (1-1) to (1-7), wherein at least one of the two respective residues on the 3'- and 5'-sides of the target-corresponding nucleotide residue is at least one modified nucleotide residue selected from the group consisting of a 2'-O-alkylribonucleotide residue and a 2'-deoxy-2'-fluororibonucleotide residue.
(1-9) The oligonucleotide, or a pharmaceutically acceptable salt thereof, according to any one of (1-1) to (1-8), wherein at least one of the second oligonucleotide and the third oligonucleotide contains at least one modified nucleotide residue selected from the group consisting of a 2'-O-alkylribonucleotide residue, a 2'-deoxy-2'-fluororibonucleotide residue, and a 2'-deoxyribonucleotide residue.
(1-10) The oligonucleotide, or a pharmaceutically acceptable salt thereof, according to any one of (1-1) to (1-9), wherein the site-specific editing is caused by an enzyme reaction by adenosine deaminase.
(1-11) A medicine containing the oligonucleotide, or a pharmaceutically acceptable salt thereof, according to any one of (1-1) to (1-10).
(1-12) A therapeutic agent for a hereditary disease containing the oligonucleotide, or a pharmaceutically acceptable salt thereof, according to any one of (1-1) to (1-10).
(1-13) A pharmaceutical composition containing the oligonucleotide, or a pharmaceutically acceptable salt thereof, according to any one of (1-1) to (1-10) as an active ingredient.
(1-14) The pharmaceutical composition according to (1-13) for prevention or treatment of a hereditary disease.
(1-15) The pharmaceutical composition according to (1-14), wherein the hereditary disease is a disease treatable by converting an adenosine residue in a target RNA into an inosine residue.
(1-16) The pharmaceutical composition according to (1-14), wherein the hereditary disease is a hereditary disease caused by a mutation of a guanosine residue to an adenosine residue in a gene.
(1-17) A use of the oligonucleotide, or a pharmaceutically acceptable salt thereof, according to any one of (1-1) to (1-10) for producing a medicine for prevention or treatment of a disease.
(1-18) A use of the oligonucleotide, or a pharmaceutically acceptable salt thereof, according to any one of (1-1) to (1-10) for use in prevention or treatment of a disease.
(2-1) An oligonucleotide, or a pharmaceutically acceptable salt thereof, comprising: a first oligonucleotide identifying a target RNA; a second oligonucleotide linked to the 3'-side of the first oligonucleotide; a third oligonucleotide capable of forming a complementary pair with the second oligonucleotide; and a first linking portion linking the second oligonucleotide and the third oligonucleotide, wherein the first oligonucleotide is composed of a target-corresponding nucleotide residue corresponding to an adenosine residue in the target RNA, an oligonucleotide of 10 to 30 residues linked to the 5'-side of the target-corresponding nucleotide residue and having a base sequence complementary to the target RNA, and an oligonucleotide of 3 to 6 residues linked to the 3'-side of the target-corresponding nucleotide residue and having a base sequence complementary to the target RNA, wherein the number of residues of the second oligonucleotide is 5 to 8, wherein the number of residues of the third oligonucleotide is 5 to 8, wherein at least one residue selected from a counter region composed of the target-corresponding nucleotide residue and two respective residues on the 3'- and 5'-sides thereof is a nucleotide residue other than a natural ribonucleotide residue, and wherein the oligonucleotide or a pharmaceutically acceptable salt thereof induces site-specific editing for the target RNA.
(2-2) The oligonucleotide, or a pharmaceutically acceptable salt thereof, according to (2-1), wherein the first linking portion contains at least one selected from the group consisting of an oligonucleotide of 4 or 5 residues and a polyalkyleneoxy group consisting of 1 to 8 alkyleneoxy units.
(2-3) The oligonucleotide, or a pharmaceutically acceptable salt thereof, according to (2-2), wherein the first linking portion contains an oligonucleotide having a base sequence selected from the group consisting of GCUAA, UUCG, UACG, UGCG, UCCG, GAAA, GUAA, GCAA, GGAA, GAGA, GUGA, GCGA, and GGGA.
(2-4) The oligonucleotide, or a pharmaceutically acceptable salt thereof, according to (2-1) or (2-3), wherein the first linking portion contains a polyethyleneoxy group consisting of 1 to 8 ethyleneoxy units.
(2-5) The oligonucleotide, or a pharmaceutically acceptable salt thereof, according to (2-4), wherein the first linking portion contains a hexaethyleneoxy group.
(2-6) The oligonucleotide, or a pharmaceutically acceptable salt thereof, according to any one of (2-1) to (2-5), comprising a second linking portion containing an alkyleneoxy unit between the first oligonucleotide and the second oligonucleotide.
(2-7) The oligonucleotide, or a pharmaceutically acceptable salt thereof, according to (2-6), wherein the second linking portion contains an alkyleneoxy group having the carbon number of 3 to 6.
(2-8) The oligonucleotide, or a pharmaceutically acceptable salt thereof, according to (2-6), wherein the second linking portion contains a hexaethyleneoxy group consisting of 6 ethyleneoxy units.
(2-9) The oligonucleotide, or a pharmaceutically acceptable salt thereof, according to any one of (2-1) to (2-8), wherein the first oligonucleotide contains a phosphorothioate bond.
(2-10) The oligonucleotide, or a pharmaceutically acceptable salt thereof, according to any one of (2-1) to (2-9), wherein the first oligonucleotide contains at least one modified nucleotide selected from the group consisting of a 2'-O-alkylribonucleotide residue, a 2'-deoxy-2'-fluororibonucleotide residue, a bridged nucleotide residue, and a 2'-deoxyribonucleotide residue.
(2-11) The oligonucleotide, or a pharmaceutically acceptable salt thereof, according to any one of (2-1) to (2-9), wherein the first oligonucleotide contains at least one modified nucleotide selected from the group consisting of a 2'-O-methylribonucleotide residue, a 2'-deoxy-2'-fluororibonucleotide residue, a bridged nucleotide residue, and a 2'-deoxyribonucleotide residue.
(2-12) The oligonucleotide, or a pharmaceutically acceptable salt thereof, according to any one of (2-1) to (2-11), wherein the counter region contains a phosphorothioate bond.
(2-13) The oligonucleotide, or a pharmaceutically acceptable salt thereof, according to any one of (2-1) to (2-12), wherein the target-corresponding nucleotide residue contains a phosphorothioate bond.
(2-14) The oligonucleotide, or a pharmaceutically acceptable salt thereof, according to any one of (2-1) to (2-13), wherein at least one of the two respective residues on the 3'- and 5'-sides of the target-corresponding nucleotide residue is at least one modified nucleotide residue selected from the group consisting of a 2'-O-alkylribonucleotide residue and a 2'-deoxy-2'-fluororibonucleotide residue.
(2-15) The oligonucleotide, or a pharmaceutically acceptable salt thereof, according to any one of (2-1) to (2-14), wherein at least one of the second oligonucleotide and the third oligonucleotide contains at least one modified nucleotide residue selected from the group consisting of a 2'-O-alkylribonucleotide residue, a 2'-deoxy-2'-fluororibonucleotide residue, and a 2'-deoxyribonucleotide residue.
(2-16) The oligonucleotide, or a pharmaceutically acceptable salt thereof, according to (2-15), wherein the second oligonucleotide and the third oligonucleotide are all composed of 2'-O-methylribonucleotide residues.
(2-17) The oligonucleotide, or a pharmaceutically acceptable salt thereof, according to (2-15) or (2-16), wherein the second oligonucleotide has a base sequence of GGGUGG and the third oligonucleotide has a base sequence of CCACCU.
(2-18) An oligonucleotide, or a pharmaceutically acceptable salt thereof, comprising:
   a first oligonucleotide identifying a target RNA;
   a second oligonucleotide linked to the 3'-side of the first oligonucleotide;
   a third oligonucleotide capable of forming a complementary pair with the second oligonucleotide; and
   a first linking portion linking the second oligonucleotide and the third oligonucleotide, wherein the first oligonucleotide is composed of a target-corresponding nucleotide residue corresponding to an adenosine residue in the target RNA, an oligonucleotide of 10 to 30 residues linked to the 5'-side of the target-corresponding nucleotide residue and having a base sequence complementary to the target RNA, and an oligonucleotide of 3 to 6 residues linked to the 3'-side of the target-corresponding nucleotide residue and having a base sequence complementary to the target RNA, wherein the number of residues of the second oligonucleotide is 5 to 8, wherein the number of residues of the third oligonucleotide is 5 to 8, wherein the second oligonucleotide and the third oligonucleotide are all composed of 2'-O-alkylribonucleotide residues, wherein at least one residue selected from a counter region composed of the target-corresponding nucleotide residue and two respective residues on the 3'- and 5'-sides thereof is a nucleotide residue other than a natural ribonucleotide residue, wherein all phosphodiester bonds are phosphorothioate bonds, and wherein the oligonucleotide or a pharmaceutically acceptable salt thereof induces site-specific editing for the target RNA.
(2-19) The oligonucleotide, or a pharmaceutically acceptable salt thereof, according to (2-18), wherein the first linking portion contains at least one selected from the group consisting of an oligonucleotide of 4 or 5 residues and a polyalkyleneoxy group consisting of 2 to 8 alkyleneoxy units.
(2-20) The oligonucleotide, or a pharmaceutically acceptable salt thereof, according to (2-19), wherein the first linking portion contains an oligonucleotide having a base sequence selected from the group consisting of GCUAA, UUCG, UACG, UGCG, UCCG, GAAA, GUAA, GCAA, GGAA, GAGA, GUGA, GCGA, and GGGA.
(2-21) The oligonucleotide, or a pharmaceutically acceptable salt thereof, according to (2-19), wherein the first linking portion contains a polyethyleneoxy group consisting of 1 to 8 ethyleneoxy units.
(2-22) The oligonucleotide, or a pharmaceutically acceptable salt thereof, according to (2-21), wherein the first linking portion contains a hexaethyleneoxy group.
(2-23) The oligonucleotide, or a pharmaceutically acceptable salt thereof, according to any one of (2-18) to (2-22), comprising a second linking portion containing an alkyleneoxy unit between the first oligonucleotide and the second oligonucleotide.
(2-24) The oligonucleotide, or a pharmaceutically acceptable salt thereof, according to (2-23), wherein the second linking portion contains an alkyleneoxy group having the carbon number of 3 to 6.
(2-25) The oligonucleotide, or a pharmaceutically acceptable salt thereof, according to (2-23), wherein the second linking portion contains a hexaethyleneoxy group.
(2-26) The oligonucleotide, or a pharmaceutically acceptable salt thereof, according to any one of (2-18) to (2-25), wherein the target-corresponding nucleotide residue is a cytidine residue, a uridine residue, an adenosine residue, or a derivative thereof.
(2-27) The oligonucleotide, or a pharmaceutically acceptable salt thereof, according to (2-26), wherein the target-corresponding nucleotide residue is a cytidine residue or a derivative thereof.
(2-28) The oligonucleotide, or a pharmaceutically acceptable salt thereof, according to any one of (2-18) to (2-27), wherein the first oligonucleotide contains at least one modified nucleotide selected from the group consisting of a 2'-O-alkylribonucleotide residue, a 2'-deoxy-2'-fluororibonucleotide residue, a bridged nucleotide residue, and a 2'-deoxyribonucleotide residue.
(2-29) The oligonucleotide, or a pharmaceutically acceptable salt thereof, according to any one of (2-18) to (2-27), wherein the first oligonucleotide contains at least one modified nucleotide selected from the group consisting of a 2'-O-methylribonucleotide residue, a 2'-deoxy-2'-fluororibonucleotide residue, a bridged nucleotide residue, and a 2'-deoxyribonucleotide residue.
(2-30) The oligonucleotide, or a pharmaceutically acceptable salt thereof, according to any one of (2-18) to (2-29), wherein at least one of the two respective residues on the 3'- and 5'-sides of the target-corresponding nucleotide residue is at least one modified nucleotide residue selected from the group consisting of a 2'-O-alkylribonucleotide residue and a 2'-deoxy-2'-fluororibonucleotide residue.
(2-31) The oligonucleotide, or a pharmaceutically acceptable salt thereof, according to any one of (2-18) to (2-30), wherein the second oligonucleotide has a base sequence of GGGUGG and the third oligonucleotide has a base sequence of CCACCU.
(2-32) The oligonucleotide, or a pharmaceutically acceptable salt thereof, according to any one of (2-1) to (2-31), wherein the site-specific editing is caused by an enzyme reaction by adenosine deaminase.
(2-33) A medicine containing the oligonucleotide, or a pharmaceutically acceptable salt thereof, according to any one of (2-1) to (2-32).
(2-34) A therapeutic agent for a hereditary disease containing the oligonucleotide, or a pharmaceutically acceptable salt thereof, according to any one of (2-1) to (2-32).
(2-35) A pharmaceutical composition containing the oligonucleotide, or a pharmaceutically acceptable salt thereof, according to any one of (2-1) to (2-32) as an active ingredient.
(2-36) A use of the oligonucleotide, or a pharmaceutically acceptable salt thereof, according to any one of (2-1) to (2-32) for producing a medicine for prevention or treatment of a disease.
(2-37) A use of the oligonucleotide, or a pharmaceutically acceptable salt thereof, according to any one of (2-1) to (2-32) for use in prevention or treatment of a disease.
(2-38) A method for prevention or treatment of a disease by administering a pharmacologically effective amount of the oligonucleotide according to any one of (2-1) to (2-32) or a pharmacologically acceptable salt thereof to a warm-blooded animal.
(2-39) The method according to (2-38), wherein the warm-blooded animal is a human.
(3-1) An oligonucleotide, or a pharmaceutically acceptable salt thereof, comprising: a first oligonucleotide identifying a target RNA; a second oligonucleotide linked to the 3'-side of the first oligonucleotide; a third oligonucleotide capable of forming a complementary pair with the second oligonucleotide; and a first linking portion linking the second oligonucleotide and the third oligonucleotide, wherein the first oligonucleotide is composed of a target-corresponding nucleotide residue corresponding to an adenosine residue in the target RNA, an oligonucleotide of 10 to 30 residues linked to the 5'-side of the target-corresponding nucleotide residue and having a base sequence complementary to the target RNA, and an oligonucleotide of 3 to 6 residues linked to the 3'-side of the target-corresponding nucleotide residue and having a base sequence complementary to the target RNA, wherein the number of residues of the second oligonucleotide is 5 to 8, wherein the number of residues of the third oligonucleotide is 5 to 8, wherein at least one residue selected from a counter region composed of the target-corresponding nucleotide residue and two respective residues on the 3'- and 5'-sides thereof is a nucleotide residue other than a natural ribonucleotide residue, and wherein the oligonucleotide or a pharmaceutically acceptable salt thereof induces site-specific editing for the target RNA.
(3-2) The oligonucleotide, or a pharmaceutically acceptable salt thereof, according to (3-1), wherein the first linking portion contains at least one selected from the group consisting of an oligonucleotide of 4 or 5 residues and a polyalkyleneoxy group consisting of 1 to 8 alkyleneoxy units.
(3-3) The oligonucleotide, or a pharmaceutically acceptable salt thereof, according to (3-1) or (3-2), comprising a second linking portion containing an alkyleneoxy unit between the first oligonucleotide and the second oligonucleotide.
(3-4) The oligonucleotide, or a pharmaceutically acceptable salt thereof, according to any one of (3-1) to (3-3), wherein the first oligonucleotide contains a phosphorothioate bond.
(3-5) The oligonucleotide, or a pharmaceutically acceptable salt thereof, according to any one of (3-1) to (3-4), wherein the first oligonucleotide contains at least one modified nucleotide residue selected from the group consisting of a 2'-O-alkylribonucleotide residue, a 2'-deoxy-2'-fluororibonucleotide residue, a bridged nucleotide residue, and a 2'-deoxyribonucleotide residue.
(3-6) The oligonucleotide, or a pharmaceutically acceptable salt thereof, according to any one of (3-1) to (3-5), wherein the counter region contains a phosphorothioate bond.
(3-7) The oligonucleotide, or a pharmaceutically acceptable salt thereof, according to any one of (3-1) to (3-6), wherein the target-corresponding nucleotide residue contains a phosphorothioate bond.
(3-8) The oligonucleotide, or a pharmaceutically acceptable salt thereof, according to any one of (3-1) to (3-7), wherein at least one of the two respective residues on the 3'- and 5'-sides of the target-corresponding nucleotide residue is at least one modified nucleotide residue selected from the group consisting of a 2'-O-alkylribonucleotide residue and a 2'-deoxy-2'-fluororibonucleotide residue.
(3-9) The oligonucleotide, or a pharmaceutically acceptable salt thereof, according to any one of (3-1) to (3-8), wherein at least one of the second oligonucleotide and the third oligonucleotide contains at least one modified nucleotide residue selected from the group consisting of a 2'-O-alkylribonucleotide residue, a 2'-deoxy-2'-fluororibonucleotide residue, and a 2'-deoxyribonucleotide residue.
(3-10) The oligonucleotide, or a pharmaceutically acceptable salt thereof, according to any one of (3-1) to (3-9), wherein the oligonucleotide linked to the 5'-side of the target-corresponding nucleotide residue has a base sequence in which 2'-deoxy-2'-fluoronucleotide residues and 2'-O-alkylribonucleotide residues are alternately linked.
(3-11) The oligonucleotide, or a pharmaceutically acceptable salt thereof, according to (3-10), wherein in the oligonucleotide linked to the 5'-side of the target-corresponding nucleotide residue, a third nucleotide residue counted in the 5'-direction from the target-corresponding nucleotide residue is a 2'-deoxy-2'-fluoronucleotide residue.
(3-12) The oligonucleotide, or a pharmaceutically acceptable salt thereof, according to any one of (3-1) to (3-9), wherein the oligonucleotide linked to the 5'-side of the target-corresponding nucleotide residue has a base sequence in which bridged nucleotide residues and 2'-O-alkylribonucleotide residues are alternately linked.
(3-13) The oligonucleotide, or a pharmaceutically acceptable salt thereof, according to (3-12), wherein in the oligonucleotide linked to the 5'-side of the target-corresponding nucleotide residue, a third nucleotide residue counted in the 5'-direction from the target-corresponding nucleotide residue is a bridged nucleotide residue.
(3-14) The oligonucleotide, or a pharmaceutically acceptable salt thereof, according to any one of (3-1) to (3-9), wherein the oligonucleotide linked to the 5'-side of the target-corresponding nucleotide residue has a base sequence in which 2'-deoxy-2'-fluoronucleotide residues and bridged nucleotide residues are alternately linked
(3-15) The oligonucleotide, or a pharmaceutically acceptable salt thereof, according to (3-14), wherein in the oligonucleotide linked to the 5'-side of the target-corresponding nucleotide residue, a third nucleotide residue counted in the 5'-direction from the target-corresponding nucleotide is a 2'-deoxy-2'-fluoronucleotide residue.
(3-16) The oligonucleotide, or a pharmaceutically acceptable salt thereof, according to any one of (3-1) to (3-15), wherein in the first oligonucleotide, the oligonucleotide linked to the 3'-side of the target-corresponding nucleotide residue has a base sequence in which the 2'-O-alkylribonucleotide residues are linked.
(3-17) The oligonucleotide, or a pharmaceutically acceptable salt thereof, according to any one of (3-1) to (3-15), wherein in the first oligonucleotide, the oligonucleotide linked to the 3'-side of the target-corresponding nucleotide residue has a base sequence in which 2'-O-alkylribonucleotide residues and bridged nucleotide residues are alternately linked.
(3-18) The oligonucleotide, or a pharmaceutically acceptable salt thereof, according to any one of (3-1) to (3-17), wherein in the first oligonucleotide, the oligonucleotide linked to the 3'-side of the target-corresponding nucleotide residue consists of 4 to 6 residues.
(3-19) The oligonucleotide, or a pharmaceutically acceptable salt thereof, according to any one of (3-1) to (3-18), wherein the second oligonucleotide and the third oligonucleotide have a base sequence in which 2'-O-alkylribonucleotide residues are linked.
(3-20) The oligonucleotide, or a pharmaceutically acceptable salt thereof, according to any one of (3-1) to (3-19), wherein each of the first oligonucleotide, the second oligonucleotide, and the third oligonucleotide has nucleotide residues linked by a phosphorothioate bond.
(3-21) The oligonucleotide, or a pharmaceutically acceptable salt thereof, according to any one of (3-1) to (3-20), wherein the site-specific editing is caused by an enzyme reaction by adenosine deaminase.
(3-22) A medicine containing the oligonucleotide, or a pharmaceutically acceptable salt thereof, according to any one of (3-1) to (3-21).
(3-23) A therapeutic agent for a hereditary disease containing the oligonucleotide, or a pharmaceutically acceptable salt thereof, according to any one of (3-1) to (3-21).
(3-24) A pharmaceutical composition containing the oligonucleotide, or a pharmaceutically acceptable salt thereof, according to any one of (3-1) to (3-21) as an active ingredient.
(3-25) The pharmaceutical composition according to (3-24) for prevention or treatment of a hereditary disease.
(3-26) The pharmaceutical composition according to (3-25), wherein the hereditary disease is a disease treatable by converting an adenosine residue in a target RNA into an inosine residue.
(3-27) The pharmaceutical composition according to (3-25), wherein the hereditary disease is a hereditary disease caused by a mutation of a guanosine residue to an adenosine residue in a gene.
(3-28) A use of the oligonucleotide, or a pharmaceutically acceptable salt thereof, according to any one of (3-1) to (3-21) for producing a medicine for prevention or treatment of a disease.
(3-29) A use of the oligonucleotide, or a pharmaceutically acceptable salt thereof, according to any one of (3-1) to (3-21) for use in prevention or treatment of a disease.
(3-30) A method for prevention or treatment of a disease by administering a pharmacologically effective amount of the oligonucleotide according to any one of (3-1) to (3-29) or a pharmacologically acceptable salt thereof to a warm-blooded animal.
(3-31) The method according to (3-30), wherein the disease is a hereditary disease.
(3-32) The method according to (3-31), wherein the hereditary disease is a disease treatable by converting an adenosine residue in a target RNA into an inosine residue.
(3-33) The method according to (3-31), wherein the hereditary disease is a hereditary disease caused by a mutation of a guanosine residue to an adenosine residue in a gene.
(3-34) The method according to any one of (3-30) to (3-33), wherein the warm-blooded animal is a human.
(4-1) An oligonucleotide, or a pharmaceutically acceptable salt thereof, comprising: a first oligonucleotide identifying a target RNA; a second oligonucleotide linked to the 3'-side of the first oligonucleotide; a third oligonucleotide capable of forming a complementary pair with the second oligonucleotide; and a first linking portion linking the second oligonucleotide and the third oligonucleotide, wherein the first oligonucleotide is composed of a target-corresponding nucleotide residue corresponding to an adenosine residue in the target RNA, an oligonucleotide of 10 to 30 residues linked to the 5'-side of the target-corresponding nucleotide residue and having a base sequence complementary to the target RNA, and an oligonucleotide of 3 to 6 residues linked to the 3'-side of the target-corresponding nucleotide residue and having a base sequence complementary to the target RNA, wherein the number of residues of the second oligonucleotide is 5 to 8, wherein the number of residues of the third oligonucleotide is 5 to 8, wherein at least one residue selected from a counter region composed of the target-corresponding nucleotide residue and two respective residues on the 3'- and 5'-sides thereof is a nucleotide residue other than a natural ribonucleotide residue, and wherein the oligonucleotide or a pharmaceutically acceptable salt thereof induces site-specific editing for the target RNA.
(4-2) The oligonucleotide, or a pharmaceutically acceptable salt thereof, according to (4-1), wherein the first linking portion contains at least one selected from the group consisting of an oligonucleotide of 4 or 5 residues and a polyalkyleneoxy group consisting of 1 to 8 alkyleneoxy units.
(4-3) The oligonucleotide, or a pharmaceutically acceptable salt thereof, according to (4-2), wherein the first linking portion contains an oligonucleotide having a base sequence selected from the group consisting of GCUAA, UUCG, UACG, UGCG, UCCG, GAAA, GUAA, GCAA, GGAA, GAGA, GUGA, GCGA, and GGGA.
(4-4) The oligonucleotide, or a pharmaceutically acceptable salt thereof, according to (4-1) or (4-3), wherein the first linking portion contains a polyethyleneoxy group consisting of 1 to 8 ethyleneoxy units.
(4-5) The oligonucleotide, or a pharmaceutically acceptable salt thereof, according to (4-4), wherein the first linking portion contains a hexaethyleneoxy group.
(4-6) The oligonucleotide, or a pharmaceutically acceptable salt thereof, according to any one of (4-1) to (4-5), comprising a second linking portion containing an alkyleneoxy unit between the first oligonucleotide and the second oligonucleotide.
(4-7) The oligonucleotide, or a pharmaceutically acceptable salt thereof, according to (4-6), wherein the second linking portion contains an alkyleneoxy group having the carbon number of 3 to 6.
(4-8) The oligonucleotide, or a pharmaceutically acceptable salt thereof, according to (4-6), wherein the second linking portion contains a hexaethyleneoxy group consisting of 6 ethyleneoxy units.
(4-9) The oligonucleotide, or a pharmaceutically acceptable salt thereof, according to any one of (4-1) to (4-8), wherein the first oligonucleotide contains a phosphorothioate bond.
(4-10) The oligonucleotide, or a pharmaceutically acceptable salt thereof, according to any one of (4-1) to (4-9), wherein the first oligonucleotide contains at least one modified nucleotide selected from the group consisting of a 2'-O-alkylribonucleotide residue, a 2'-deoxy-2'-fluororibonucleotide residue, a bridged nucleotide residue, and a 2'-deoxyribonucleotide residue.
(4-11) The oligonucleotide, or a pharmaceutically acceptable salt thereof, according to any one of (4-1) to (4-9), wherein the first oligonucleotide contains at least one modified nucleotide selected from the group consisting of a 2'-O-methylribonucleotide residue, a 2'-deoxy-2'-fluororibonucleotide residue, a bridged nucleotide residue, and a 2'-deoxyribonucleotide residue.
(4-12) The oligonucleotide, or a pharmaceutically acceptable salt thereof, according to any one of (4-1) to (4-11), wherein the counter region contains a phosphorothioate bond.
(4-13) The oligonucleotide, or a pharmaceutically acceptable salt thereof, according to any one of (4-1) to (4-12), wherein the target-corresponding nucleotide residue contains a phosphorothioate bond.
(4-14) The oligonucleotide, or a pharmaceutically acceptable salt thereof, according to any one of (4-1) to (4-13), wherein at least one of the two respective residues on the 3'- and 5'-sides of the target-corresponding nucleotide residue is at least one modified nucleotide residue selected from the group consisting of a 2'-O-alkylribonucleotide residue and a 2'-deoxy-2'-fluororibonucleotide residue.
(4-15) The oligonucleotide, or a pharmaceutically acceptable salt thereof, according to any one of (4-1) to (4-14), wherein at least one of the second oligonucleotide and the third oligonucleotide contains at least one modified nucleotide residue selected from the group consisting of a 2'-O-alkylribonucleotide residue, a 2'-deoxy-2'-fluororibonucleotide residue, and a 2'-deoxyribonucleotide residue.
(4-16) The oligonucleotide, or a pharmaceutically acceptable salt thereof, according to (4-15), wherein the second oligonucleotide and the third oligonucleotide are all composed of 2'-O-methylribonucleotides.
(4-17) The oligonucleotide, or a pharmaceutically acceptable salt thereof, according to (4-15) or (4-16), wherein the second oligonucleotide has a base sequence of GGGUGG and the third oligonucleotide has a base sequence of CCACCU.
(4-18) An oligonucleotide, or a pharmaceutically acceptable salt thereof, comprising:
   a first oligonucleotide identifying a target RNA;
   a second oligonucleotide linked to the 3'-side of the first oligonucleotide;
   a third oligonucleotide capable of forming a complementary pair with the second oligonucleotide; and
   a first linking portion linking the second oligonucleotide and the third oligonucleotide, wherein the first oligonucleotide is composed of a target-corresponding nucleotide residue corresponding to an adenosine residue in the target RNA, an oligonucleotide of 10 to 30 residues linked to the 5'-side of the target-corresponding nucleotide residue and having a base sequence complementary to the target RNA, and an oligonucleotide of 3 to 6 residues linked to the 3'-side of the target-corresponding nucleotide residue and having a base sequence complementary to the target RNA, wherein the number of residues of the second oligonucleotide is 5 to 8, wherein the number of residues of the third oligonucleotide is 5 to 8, wherein the second oligonucleotide and the third oligonucleotide are all composed of 2'-O-alkylribonucleotides, wherein at least one residue selected from a counter region composed of the target-corresponding nucleotide residue and two respective residues on the 3'- and 5'-sides thereof is a nucleotide residue other than a natural ribonucleotide residue, wherein all phosphodiester bonds are phosphorothioate bonds, and wherein the oligonucleotide or a pharmaceutically acceptable salt thereof induces site-specific editing for the target RNA.
(4-19) The oligonucleotide, or a pharmaceutically acceptable salt thereof, according to (4-18), wherein the first linking portion contains at least one selected from the group consisting of an oligonucleotide of 4 or 5 residues and a polyalkyleneoxy group consisting of 2 to 8 alkyleneoxy units.
(4-20) The oligonucleotide, or a pharmaceutically acceptable salt thereof, according to (4-19), wherein the first linking portion contains an oligonucleotide having a base sequence selected from the group consisting of GCUAA, UUCG, UACG, UGCG, UCCG, GAAA, GUAA, GCAA, GGAA, GAGA, GUGA, GCGA, and GGGA.
(4-21) The oligonucleotide, or a pharmaceutically acceptable salt thereof, according to (4-19), wherein the first linking portion contains a polyethyleneoxy group
(4-22) The oligonucleotide, or a pharmaceutically acceptable salt thereof, according to (4-21), wherein the first linking portion contains a hexaethyleneoxy group.
(4-23) The oligonucleotide, or a pharmaceutically acceptable salt thereof, according to any one of (4-18) to (4-22), comprising a second linking portion containing an alkyleneoxy unit between the first oligonucleotide and the second oligonucleotide.
(4-24) The oligonucleotide, or a pharmaceutically acceptable salt thereof, according to (4-23), wherein the second linking portion contains an alkyleneoxy group having the carbon number of 3 to 6.
(4-25) The oligonucleotide, or a pharmaceutically acceptable salt thereof, according to (4-23), wherein the second linking portion contains a hexaethyleneoxy group.
(4-26) The oligonucleotide, or a pharmaceutically acceptable salt thereof, according to any one of (4-18) to (4-25), wherein the target-corresponding nucleotide residue is a cytidine residue, a uridine residue, an adenosine residue, or a derivative thereof.
(4-27) The oligonucleotide, or a pharmaceutically acceptable salt thereof, according to (4-26), wherein the target-corresponding nucleotide residue is a cytidine residue or a derivative thereof.
(4-28) The oligonucleotide, or a pharmaceutically acceptable salt thereof, according to any one of (4-18) to (4-27), wherein the first oligonucleotide contains at least one modified nucleotide selected from the group consisting of a 2'-O-alkylribonucleotide residue, a 2'-deoxy-2'-fluororibonucleotide residue, a bridged nucleotide residue, and a 2'-deoxyribonucleotide residue.
(4-29) The oligonucleotide, or a pharmaceutically acceptable salt thereof, according to any one of (4-18) to (4-27), wherein the first oligonucleotide contains at least one modified nucleotide selected from the group consisting of a 2'-O-methylribonucleotide residue, a 2'-deoxy-2'-fluororibonucleotide residue, a bridged nucleotide residue, and a 2'-deoxyribonucleotide residue.
(4-30) The oligonucleotide, or a pharmaceutically acceptable salt thereof, according to any one of (4-18) to (4-29), wherein at least one of the two respective residues on the 3'- and 5'-sides of the target-corresponding nucleotide residue is at least one modified nucleotide residue selected from the group consisting of a 2'-O-alkylribonucleotide residue and a 2'-deoxy-2'-fluororibonucleotide residue.
(4-31) The oligonucleotide, or a pharmaceutically acceptable salt thereof, according to any one of (4-18) to (4-30), wherein the second oligonucleotide has a base sequence of GGGUGG and the third oligonucleotide has a base sequence of CCACCU.
(4-32) The oligonucleotide, or a pharmaceutically acceptable salt thereof, according to any one of (4-1) to (4-31), wherein the oligonucleotide linked to the 5'-side of the target-corresponding nucleotide residue has a base sequence in which 2'-deoxy-2'-fluoronucleotide residues and 2'-O-alkylribonucleotide residues are alternately linked.
(4-33) The oligonucleotide, or a pharmaceutically acceptable salt thereof, according to (4-32), wherein in the oligonucleotide linked to the 5'-side of the target-corresponding nucleotide residue, a third nucleotide residue counted in the 5'-direction from the target-corresponding nucleotide residue is a 2'-deoxy-2'-fluoronucleotide residue.
(4-34) The oligonucleotide, or a pharmaceutically acceptable salt thereof, according to any one of (4-1) to (4-31), wherein the oligonucleotide linked to the 5'-side of the target-corresponding nucleotide residue has a base sequence in which bridged nucleotide residues and 2'-O-alkylribonucleotide residues are alternately linked.
(4-35) The oligonucleotide, or a pharmaceutically acceptable salt thereof, according to (4-35), wherein in the oligonucleotide linked to the 5'-side of the target-corresponding nucleotide residue, a third nucleotide residue counted in the 5'-direction from the target-corresponding nucleotide residue is a bridged nucleotide residue.
(4-36) The oligonucleotide, or a pharmaceutically acceptable salt thereof, according to any one of (4-1) to (4-31), wherein the oligonucleotide linked to the 5'-side of the target-corresponding nucleotide residue has a base sequence in which 2'-deoxy-2'-fluoronucleotide residues and bridged nucleotide residues are alternately linked
(4-37) The oligonucleotide, or a pharmaceutically acceptable salt thereof, according to (4-36), wherein in the oligonucleotide linked to the 5'-side of the target-corresponding nucleotide residue, a third nucleotide residue counted in the 5'-direction from the target-corresponding nucleotide residue is a 2'-deoxy-2'-fluoronucleotide residue.
(4-38) The oligonucleotide, or a pharmaceutically acceptable salt thereof, according to any one of (4-1) to (4-37), wherein in the first oligonucleotide, the oligonucleotide linked to the 3'-side of the target-corresponding nucleotide residue has a base sequence in which the 2'-O-alkylribonucleotide residues are linked.
(4-39) The oligonucleotide, or a pharmaceutically acceptable salt thereof, according to any one of (4-1) to (4-37), wherein in the first oligonucleotide, the oligonucleotide linked to the 3'-side of the target-corresponding nucleotide residue has a base sequence in which 2'-O-alkylribonucleotide residues and bridged nucleotide residues are alternately linked.
(4-40) The oligonucleotide, or a pharmaceutically acceptable salt thereof, according to any one of (4-1) to (4-39), wherein in the first oligonucleotide, the oligonucleotide linked to the 3'-side of the target-corresponding nucleotide residue consists of 4 to 6 residues.
(4-41) The oligonucleotide, or a pharmaceutically acceptable salt thereof, according to any one of (4-1) to (4-40), wherein the second oligonucleotide and the third oligonucleotide have a base sequence in which 2'-O-alkylribonucleotide residues are linked.
(4-42) The oligonucleotide, or a pharmaceutically acceptable salt thereof, according to any one of (4-1) to (4-41), wherein each of the first oligonucleotide, the second oligonucleotide, and the third oligonucleotide has nucleotide residues linked by a phosphorothioate bond.
(4-43) The oligonucleotide, or a pharmaceutically acceptable salt thereof, according to any one of (2-1) to (4-42), wherein the site-specific editing is caused by an enzyme reaction by adenosine deaminase.
(4-45) A medicine containing the oligonucleotide, or a pharmaceutically acceptable salt thereof, according to any one of (4-1) to (4-44).
(4-46) A therapeutic agent for a hereditary disease containing the oligonucleotide, or a pharmaceutically acceptable salt thereof, according to any one of (4-1) to (4-44).
(4-47) A pharmaceutical composition containing the oligonucleotide, or a pharmaceutically acceptable salt thereof, according to any one of (4-1) to (4-44) as an active ingredient.
(4-48) A use of the oligonucleotide, or a pharmaceutically acceptable salt thereof, according to any one of (4-1) to (4-44) for producing a medicine for prevention or treatment of a disease.
(4-49) A use of the oligonucleotide, or a pharmaceutically acceptable salt thereof, according to any one of (4-1) to (4-44) for use in prevention or treatment of a disease.
(4-50) A method for prevention or treatment of a disease by administering a pharmacologically effective amount of the oligonucleotide according to any one of (4-1) to (4-44) or a pharmacologically acceptable salt thereof to a warm-blooded animal.
(4-51) The method according to (4-50), wherein the disease is a hereditary disease.
(4-52) The method according to (4-51), wherein the hereditary disease is a disease treatable by converting an adenosine residue in a target RNA into an inosine residue.
(4-53) The method according to (4-51), wherein the hereditary disease is a hereditary disease caused by a mutation of a guanosine residue to an adenosine residue in a gene.
(4-54) The method according to any one of (4-50) to (4-53), wherein the warm-blooded animal is a human.
(4-51) The method according to (4-50), wherein the warm-blooded animal is a human.

### EFFECT OF THE INVENTION

An aspect of the present invention can provide the target-editing guide RNA having a small number of nucleotides added to a target recognition site, having excellent stability in vivo, and capable of inducing site-specific editing in a cell.

### BRIEF DESCRIPTION OF DRAWINGS

Fig. 1A is a chromatograph showing the editing rate of a target RNA with oligonucleotides.
Fig. 1B is a graph showing the editing rate of target RNA with oligonucleotides.
Fig. 2 is a graph showing the editing rate of target RNA with oligonucleotides.
Fig. 3A is a chromatograph showing the editing rate of target RNA with oligonucleotides.
Fig. 3B is a graph showing the editing rate of target RNA with oligonucleotides.
Fig. 4A is a chromatograph showing the editing rate of target RNA with oligonucleotides.
Fig. 4B is a graph showing the editing rate of target RNA with oligonucleotides.
Fig. 5A is a chromatograph showing the editing rate of target RNA with oligonucleotides.
Fig. 5B is a graph showing the editing rate of target RNA with oligonucleotides.
Fig. 6A is a graph showing the editing rate of target RNA with oligonucleotides in cells.
Fig. 6B is a graph showing changes in emission intensity due to RNA editing of oligonucleotides in cells.
Fig. 6C is a graph showing the editing rate of target RNA with oligonucleotides in cells.
Fig. 6D is a graph showing changes in emission intensity due to RNA editing of oligonucleotides in cells.
Fig. 7A is a graph showing the editing rate of target RNA with oligonucleotides in cells.
Fig. 7B is a graph showing changes in emission intensity due to RNA editing of oligonucleotides in cells.
Fig. 8A is a graph showing the editing rate of target RNA with oligonucleotides in cells.
Fig. 8B is a graph showing changes in emission intensity due to RNA editing of oligonucleotides in cells.
Fig. 9A is a chromatograph showing the editing rate of RNA of ATCB and GAPDH with oligonucleotides.
Fig. 9B is a graph showing the editing rate of RNA of ATCB and GAPDH with oligonucleotides.
Fig. 10 is a graph showing the editing rate of target RNA with oligonucleotides in the presence of hADAR1.
Fig. 11A is a graph showing the editing rate of target RNA with oligonucleotides in the presence of ADAR2.
Fig. 11B is a graph showing the editing rate of target RNA with oligonucleotides in the presence of ADAR1.
Fig. 12A is a graph showing the editing rate of target RNA with oligonucleotides in the presence of ADAR2.
Fig. 12B is a graph showing the editing rate of target RNA with oligonucleotides in the presence of ADAR1.
Fig. 13A is a graph showing the editing rate of target RNA with oligonucleotides in cells.
Fig. 13B is a graph showing changes in emission intensity due to RNA editing of oligonucleotides in cells.
Fig. 14A is a graph showing the editing rate of target RNA with oligonucleotides in cells.
Fig. 14B is a graph showing changes in emission intensity due to RNA editing of oligonucleotides in cells.
Fig. 15A is a graph showing the editing rate of target RNA with oligonucleotides in cells.
Fig. 15B is a graph showing changes in emission intensity due to RNA editing of oligonucleotides in cells.
Fig. 16A is a graph showing the editing rate of target RNA with oligonucleotides in the presence of ADAR2.
Fig. 16B is a graph showing the editing rate of target RNA with oligonucleotides in the presence of ADAR1.
Fig. 16C is a graph showing the editing rate of target RNA with oligonucleotides in cells.
Fig. 17A is a graph showing the editing rate of target RNA with oligonucleotides in the presence of ADAR2.
Fig. 17B is a graph showing the editing rate of target RNA with oligonucleotides in the presence ofADAR1.
Fig. 18 is a graph showing the editing rate of target RNA with oligonucleotides in cells.

### MODES FOR CARRYING OUT THE INVENTION

The term "step" as used herein comprises not only an independent step but also a step not clearly distinguishable from another step as long as the intended purpose of the step is achieved. If multiple substances correspond to a component in a composition, the content of the component in the composition means the total amount of the multiple substances present in the composition unless otherwise specified. Embodiments of the present invention will now be described in detail. It should be noted that the embodiments described below are exemplifications of oligonucleotides for embodying the technical ideas of the present disclosure, and the present disclosure is not limited to the oligonucleotides described below.

An oligonucleotide inducing site-specific editing for a target RNA (hereinafter, also referred to as a target-editing guide RNA) includes a first oligonucleotide identifying the target RNA, a second oligonucleotide linked to the 3'-side of the first oligonucleotide, a third oligonucleotide capable of forming a complementary pair with the second oligonucleotide, and a first linking portion linking the second oligonucleotide and the third oligonucleotide. The first oligonucleotide is composed of a target-corresponding nucleotide residue corresponding to an adenosine residue in the target RNA, an oligonucleotide of 10 to 30 residues linked to the 5'-side of the target-corresponding nucleotide residue and having a base sequence complementary to the target RNA, and an oligonucleotide of 3 to 6 residues linked to the 3'-side of the target-corresponding nucleotide residue and having a base sequence complementary to the target RNA. The number of residues of the second oligonucleotide is 5 to 8, and the number of residues of the third oligonucleotide is 5 to 8. At least one residue selected from a counter region composed of the target-corresponding nucleotide residue and two respective residues on the 3'- and 5'-sides thereof is a nucleotide residue other than a natural ribonucleotide residue.

Since the target-editing guide RNA has the second oligonucleotide, the first linking portion, and the third oligonucleotide on the 3'-side of the first oligonucleotide identifying the target RNA and has the nucleotide residue other than the natural ribonucleotide residue in the counter region, the target-editing guide RNA is excellent in stability in cells and can induce excellent site-specific editing. This is probably because, for example, ADAR catalyzing target editing is enhanced in editing activity by at least a portion of the second oligonucleotide, the first linking portion, and the third oligonucleotide that may present in a state of being free from the target RNA without forming a double strand with the target RNA while recognizing a double-stranded region composed of the target RNA and the first oligonucleotide. Therefore, it is considered that in the target-editing guide RNA, the first oligonucleotide functions as a complementary region (antisense region; ASR) for the target RNA, while at least a portion of the second oligonucleotide, the first linking portion, and the third oligonucleotide functions as an editing enhancement region, an ADAR coupling region (ADAR recruiting region; ARR), etc.

The target-editing guide RNA induces site-specific editing for the target RNA, for example, by recruiting ADAR, which catalyzes the target editing, to the target RNA. ADAR is an enzyme converting an adenosine residue in double-stranded RNA into an inosine residue by a hydrolytic deamination reaction and is widely present in mammalian cells. The inosine residue is similar in structure to the guanosine residue and is therefore translated as the guanosine residue at the time of translation of RNA information, and as a result, the RNA information is edited. When such RNA editing occurs in a portion encoding an amino acid, amino acid substitution etc. occur even though no DNA mutation exists on the genome. ADAR1, ADAR2, and ADAR3 having different genes are known as ADARs in mammals. The target-editing guide RNA enhances the target editing activity of at least ADAR1 or ADAR2 among them.

When introduced into a mammalian cell, the target-editing guide RNA can recruit ADAR existing in the cell to the target RNA and induce site-specific editing for the target RNA.

Nucleotides constituting the target-editing guide RNA may be composed of any of natural ribonucleotides (RNA), natural deoxyribonucleotides (DNA), RNA/DNA chimeras, and modified nucleotides that are modifications thereof. The nucleotides constituting the target-editing guide RNA may be linked to each other by a phosphodiester bond binding to a hydroxyl group of a sugar moiety of a nucleoside. The phosphodiester bond may be formed by utilizing the 2'-hydroxyl group, the 3'-hydroxyl group, or the 5'-hydroxyl group of the sugar moiety. The nucleotides constituting the target-editing guide RNA may form a 3'-5'phosphodiester bond, which is the natural form. At least one of the nucleotides constituting the target-editing guide RNA may be a modified nucleotide. Examples of the modified nucleotide include those with a modified sugar moiety, those with a modified phosphodiester bond, those with a modified base, and combinations thereof.

Examples of those with a modified sugar moiety include: 2'-O-alkylated ribonucleotides of D-ribofuranose (e.g., 2'-O-methylated, 2'-O-aminoethylated, 2'-O-propylated, 2'-O-allylated, 2'-O-methoxyethylated, 2'-O-butylated, 2'-O-pentylated, 2'-O-propargylated); bridged ribonucleotides in which the 2'- and 4'-positions of D-ribofuranose are bridged (e.g., 2'-O,4'-C-ethyleneated, 2'-O,4'-C-methylenated, 2'-O,4'-C-propyleneated, 2'-O,4'-C-tetramethylenated, 2'-O,4'-C-pentamethylenated, 2'-S,4'-C-methylenated, 2'-deoxy-2'-C, 4'-C-methyleneoxymethylenated form, S-cEt (2',4'-constrained ethyl), AmNA); 3'-deoxy-3'-amino-2'-deoxy-D-ribofuranose; 3'-deoxy-3'-amino-2'-deoxy-2'-fluoro-D-ribofuranose; and 2' -deoxy-2'-fluoro-D-ribofuranose.

Examples of those with a modified phosphodiester bond include a phosphorothioate bond (including optically active form derived from an asymmetric phosphorus atom), a methylphosphonate bond, a methylthiophosphonate bond, a phosphorodithioate bond, and a phosphoramidate bond.

Examples of those with a modified base include: halogenation; alkylation with the carbon number of 1 to 6 or 1 to 4 such as methylation, ethylation, propylation, isopropylation, cyclopropylation, butylation, isobutylation, s-butylation, t-butylation, and cyclobutylation; hydroxylation; amination; deaminolation; and demethylation. Specific examples include 5-methylation, 5-fluorination, 5-bromination, 5-iodination, N4-methylation, etc. of cytosine; 5-demethylation (uracil), 5-fluorination, 5-bromination, 5-iodination of thymine; N6-methylation, 8-bromination, etc. of adenine; and N2-methylation, 8-bromination, etc. of guanine.

The first oligonucleotide contained in the target-editing guide RNA identifies the target RNA. The target RNA is not particularly limited as long as an adenosine residue to be edited is contained, may be either cellular RNA or viral RNA, and is usually an mRNA precursor or mRNA encoding a protein. An editing site in the target RNA may be in untranslated regions, splice regions, exons, introns, or in any region affecting stability, structure, or function of RNA. The target RNA may also contain a mutation to be modified or altered. Alternatively, the target RNA may have a sequence mutated to encode a phenotype different from the natural form.

The target RNA is preferably an RNA encoding a protein, and specifically, examples of the encoded protein includes phosphorylated proteins involved in signal transduction of serotonin receptors, glutamate receptors, voltage-dependent potassium channels, STAT3, NFkBIA, MAPK14 etc.

The target-editing guide RNA can be applied, for example, to treatment of hereditary diseases. The hereditary disease may be, for example, a disease that may be treated by converting an adenosine residue in the target RNA into an inosine residue. The hereditary disease may be, for example, a hereditary disease caused by a mutation in a gene from a guanosine residue to an adenosine residue. Examples of the hereditary diseases include, for example, cystic fibrosis, albinism, α-1 antitrypsin deficiency, Alzheimer's disease, amyotrophic lateral sclerosis, asthma, β-thalassemia, CADASIL syndrome, Charcot Marie Tooth Disease, Chronic obstructive pulmonary disease (COPD), distal spinal muscular atrophy (DSMA), Duchenne/Becker muscular dystrophy, dystrophic epidermolysis bullosa, epidermolysis bullosa, Fabry's Disease, Factor V Leiden-related disorders, familial adenoma, polyposis, galactosemia, Gaucher's disease, glucose-6-phosphate dehydrogenase deficiency, hemophilia, hereditary hemochromatosis, Hunter's syndrome, Huntington's disease, Hurler's syndrome, inflammatory bowel disease (IBD), hereditary polyagglutination syndrome, Leber's congenital amaurosis, Lesch-Nyan syndrome, Lynch syndrome, Marfan syndrome, mucopolysaccharidosis, muscular dystrophy, myotonic dystrophy I and II, neurofibromatosis, Niemann-Pick disease Type A, B and C, NY-eso1-related pancreatic cancer, Parkinson's disease, Peutz-Jeghers syndrome, phenylketonuria, Pompe's disease, primary ciliary body disease, diseases related to prothrombin mutation such as prothrombin G20210A mutation, pulmonary hypertension, retinal pigment degeneration, Sandhoff's disease, severe combined immunodeficiency disease (SCID), sickle cell anemia, spinal muscular atrophy, Stargardt's disease, Tay-Sachs disease, Usher's syndrome, X-linked immunodeficiency, various forms of cancer (e.g., BRCA 1 and 2 related breast cancer, ovarian cancer).

Specific examples of the hereditary diseases include citrullinemia type I (ASS1), citrullinemia type II (SLC25A13), hemophilia (Factor V Leiden), primary hyperoxaluria (AGXT), distal myopathy (GNE), Cystic fibrosis (CFCFTR), homocystinuria (CBS), Hurler syndrome: Mucopolysaccharidosis (IDUA(SLC26A1)), Alexander's disease (GFAP), retinitis pigmentosa (EYS), phenylketonuria (PAH), hemochromatosis (HFE), and Gilbert syndrome (UGT1A1). The names in parentheses are the target gene names.

The first oligonucleotide is composed of a target-corresponding nucleotide residue corresponding to an adenosine residue to be edited in the target RNA, a 5'-side oligonucleotide of 10 to 30 residues linked to the 5'-side of the target-corresponding nucleotide residue and having a base sequence complementary to a corresponding base sequence of the target RNA, a 3'-side oligonucleotide of 3 to 6 residues linked to the 3'-side of the target-corresponding nucleotide residue and having a base sequence complementary to a corresponding base sequence of the target RNA. The oligonucleotides respectively linked to the 5'-side and the 3'-side of the target-corresponding nucleotide residue form a double strand with the target RNA, thereby identifying the target RNA and the site to be edited in the target RNA. Hereinafter, the region consisting of the target-corresponding nucleotide residue, one residue on the 3'-side thereof, and one residue on the 5'-side thereof will be referred to as a counter region for convenience, and the region other than the counter region of the first oligonucleotide will be sometimes referred to as a non-counter region.

The target-corresponding nucleotide residue is a nucleotide residue corresponding to the adenosine residue to be edited, and is, for example, a cytidine residue, a uridine residue, an adenosine residue, or a derivative thereof. The target-corresponding nucleotide residue is preferably a base not forming a base pair with the adenosine residue to be edited, more preferably a cytidine residue or a derivative thereof, and further preferably a cytidine residue.

The base sequence of the oligonucleotide linked to the 5'-side or 3'-side of the target-corresponding nucleotide residue is a base sequence complementary to the corresponding base sequence of the target RNA. The number of residues of the oligonucleotide linked to the 5'-side of the target-corresponding nucleotide residue is, for example, 10 or more, 12 or more, 14 or more, or 15 or more, and 26 or less, 20 or less, or 16 or less, from the viewpoint of specificity to the target RNA. The number of residues of the oligonucleotide linked to the 5'-side of the target-corresponding nucleotide residue may be 10 or more and 20 or less, 13 or more and 20 or less, or 13 or more and 15 or less, from the viewpoint of specificity to the target RNA. The number of residues of the oligonucleotide linked to the 3'-side of the target-corresponding nucleotide residue may be 3, or 3 or 4, from the viewpoint of editing activity. The number of residues of the oligonucleotide linked to the 3'-side of the target-corresponding nucleotide residue may be 4 to 6, 4 or 5, or 5 or 6, from the viewpoint of ADAR specificity.

In the target-editing guide RNA, at least one residue, at least two residues, or all three residues selected from the counter region may be nucleotide residues other than the natural ribonucleotide (RNA) residue, and preferably, at least one residue, at least two residues, or all three residues are modified nucleotide residues. In the modified nucleotide residue in the counter region, at least one of the sugar moiety and the phosphodiester bond may be modified, at least the sugar moiety may be modified, at least the phosphodiester bond may be modified, or the sugar moiety and the phosphodiester bond may be modified. For example, the target-corresponding nucleotide residue may be a cytidine residue having a phosphorothioate bond. For example, in each residue on the 5'-side or 3'-side of the target-corresponding nucleotide residue, at least one of the sugar moiety and the phosphodiester bond may be modified, at least the sugar moiety may be modified, at least the phosphodiester bond may be modified, or the sugar moiety and the phosphodiester bond may be modified. The modification of the sugar moiety in the counter region may be, for example, 2'-O-alkylation or 2'-deoxy-2'-fluorination.

In at least one residue, at least three residues, or all residues of the oligonucleotide residues (in a non-counter region) other than the counter region of the first oligonucleotide, at least one of the sugar moiety and the phosphodiester bond may be modified, at least the sugar moiety may be modified, at least the phosphodiester bond may be modified, or the sugar moiety and the phosphodiester bond may be modified. The modification of the sugar moiety in the non-counter region may be, for example, 2'-O-alkylation, 2'-deoxy-2'-fluorocation, bridging between the 2'- and 4'-positions, or 2'-deoxygenation (DNA). When the first oligonucleotide has multiple modified nucleotides, the multiple modified nucleotides may be arranged contiguously or may be arranged separately. The first oligonucleotide may be composed of all the nucleotide residues linked by phosphorothioate bonds.

The oligonucleotide linked to the 5'-side of the target-corresponding nucleotide residue of the first oligonucleotide may have a base sequence in which two types of modified nucleotide residues selected from the group consisting of 2'-deoxy-2'-fluoronucleotide residues, 2'-O-alkylribonucleotide residues, and bridged nucleotide residues are alternately linked. Therefore, the oligonucleotide linked to the 5'-side of the target-corresponding nucleotide residue may have a base sequence in which the 2'-deoxy-2'-fluoronucleotide residues and the 2'-O-alkylribonucleotide residues are alternately linked, may have a base sequence in which the bridged nucleotide residues and the 2'-O-alkylribonucleotide residues are alternately linked, or may have a base sequence in which the 2'-deoxy-2'-fluoronucleotide residues and the bridged nucleotide residues are alternately linked. As used herein, the phrase "having a base sequence in which two types of modified nucleotide residues are alternately linked" means both that the oligonucleotide of interest (in this description, corresponding to the oligonucleotide linked to the 5'-side of the target-corresponding nucleotide residue of the first oligonucleotide) partially has a base sequence in which the two types of modified nucleotide residues are alternately linked, and that the oligonucleotide of interest wholly has a base sequence in which the two types of modified nucleotide residues are alternately linked, and is used in the same meaning in the following description.

In the oligonucleotide linked to the 5'-side of the target-corresponding nucleotide residue of the first oligonucleotide, a third nucleotide residue counted in the 5'-direction from the target-corresponding nucleotide residue may be a modified nucleotide residue selected from the group consisting of a 2'-deoxy-2'-fluoronucleotide residue, a 2'-O-alkylribonucleotide residue, and a bridged nucleotide residue. Furthermore, in the oligonucleotide linked to the 5'-side of the target-corresponding nucleotide residue, the third nucleotide residue counted in the 5'-direction from the target-corresponding nucleotide residue may be a modified nucleotide residue selected from the group consisting of a 2'-deoxy-2'-fluoronucleotide residue, a 2'-O-alkylribonucleotide residue, and a bridged nucleotide residue, and a fourth nucleotide residue may be a modified nucleotide residue different from the third nucleotide residue. In this case, the third nucleotide residue counted in the 5'-direction from the target-corresponding nucleotide residue refers to the third nucleotide residue counted in the 5'-direction when the nucleotide residue adjacent to the target-corresponding oligonucleotide residue in the 5'-direction is considered as the first nucleotide without including the target-corresponding nucleotide residue itself.

In the first oligonucleotide, the oligonucleotide linked to the 3'-side of the target-corresponding nucleotide residue may have a base sequence in which 2'-O-alkylribonucleotide residues are linked. In the first oligonucleotide, the oligonucleotide linked to the 3'-side of the target-corresponding nucleotide residue may have a base sequence in which two types selected from the group consisting of 2'-deoxy-2'-fluoronucleotide residues, 2'-O-alkylribonucleotide residues, and bridged nucleotide residues are alternately linked, may have a base sequence in which the 2'-deoxy-2'-fluoronucleotide residues and the bridged nucleotide residues are alternately linked, or may have a base sequence in which the 2'-O-alkylribonucleotide residues and the bridged nucleotide residues are alternately linked. Furthermore, in the first oligonucleotide, from the viewpoint of ADAR specificity, a second nucleotide residue counted in the 3'-direction from the target-corresponding nucleotide residue may be one selected from the group consisting of a 2'-deoxy-2'-fluoronucleotide residue, a 2'-O-alkylribonucleotide residue, and a bridged nucleotide residue, and may be a bridged nucleotide residue. In this case, the second nucleotide residue counted in the 3'-direction from the target-corresponding nucleotide residue refers to the second nucleotide residue counted in the 3'-direction when the nucleotide residue adjacent to the target-corresponding oligonucleotide residue in the 3'-direction is considered as the first nucleotide without including the target-corresponding nucleotide residue itself.

The second oligonucleotide and the third oligonucleotide each consist of 5 to 8 residues, 5 to 7 residues, or 6 residues, and have base sequences capable of forming a complementary pair with each other. The numbers of residues of the second oligonucleotide and the third oligonucleotide may be the same or different. Examples of the base sequence of the second oligonucleotide can include base sequences including GGGUGG, GGGUG, GGUGG, GGGU, GGUG, GUGG, GGG, GGU, GUG, UGG, GG, GC, GA, GU, UC, UG, UA, UU, CG, CA, CU, CC, AG, AA, AC, AU, etc. The second oligonucleotide may have a base sequence including at least one selected from the group consisting of a sequence consisting of two or three contiguous guanines (GG or GGG), a sequence consisting of contiguous uracil and guanine (UG) and a sequence consisting of contiguous guanine, uracil, and guanine (GUG) and may have a base sequence containing at least two or three selected from this group. The second oligonucleotide may also have a sequence of contiguous guanine, uracil, and guanine (GUG) or a sequence of contiguous uracil, uracil, and guanine (UGG) binding to the first linking portion. The base sequence of the third oligonucleotide may be selected to be capable of forming a complementary pair with the base sequence of the second oligonucleotide. In this case, the second oligonucleotide and the third oligonucleotide being capable of forming a complementary pair means not only that all the residues of the second oligonucleotide form Watson-click type base pairs with all the residues of the corresponding third oligonucleotide, but also that the second oligonucleotide and the third oligonucleotide contain one or two mismatched base pairs and contain one or two wobble base pairs. The mismatched base pair means a thermodynamically unstable base pair, and the wobble base pair means a thermodynamically stable non-Watson-click type base pair such as GU base pair. The wobble base pair may be formed, for example, at the 3'-end of the third oligonucleotide.

The second and third oligonucleotides may each have at least one residue, at least three residues, or all residues modified in at least one of the sugar moiety and the phosphodiester bond, modified in at least the sugar moiety, modified in at least the phosphodiester bond, or modified in the sugar moiety and the phosphodiester bond. The modification of the sugar moiety in the second and third oligonucleotides may be, for example, 2'-O-alkylation, or 2'-deoxy-2'-fluorination, 2'-deoxygenation (DNA). When the second or third oligonucleotide has multiply modified nucleotides, the multiply modified nucleotides may be arranged contiguously or may be arranged separately.

The second and third oligonucleotides may have a base sequence in which 2'-O-alkylribonucleotide residues are linked. The nucleotide residues of each of the second and third oligonucleotides may all be linked by phosphorothioate bonds.

The first linking portion may contain at least one selected from the group consisting of an oligonucleotide of 4 or 5 residues and a polyalkyleneoxy group consisting of 1 to 8 alkyleneoxy units. The first linking portion may be bonded by a phosphodiester bond or a modified phosphodiester bond to each of the 3'-side of the second oligonucleotide and the 5'-side of the third oligonucleotide. Therefore, the first linking portion may be bonded by a phosphodiester bond or a modified phosphodiester bond to each of the hydroxyl group of the sugar moiety at the 3'-end of the second oligonucleotide and the hydroxyl group of the sugar moiety at the 5'-end of the third oligonucleotide. As a result, the second and third oligonucleotides may be configured to form complementary pairs to form a stem structure, and the first linking portion may be configured to form a loop structure.

When the first linking portion contains the oligonucleotide of 4 or 5 residues, specific examples of the base sequence thereof include: GCUAA; UNCG fold type such as UUCG, UACG, UGCG, UCCG; GNRA fold type such as GAAA, GUAA, GCAA, GGAA, GAGA, GUGA, GCGA, GGGA; RNYA fold type such as GUCA, GCCA, GGCA, GACA, AUCA, ACCA, AGCA, AACA, GUUA, GCUA, GGUA, GAUA, AUUA, ACUA, AGUA, AAUA; GGUG fold type; CUUG fold type; and AGNN fold type such as AGUU, AGUC, AGUG, AGUA, AGCU, AGCC, AGCG, AGCA. For details of these loop structures, see, for example, Biophys. J., 113, 257-267, 2017. The base sequence of the first linking portion may have GCUAA, a UNCG fold type base sequence, or a GNRA fold type base sequence and may have a base sequence of UUCG.

When the first linking portion contains an oligonucleotide of 4 or 5 residues, at least 1 residue or at least 3 residues of the nucleotide residues constituting the first linking portion may be modified nucleotide residues. The modified nucleotide in the first linking portion may be modified in at least one of the sugar moiety and the phosphodiester bond, may be modified in at least the sugar moiety, may be modified in at least the phosphodiester bond, or may be modified in the sugar moiety and the phosphodiester bond.

When the first linking portion contains a polyalkyleneoxy group, the carbon number of the alkyleneoxy unit may be, for example, 2 to 4, 2 to 3, or 2. Therefore, the alkyleneoxy unit may be an ethyleneoxy unit, a propyleneoxy unit, or a butyleneoxy unit. The number of the alkyleneoxy units constituting the polyalkyleneoxy group may be, for example, 1 to 8, 5 to 7, or 6. The alkyleneoxy units constituting the polyalkyleneoxy group may be the same or different. Furthermore, the first linking portion may contain a polyethyleneoxy group of 1 to 8 units and may contain a hexaethyleneoxy group. When the first linking portion contains a polyalkyleneoxy group, the polyalkyleneoxy group may link the hydroxyl group of the sugar moiety at the 3'-end of the second oligonucleotide, via a phosphodiester bond or a modified phosphodiester bond, to the hydroxyl group of the sugar moiety at the 5'-end of the third oligonucleotide.

The first linking portion may be composed of only an oligonucleotide, may be configured to contain a nucleotide and an alkyleneoxy unit, or may be configured to contain only an alkyleneoxy unit. The target-editing guide RNA can exhibit excellent target editing activity even when the first linking portion has a loop structure containing an alkyleneoxy unit.

The target-editing guide RNA may have a second linking portion between the first oligonucleotide and the second oligonucleotide. Therefore, the first oligonucleotide and the second oligonucleotide may be linked by the second linking portion. The second linking portion is configured to contain, for example, an alkyleneoxy unit. The carbon number of the alkylene portion of the alkyleneoxy unit may be 2 to 8 or 2 to 6. The second linking portion may contain 1 to 8 or 1 to 6 alkyleneoxy units having the carbon number of 2 or 3, may contain a polyalkyleneoxy group contiguously containing 1 to 8 or 1 to 6 alkyleneoxy units having the carbon number of 2 or 3, and may contain a hexaethyleneoxy group. The second linking portion may contain an alkyleneoxy group having the carbon number of 3 to 6. The polyalkyleneoxy group constituting the second linking portion may link the hydroxyl group of the sugar moiety at the 3'-end of the first oligonucleotide, via a phosphodiester bond or a modified phosphodiester bond, to the hydroxyl group of the sugar moiety at the 5'-end of the second oligonucleotide.

The target-editing guide RNA can be synthesized according to a method described in a known document (see, e.g., Nucleic Acids Research, 12, 4539 (1984)) by using a commercially available synthesizer (e.g., model 392 manufactured by PerkinElmer using a phosphoramidite method). The phosphoramidite reagent to be used may be a commercially available reagent or may be a reagent appropriately synthesized according to a method described in a known document. After coupling the phosphoramidite reagent, a phosphorothioate bond can be introduced by reacting with a reagent such as sulfur, tetraethylthiuram disulfide (TETD, Applied Biosystems), the Beaucage reagent (Glen Research), or xanthan hydride (see, e.g., Tetrahedron Letters, 32, 3005 (1991), J.Am. Chem. Soc., 112, 1253 (1990), PCT/WO98/54198).

The oligonucleotides (target-editing guide RNA) may be used in the form of a pharmaceutically acceptable salt. The "pharmaceutically acceptable salt" is a salt of an oligonucleotide. Examples of such a salt include: metal salts including alkali metal salts such as sodium salt, potassium salt, and lithium salt, alkaline earth metal salts such as calcium salt and magnesium salt, aluminum salt, iron salt, zinc salt, copper salt, nickel salt, and cobalt salt; amine salts including inorganic salts such as ammonium salt, and organic salts such as t-octylamine salt, dibenzylamine salt, morpholine salt, glucosamine salt, phenylglycine alkyl ester salt, ethylenediamine salt, N-methylglucamine salt, guanidine salt, diethylamine salt, triethylamine salt, dicyclohexylamine salt, N,N'-dibenzylethylenediamine salt, chloroprocaine salt, procaine salt, diethanolamine salt, N-benzyl-phenethylamine salt, piperazine salt, tetramethylammonium salt, and tris(hydroxymethyl)aminomethane salt; inorganic acid salts including hydrohalic acid salts such as hydrofluoride, hydrochloride, hydrobromide, and hydroiodide, nitrate, perchlorate, sulfate, and phosphate; organic acid salts including lower alkane sulfonates such as methane sulfonate, trifluoromethane sulfonate, and ethane sulfonate, aryl sulfonates such as benzene sulfonate and p-toluene sulfonate, acetate, malate, fumarate, succinate, citrate, tartrate, oxalate, maleate; and amino acid salts such as glycine salts, lysine salts, arginine salts, ornitine salts, glutamates, and aspartate. The form of the pharmaceutically acceptable salt of the oligonucleotide is preferably an alkali metal salt of the oligonucleotide, more preferably a sodium salt. These salts can be produced by known methods.

The oligonucleotide and the pharmaceutically acceptable salt thereof may also exist as solvates (e.g., hydrates) and may be such solvates.

The oligonucleotide and the pharmaceutically acceptable salt thereof may have optically active substances derived from asymmetric phosphorus atoms, and the oligonucleotides of the present invention include such optically active substances. Such optically active substances can be synthesized by known methods (e.g., Org. Lett., 114, 967 (2009), Bioorganic & Medical Chemistry Letters, 8, 2359 (1998)).

When used in the treatment of a disease, the oligonucleotides, the pharmaceutically acceptable salts thereof, or the solvates are mixed with itself or an appropriate pharmaceutically acceptable excipient, diluent, etc., and can be administered orally as tablets, capsules, granules, powders, or syrups, or parenterally as injections, suppositories, patches, or topical agents.

These formulations are manufactured by well-known methods by using additives such as excipients (e.g., organic excipients including: sugar derivatives such as lactose, sucrose, dextrose, mannitol, and sorbitol; starch derivatives such as corn starch, potato starch, α-starch, and dextrin; cellulose derivatives such as crystalline cellulose; gum arabic; dextran; and pullulan; and inorganic excipients including: silicate derivatives such as light anhydrous silicic acid, synthetic aluminum silicate, calcium silicate, and magnesium aluminometasilicate; phosphates such as calcium hydrogen phosphate; carbonates such as calcium carbonate; and sulfates such as calcium sulfate), lubricants (e.g., stearic acid; metallic stearate such as calcium stearate, magnesium stearate; talc; colloidal silica; waxes such as bead wax and gay wax; boric acid; adipic acid; sulfates such as sodium sulfate; glycol; fumaric acid; sodium benzoate; DL leucine; lauryl sulfate such as sodium lauryl sulfate and magnesium lauryl sulfate; silicic acids such as anhydrous silicic acid and silicic acid hydrate; and starch derivatives described above), binders (e.g., hydroxypropyl cellulose, hydroxypropyl methyl cellulose, polyvinylpyrrolidone, macrogol, and the same compounds as the excipients), disintegrants (e.g., cellulose derivatives such as low-substituted hydroxypropyl cellulose, carboxymethyl cellulose, carboxymethyl cellulose calcium, internally cross-linked sodium carboxymethyl cellulose; chemically modified starches/celluloses such as carboxymethyl starch, sodium carboxymethyl starch, and cross-linked polyvinylpyrrolidone), emulsifiers (e.g., colloidal clays such as bentonite and veegum; metal hydroxides such as magnesium hydroxide and aluminum hydroxide; anionic surfactants such as sodium lauryl sulfate and calcium stearate; cationic surfactants such as benzalkonium chloride; and nonionic surfactants such as polyoxyethylene alkyl ether, polyoxyethylene sorbitan fatty acid ester, sucrose fatty acid ester), stabilizers (p-hydroxybenzoates such as methylparaben and propylparaben; alcohols such as chlorobutanol, benzyl alcohol, and phenylethyl alcohol; benzalconium chloride; phenols such as phenol, cresol; thimerosal; dehydroacetic acid; sorbic acid, etc.), flavoring and flavoring agents (e.g., commonly used sweeteners, acidulants, and flavors), diluents, etc.

A therapeutic agent containing the oligonucleotide, the pharmaceutically acceptable salt thereof, or the solvate may contain 0.1 to 250 µmoles/ml, preferably 1 to 50 µmoles/ml oligonucleotide. The therapeutic agent may be configured to contain a predetermined amount of the oligonucleotide, the pharmaceutically acceptable salt thereof, or the solvate, 0.02 to 10 %w/v carbohydrate or polyhydric alcohol, and 0.01 to 0.4 %w/v pharmaceutically acceptable surfactant.

The carbohydrate is particularly preferably at least one of monosaccharides and disaccharides. Examples of these carbohydrates and polyhydric alcohols include glucose, galactose, mannose, lactose, maltose, mannitol, and sorbitol. These may be used alone or in combination.

Preferable examples of the surfactant include polyoxyethylene sorbitan mono to tri-ester, alkylphenyl polyoxyethylene, sodium taurocholate, sodium collate, and polyhydric alcohol ester. Among these, polyoxyethylene sorbitan mono to tri-esters are particularly preferable, and particularly preferable esters are oleate, laurate, stearate, and palmitate. These may be used alone or in combination.

The therapeutic agent containing the oligonucleotide, the pharmaceutically acceptable salt thereof, or the solvate may more preferably contain 0.03 M to 0.09 M pharmaceutically acceptable neutral salt such as sodium chloride, potassium chloride, and/or calcium chloride.

The therapeutic agent containing the oligonucleotide, the pharmaceutically acceptable salt thereof, or the solvate can more preferably contain a 0.002 to 0.05 M pharmaceutically acceptable buffer. Examples of the preferred buffer include sodium citrate, sodium glycinate, sodium phosphate, and tris(hydroxymethyl) aminomethane. These buffers may be used alone or in combination.

The therapeutic agent described above may be supplied in a solution state. However, for the case that the therapeutic agent needs to be stored for a certain period of time, it is preferable that the therapeutic agent is usually freeze-dried for the purpose of stabilizing the oligonucleotide and preventing a decrease in the therapeutic effect, and in this case, the therapeutic agent may be reconstituted with a solution (such as distilled water for administration), i.e., put into a liquid state to be administered, before use. Therefore, the therapeutic agent of the present invention also includes those in a freeze-dried state for use after reconstitution with a solution so that each component has a predetermined concentration range. Amino acids such as albumin and glycine may further be contained for the purpose of promoting the solubility of the freeze-dried product.

When the oligonucleotide, the pharmaceutically acceptable salt thereof, or the solvate is administered to a human, for example, a dosage of about 0.01 mg/kg to 100 mg/kg (body weight), preferably 0.1 mg/kg to 20 mg/kg (body weight) may be administered in one or several divided doses per day for an adult by subcutaneous injection, intravenous drip injection, or intravenous injection, and the dosage and the number of doses may appropriately be changed depending on a type of disease, symptom, the age, an administration method, etc.

### Method for Treating Disease

The method for treating a disease includes a step of administering the therapeutic agent to a subject. As used herein, the term "treatment" may be any treatment performed for the disease, and examples thereof include treatment, improvement, suppression of progression (prevention of exacerbation), prevention, etc. of the disease (preferably treatment or prevention). The subject of treatment may be a warm-blooded animal including humans and may be a non-human warm-blooded animal.

### Site-Specific Editing Method for Target RNA

A site-specific editing method of a target RNA includes a step of bringing the target RNA into contact with the target-editing guide RNA that is an oligonucleotide inducing site-specific editing for the target RNA in the presence of adenosine deaminase. By partially forming a double strand with the target RNA and recruiting adenosine deaminase, the target-editing guide RNA can site-specifically convert an adenosine residue contained in the target RNA into an inosine residue. The site-specific editing method of a target RNA may further include a step of preparing the target-editing guide RNA.

The site-specific editing method of a target RNA can be performed, for example, by introducing the target-editing guide RNA described above into a eukaryotic cell having the target RNA. To a method of introducing the target-editing guide RNA into a eukaryotic cell, various techniques used in nucleic acid medicines can appropriately be selected and applied. The site-specific editing method of a target RNA may be performed in vitro or in vivo.

In another aspect, the present invention includes a use of the target-editing guide RNA in manufacture of a pharmaceutical composition used in treatment of a disease (e.g., a hereditary disease), a use of the target-editing guide RNA in treatment of a disease (e.g., a hereditary disease), and the target-editing guide RNA used in treatment of a disease (e.g., a hereditary disease).

### Examples

Hereinafter, the present invention will specifically be described with reference to Examples; however, the present invention is not limited to these Examples.

### (Reference Example 1)

An oligonucleotide having the sequences described in Table 1 and consisting entirely of natural RNA residues (hereinafter, also referred to as del03_01) was synthesized by the phosphoramidite method (see, e.g., Nucleic Acids Research, 12, 4539 (1984), Nature Communications 6, Article number: 6317 (2015)). The obtained compound was identified by negative ion ESI mass spectrometry (measured value: 11194.40).

In Table 1, the underlined part corresponds to the first oligonucleotide (ASR), and the target RNA is Rluc_sRNA described later. The oligonucleotide of Reference Example 1 may probably have the following stem-loop structure, for example.

**[Table 1]**

| | Sequence name | Sequence (5'-3') | SEQ ID NO: |
|---|---|---|---|
| Reference Example 1 | del03_01 | UUCAGCAGCUCGAACCAAGGGGUGGUUCGCCACCU | 1 |

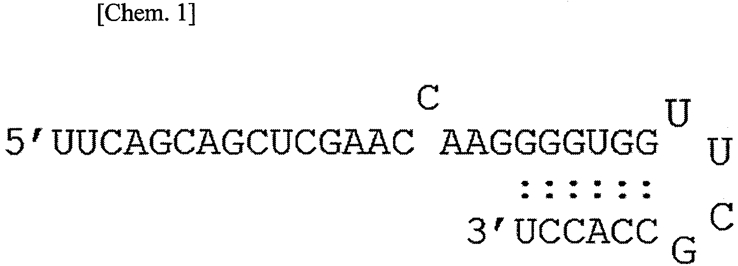

### (Examples 1 to 50)

Oligonucleotide compounds of Examples 1 to 46 were obtained based on the oligonucleotide sequence of Reference Example 1 by introducing modified nucleotides as described below. Oligonucleotide compounds of Examples 47 to 50 have the first oligonucleotide (ASR) having the sequence described below and are obtained by introducing modified nucleotides as described below. These oligonucleotide compounds were synthesized by the phosphoramidite method in the same manner as in Reference Example 1. In the sequences, when an "18" portion was synthesized, DMT Hexaethylene Glycol phosphoramidite (ChemGene, catalog number: CLP-9765) was used. When a "9" portion was synthesized, DMT-Triethoxy-glycol phosphoramidite (ChemGene, catalog number: CLP-1113) was used. When a "6" portion was synthesized, DMT-hexane-diol phosphoramidite (ChemGene, catalog number: CLP-1120) was used. When a "3" portion was synthesized, DMT-propane-Diol phosphoramidite (ChemGene, catalog number: CLP-9908) was used. A "2'-O-methylnucleoside" portion was synthesized by using the phosphoramidite compound described in Nucleic Acids Research 17, 3373 (1989). A "DNA" portion was synthesized by using the phosphoramidite compound described in Nucleic Acids Research 11, 4539 (1984). A "2'-O,4'-C-methylene nucleoside" portion was synthesized by using the phosphoramidite compound described in WO 99/14226. A "2'-deoxy-2'-fluoronucleoside" portion was synthesized by using J. Med. Chem., 36, 831 (1993).

**[Table 2]**

| Example | Sequece name | Sequence (5'-3') | Molecular weight |
|---|---|---|---|
| 1 | del03_02 | U(M)U(M)C(M)A(M)G(M)C(M)A(M)G(M)C(M)U(M)C(M)G(M)A(M)A(M)CCAA( M)G(M)GGGUGGUUCGCCACCU | 11418.51 |
| 2 | del03_03 | U(M)U(M)C(M)A(M)G(M)C(M)A(M)G(M)C(M)U(M)C(M)G(M)A(M)A(M)C(M)CA A(M)G(M)GGGUGGUUCGCCACCU | 11432.72 |
| 3 | del03_04 | U(M)U(M)C(M)A(M)G(M)C(M)A(M)G(M)C(M)U(M)C(M)G(M)A(M)A(M)CCA(M) A(M)G(M)GGGUGGUUCGCCACCU | 11432.81 |
| 4 | del03_05 | UUCAGCAGCUCGAACC(M)AAGGGGUGGUUCGCCACCU | 11208.62 |
| 5 | del03_06 | UUCAGCAGCUCGAACC(F)AAGGGGUGGUUCGCCACCU | 11196.12 |
| 6 | del03_07 | UUCAGCAGCUCGAACcAAGGGGUGGUUCGCCACCU | 11178.29 |
| 7 | del03_08 | UUCAGCAGCUCGAACCAAAGGGGUGGUUCGCCACCU | 11209.24 |
| 8 | del03_09 | UUCAGCAGCUCGAACCAAGGGGUGG18CCACCU | 10275.98 |
| 9 | del03_10 | U(M)tC(M)aG(M)cA(M)gC(M)tC(M)gA(M)aCCAA(M)G(M)GGGUGGUUCGCC ACCU | 11239.52 |
| 10 | del03_11 | tU(M)cA(M)gC(M)aG(M)cU(M)cG(M)aA(M)CCAA(M)G(M)GGGUGGUUCGCC ACCU | 11225.89 |
| 11 | del03_12 | U(M)U(F)C(M)A(F)G(M)C(F)A(M)G(F)C(M)U(F)C(M)G(F)A(M)A(F)CCAA(M)G( M)GGGUGGUUCGCCACCU | 11331.72 |
| 12 | del03_13 | U(F)U(M)C(F)A(M)G(F)C(M)A(F)G(M)C(F)U(M)C(F)G(M)A(F)A(M)CCAA(M)G( M)GGGUGGUUCGCCACCU | 11330.49 |
| 13 | del03_14 | UUCAGCAGCUCGAAC(M)C^A(M)AGGGGUGGUUCGCCACCU | 11238.28 |
| 14 | del03_15 | UUCAGCAGCUCGAAC(F)C^A(F)AGGGGUGGUUCGCCACCU | 11213.19 |
| 15 | del03_16 | UUCAGCAGCUCGAAC(F)C^A(M)AGGGGUGGUUCGCCACCU | 11225.28 |
| 16 | del03_17 | UUCAGCAGCUCGAAC(M)C^A(F)AGGGGUGGUUCGCCACCU | 11223.43 |
| 17 | del03_18 | UUCAGCAGCUCGAACCAAGG(M)G(M)G(M)U(M)G(M)G(M)18C(M)C(M)A(M )C(M)C(M)U(M) | 10444.02 |
| 18 | del03_19 | UUCAGCAGCUCGAACCAAGG(M)G(M)G(M)UGG(M)18C(M)CAC(M)C(M)U( M) | 10388.29 |
| 19 | del03_20 | UUCAGCAGCUCGAACCAAGgggtgg18ccacct | 10112.47 |
| 20 | del03_21 | UUCAGCAGCUCGAACCAAGcgcgcg18cgcgcg | 10098.29 |
| 21 | del03_23 | U(F)U(M)C(F)A(M)G(F)C(M)A(F)G(M)C(F)U(M)C(F)G(M)A(F)A(M)C(M)C^A(M) A(M)G(M)G(M)G(M)G(M)U(M)G(M)G(M)18C(M)C(M)A(M)C(M)C(M)U(M) | 10626.67 |
| 22 | del03_24 | U(F)^U(M)^C(F)A(M)G(F)C(M)A(F)G(M)C(F)U(M)C(F)G(M)A(F)A(M)C(M)C^A( M)A(M)G(M)G(M)G(M)G(M)U(M)G(M)G(M)18C(M)C(M)A(M)C(M)^C(M)^U(M) | 10694.94 |
| 23 | del03_25 | U(F)^U(M)^C(F)^A(M)^G(F)^C(M)^A(F)^G(M)^C(F)^U(M)^C(F)^G(M)^A(F)^A(M)^ C(M)^C^A(M)^A(M)^G(M)^G(M)G(M)G(M)U(M)G(M)G(M)18C(M)C(M)A(M)C(M )^C(M)^U(M) | 10948.84 |
| 24 | del03_26 | U(F)^U(M)^C(F)^A(M)^G(F)^C(M)^A(F)^G(M)^C(F)^U(M)^C(F)^G(M)^A(F)^A(M)^ C(M)^C^A(M)^A(M)^G(M)^G(M)^G(M)^U(M)^G(M)^G(M)^18^C(M)^C(M)^A (M)^C(M)^C(M)^U(M) | 11109.85 |

**[Table 3]**

| Example | Sequece name | Sequence (5'-3') | Molecular weight |
|---|---|---|---|
| 25 | del03_27 | U(L)U(M)C(L)A(M)G(L)C(M)A(F)G(M)C(F)U(M)C(F)G(M)A(F)A(M)CCAA(M)G( M)GGGUGGUUCGCCACCU | 11391.72 |
| 26 | del03_28 | U(F)U(M)C(F)A(M)G(F)C(M)A(L)G(M)C(L)U(M)C(F)G(M)A(F)A(M)CCAA(M)G( M)GGGUGGUUCGCCACCU | 11365.82 |
| 27 | del03_29 | U(F)U(M)C(F)A(M)G(F)C(M)A(F)G(M)C(F)U(M)C(L)G(M)A(L)A(M)CCAA(M)G( M)GGGUGGUUCGCCACCU | 11367.83 |
| 28 | del03_30 | U(F)^U(M)^C(F)^A(M)^G(L)^C(M)^A(L)^G(M)^C(L)^U(M)^C(L)^G(M)^A(L)^A(M)^ C(M)^C^A(M)^A(M)^G(M)^G(M)^G(M)^G(M)^U(M)^G(M)^G(M)^18^C(M)^C(M)^A (M)^C(M)^C(M)^U(M) | 11186.25 |
| 29 | del03_31 | U(L)^U(M)^C(L)^A(M)^G(L)^C(M)^A(L)^G(M)^C(L)^U(M)^C(L)^G(M)^A(L)^A(M)^ C(M)^C^A(M)^A(M)^G(M)^G(M)^G(M)^G(M)^U(M)^G(M)^G(M)^18^C(M)^C(M)^A (M)^C(M)^C(M)^U(M) | 11234.18 |
| 30 | del03_32 | C(L)^A(M)^G(L)^C(M)^A(L)^G(M)^C(L)^U(M)^C(L)^G(M)^A(L)^A(M)^C(M)^C^A( M)^A(M)^G(M)^G(M)^G(M)^G(M)^U(M)^G(M)^G(M)^18^C(M)^C(M)^A(M)^C(M)^ C(M)^U(M) | 10550.65 |
| 31 | del03_33 | ·G(L)^C(M)^A(L)^G(M)^C(L)^U(M)^C(L)^G(M)^A(L)^A(M)^C(M)^C^A(M)^A(M)^G( M)^G(M)^G(M)^G(M)^U(M)^G(M)^G(M)^18^C(M)^C(M)^A(M)^C(M)^C(M)^U(M) | 9843.76 |
| 32 | del03_34 | U(F)^U(M)^C(F)^A(M)^G(F)^C(M)^A(F)^G(M)^C(F)^U(M)^C(F)^G(M)^A(F)^A(M)^ C(M)^C^A(M)^A(M)^G(M)^3^G(M)^G(M)^G(M)^U(M)^G(M)^G(M)^18^C(M)^C(M) ^A(M)^C(M)^C(M)^U(M) | 11262.34 |
| 33 | del03_35 | U(F)^U(M)^C(F)^A(M)^G(F)^C(M)^A(F)^G(M)^C(F)^U(M)^C(F)^G(M)^A(F)^A(M)^ C(M)^C^A(M)^A(M)^G(M)^6^G(M)^G(M)^G(M)^U(M)^G(M)^G(M)^18^C(M)^C(M) ^A(M)^C(M)^C(M)^U(M) | 11304.26 |
| 34 | del03_36 | U(F)^U(M)^C(F)^A(M)^G(F)^C(M)^A(F)^G(M)^C(F)^U(M)^C(F)^G(M)^A(F)^A(M)^ C(M)^C^A(M)^A(M)^G(M)^9^G(M)^G(M)^G(M)^U(M)^G(M)^G(M)^18^C(M)^C(M) ^A(M)^C(M)^C(M)^U(M) | 11337.37 |
| 35 | del03_37 | U(F)^U(M)^C(F)^A(M)^G(F)^C(M)^A(F)^G(M)^C(F)^U(M)^C(F)^G(M)^A(F)^A(M)^ C(M)^C^A(M)^A(M)^G(M)^18^G(M)^G(M)^G(M)^U(M)^G(M)^G(M)^18^C(M)^C( M)^A(M)^C(M)^C(M)^U(M) | 11467.60 |
| 36 | del03_38 | U(F)^U(M)^C(F)^A(M)^G(F)^C(M)^A(F)^G(M)^C(F)^U(M)^C(F)^G(M)^A(F)^A(M)^ C(M)^C^A(M)^A(M)^G(M)^C(M)^G(M)^G(M)^G(M)^U(M)^G(M)^G(M)^18^C(M)^C (M)^A(M)^C(M)^C(M)^U(M) | 11442.61 |
| 37 | del03_39 | U(F)^U(M)^C(F)^A(M)^G(F)^C(M)^A(F)^G(M)^C(F)^U(M)^C(F)^G(M)^A(F)^A(M)^ C(M)^C^A(M)^A(M)^G(M)^C(M)^G(M)^G(M)^G(M)^G(M)^U(M)^G(M)^G(M)^18^ C(M)^C(M)^A(M)^C(M)^C(M)^U(M) | 11818.78 |
| 38 | del03_40 | U(F)^U(M)^C(F)^A(M)^G(F)^C(M)^A(F)^G(M)^C(F)^U(M)^C(F)^G(M)^A(F)^A(M)^ C(M)^C^A(M)^A(M)^G(M)^C(M)^G(M)^G(M)^G(M)^G(M)^G(M)^U(M)^G(M)^G(M )^18^C(M)^C(M)^A(M)^C(M)^C(M)^U(M) | 12194.30 |
| 39 | del03_41 | U(F)^U(M)^C(F)^A(M)^G(F)^C(M)^A(F)^G(M)^C(F)^U(M)^C(F)^G(M)^A(F)^A(M)^ C(M)^C^A(M)^A(M)^G(M)^G(M)^G(M)^G(M)^U(M)^G(M)^G(M)^18^C(M)^C(M)^A (M)^C(M)^C(M)^C(M) | 11108.69 |
| 40 | del03_Am1 | U(M)U(M)C(M)A(M)G(M)CAGCUCGAACCAAGGGGUGGUUCGCCACCU | 11264.97 |
| 41 | del03_Am2 | UUCAGC(M)A(M)G(M)C(M)U(M)CGAACCAAGGGGUGGUUCGCCACCU | 11269.41 |
| 42 | del03_Am3 | UUCAGCAGCUC(M)G(M)A(M)A(M)C(M)CAAGGGGUGGUUCGCCACCU | 11266.40 |
| 43 | del03_Am4 | UUCAGCAGCUCGAACCA(M)A(M)G(M)GGGUGGUUCGCCACCU | 11238.74 |
| 44 | del03_Am5 | UUCAGCAGCUCGAAC(M)CAAGGGGUGGUUCGCCACCU | 11208.73 |
| 45 | del03_Am6 | UUCAGCAGCUCGAACCA(M)AGGGGUGGUUCGCCACCU | 11208.76 |
| 46 | del03_Am7 | UUCAGCAGCUCGAACCAA(M)G(M)GGGUGGUUCGCCACCU | 11222.34 |

**[Table 4]**

| Example | Sequece name | Sequence (5'-3') | Molecular weight | SEQ ID NO: |
|---|---|---|---|---|
| 47 | ASR | ACACGAAAGCAAUGCCAUC | - | 2 |
| | ACTB_26 | A(F)^C(M)^A(F)^C(M)^G(F)^A(M)^A(F)^A(M)^G(F)^C(M)^A(F)^A(M)^U(F)^G(M)^ C(M)^C^A(M)^U(M)^C(M)^G(M)^G(M)^G(M)^U(M)^G(M)^G(M)^18^C(M)^C(M)^A (M)^C(M)^C(M)^U(M) | 11113.01 | - |
| 48 | ASR | CCUCUUCAAGGGGUCCACA | - | 3 |
| | GAPDH_26 | C(F)^C(M)^U(F)^C(M)^U(F)^U(M)^C(F)^A(M)^A(F)^G(M)^G(F)^G(M)^G(F)^U(M) ^C(M)^C^A(M)^C(M)^A(M)^G(M)^G(M)^G(M)^U(M)^G(M)^G(M)^18^C(M)^C(M)^ A(M)^C(M)^C(M)^U(M) | 11061.24 | - |
| 49 | ASR | GCCUCAUACUGCGUGCGGA | - | 4 |
| | GFAP_26 | G(F)^C(M)^C(F)^U(M)^C(F)^A(M)^U(F)^A(M)^C(F)^U(M)^G(F)^C(M)^G(F)^U(M)^ G(M)^C^G(M)^G(M)^A(M)^G(M)^G(M)^G(M)^U(M)^G(M)^G(M)^18^C(M)^C(M)^ A(M)^C(M)^C(M)^U(M) | 11117.4 | - |
| 50 | ASR | CUCAUACUGCGUGCGGA | - | 5 |
| | GFAP_32 | C(L)^U(M)^C(L)^A(M)^U(L)^A(M)^C(L)^U(M)^G(L)^C(M)^G(L)^U(M)^G(M)^C^G( M)^G(M)^A(M)^G(M)^G(M)^G(M)^U(M)^G(M)^G(M)^18^C(M)^C(M)^A(M)^C(M)^ C(M)^U(M) | 10538.45 | - |

"Molecular weight" in the tables indicates an actually measured value according to negative ion ESI mass spectrometry. In "Sequence" in the tables, uppercase letters denote RNA, lowercase letters denote DNA, N(M) denotes 2'-O-methylation of D-ribofuranose, N(F) denotes 2'-deoxy-2'-fluoration of D-ribofuranose, N(L) denotes 2'-O,4'-C-methylenation of D-ribofuranose, and N(E) denotes 2'-O,4'-C-ethylenation of D-ribofuranose. Linkers represented by -O(CH₂)₃O-, -O(CH₂)₆O-, -O(CH₂CH₂O)₃-, and -O(CH₂CH₂O)₆- are denoted by "3", "6", "9", and "18", respectively. Nucleoside units bonded by -P(=S)(OH)- are denoted by "^". Unless otherwise specified, nucleoside units, or a nucleoside unit and a linker, are bonded by -P(=O)(OH)-. The 5'- and 3'-ends of the oligonucleotide are hydroxyl groups in which a hydrogen atom is bonded to an oxygen atom in a chemical formula. The structures of the nucleoside units are shown below. A broken line in a chemical formula indicates a binding site.

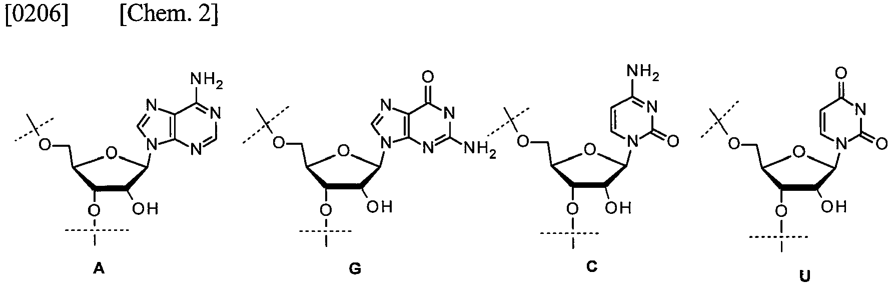

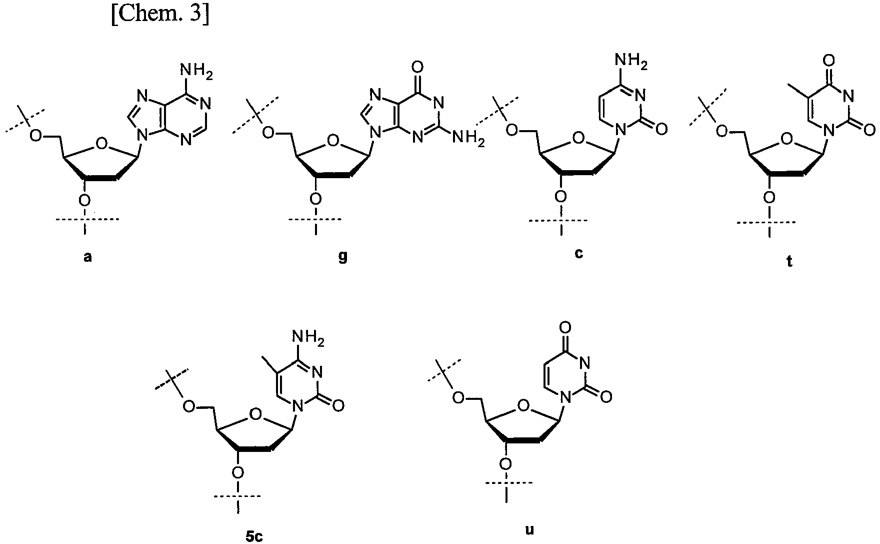

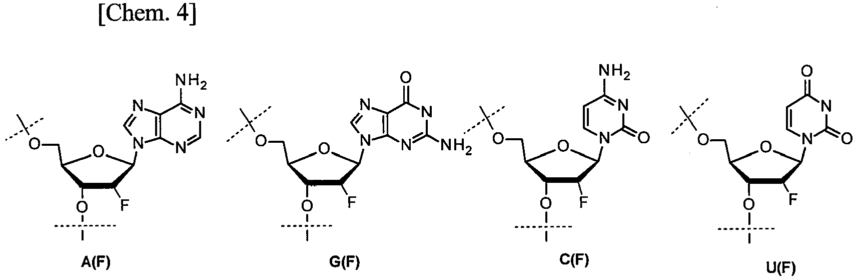

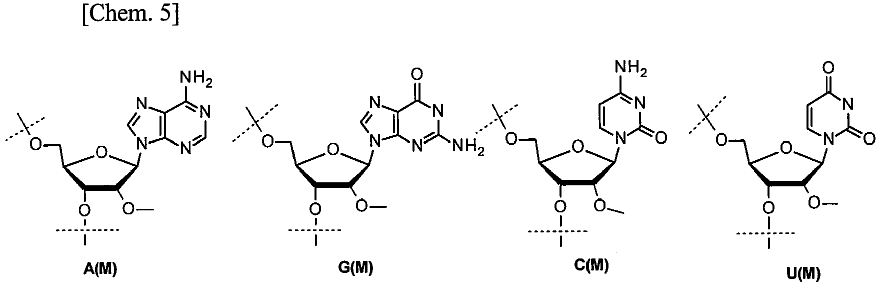

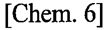

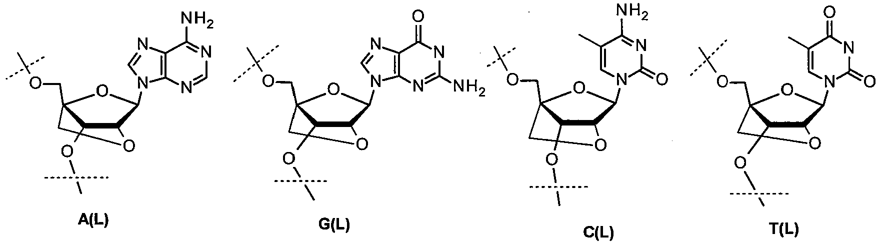

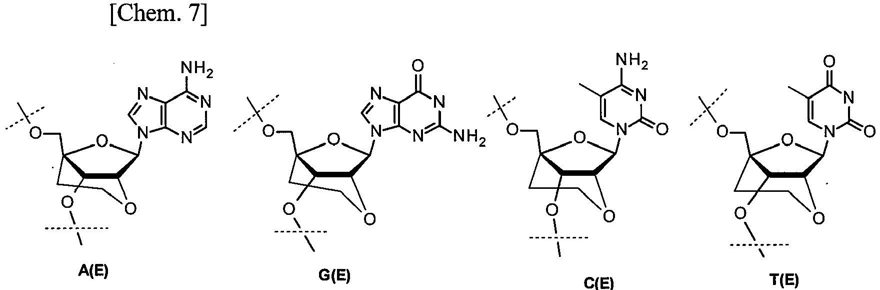

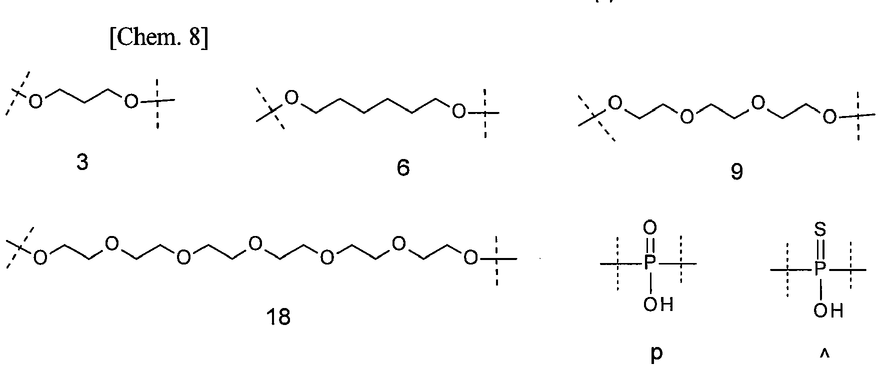

### (Reference Example 2)

An oligonucleotide compound having the base sequence and the state of chemical modification described in Table 5 were synthesized by the phosphoramidite method in the same manner as in Reference Example 1. The symbols in the table are the same as above.

**[Table 5]**

| | Sequence name | Sequence (5'-3') | Molecular weight | SEQ ID NO: |
|---|---|---|---|---|
| Reference Example 2 | del03_22 | CGCGCGTTTTCGCGCGUCGAACCAAGCGGUG | - | 6 |
| | | cgcgcgttttcgcgcgU(M)C(M)G(M)A(M)A(M)CCAA(M)G(M )C(M)G(M)G(M)U(M)G(M) | 9910.67 | - |

### (Test Example 1) Evaluation of Editing Inducibility for Rluc_sRNA (A) Synthesis of Rluc_sRNA

Rluc_WT RNA (SEQ ID NO: 7) prepared by transcription by a conventional method was prepared at 0.2 nM from psiCHECK^{™}-2 Vector (Promega). Recombinant hADAR2 (synthesized and purified by using a yeast expression system, Macbeth, MR. and Bass, BL. Methods Enzymol. 424, 319-331 (2007), Fukuda, M. et al. Sci. Rep. Srep41478 (2017)) was added to a final concentration of 1 µM and incubated at 37 °C for 2 hours in an editing reaction buffer (20 mM HEPES-KOH (pH 7.5), 2 mM MgCl₂, 100 mM NaCl, 0.5 mM DTT, 0.01 % Triton X-100, 5 % glycerol) to perform an in-vitro editing reaction.

Rluc_WT RNA after the editing reaction was purified by phenol/chloroform extraction and ethanol precipitation. Subsequently, cDNA was synthesized with Rluc_WT_BamR01 primer (SEQ ID NO: 8) by using Primescript Reverse Transcriptase II (TaKaRa). The cDNA was then amplified by PCR (30 cycles, denaturation: 98 °C, 10 seconds, annealing: 55 °C, 15 seconds, extension: 68 °C, 60 seconds) using Rluc_WT_EcoF01 primer (SEQ ID NO: 9), Rluc_WT_BamR01 primer (SEQ ID NO: 8), and PrimeStar GXL DNA Polymerase (TaKaRa). Subsequently, the obtained cDNA was cloned as insert DNA into the pUC19 plasmid as follows. The insert DNA and pUC19 plasmid were subjected to restriction enzyme digestion at 37 °C for 1 hour by using EcoRI (TaKaRa) and BamHI (TaKaRa), and respective DNAs were purified by phenol/chloroform extraction and ethanol precipitation. The pUC19 plasmid and the insert DNA after the restriction enzyme digestion were mixed at a molar ratio of 1:3, and a ligation reaction was performed by using the DNA Ligation Kit <Mighty Mix> (TaKaRa). The obtained ligation sample was transformed into DH5α and cultured overnight at 37 °C by using an LB agar plate, and a plasmid was then extracted by using the QIAprep Spin Miniprep Kit (QIAGEN). The base sequence of the obtained plasmid DNA was analyzed to obtain a sequence in which A122 of Rluc_WT was mutated to G (Rluc_K41R) and a plasmid DNA in which Rluc_K41R was cloned (pUC19-Rluc_K41R).

With pUC19-Rluc_K41R used as a template, 1st PCR (30 cycles, denaturation: 98 °C, 10 seconds, annealing: 55 °C, 15 seconds, elongation: 68 °C, 40 seconds) was performed with PrimeStar GXL DNA Polymerase (TaKaRa) by using Rluc_NheF01 primer (SEQ ID NO: 10) and RL_W104X_RV primer (SEQ ID NO: 11) for the 5'-side fragment and Rluc_XhoR01 primer (SEQ ID NO: 12) and RL_W104X_FW primer (SEQ ID NO: 13) for the 3'-side fragment. Subsequently, respective PCR products were diluted 100-fold, subjected to 2nd PCR (30 cycles, denaturation: 98 °C, 10 seconds, annealing: 55 °C, 15 seconds, extension: 68 °C, 60 seconds) with PrimeStar GXL DNA Polymerase (TaKaRa) by using Rluc_NheF01 primer and Rluc_XhoR01 primer, and purified by phenol/chloroform extraction and ethanol precipitation to obtain DNA (Rluc_K41R_W104X) (SEQ ID NO: 16) having a sequence in which G311 of Rluc_K41R was mutated to A.

The obtained DNA (Rluc_K41R_W104X) and psiCHECKTM-2 Vector (promega) were subjected to restriction enzyme digestion at 37 °C for 1 hour by using NheI (TaKaRa) and XhoI (TaKaRa), and respective DNAs were purified by phenol/chloroform extraction and ethanol precipitation. The Rluc_K41R_W104X and psiCHECKTM-2 Vector after the restriction enzyme digestion were mixed at a molar ratio of 3: 1, and a ligation reaction was performed by using the DNA Ligation Kit <Mighty Mix> (TaKaRa). The obtained ligation sample was transformed into DH5α and cultured overnight at 37 °C by using an LB agar plate. Selected colonies were cultured overnight at 37 °C in the LB liquid medium, and the plasmid was extracted by using a QIAprep Spin Miniprep Kit (QIAGEN). Subsequently, the sequence of the plasmid obtained by the base sequence analysis was confirmed.

With the sequence-confirmed plasmid used as a template, template DNA for in vitro transcription reaction was amplified by PCR (30 cycles, denaturation: 98 °C, 10 seconds, annealing: 55 °C, 15 seconds, elongation: 68 °C, 20 seconds) using T7_Rluc_sRNA_F01 primer (SEQ ID NO: 14), Rluc_sRNA_R01 primer (SEQ ID NO: 15), and PrimeStar GXL DNA Polymerase (TaKaRa) and was purified by phenol/chloroform extraction and ethanol precipitation. Rluc_sRNA (SEQ ID NO: 17) was obtained by performing in vitro transcription using the AmpliScribe T7 Kit (Epicenter Biotechnology). The synthesized Rluc_sRNA was purified by using a 5 % polyacrylamide gel containing 8M urea and used for the subsequent tests.

The sequences of Rluc_WT_RNA, Rluc_K41R_W104X, and Rluc_sRNA, and the sequences of the primers used above are listed below. The underlines in the table indicate the adenosine residues of the editing targets.

**[Table 6]**

| | Sequence | SEQ ID NO: |
|---|---|---|
| Rluc_WT_RNA | ACCAUGGCUUCCAAGGUGUACGACCCCGAGCAACGCAAACGCAUGAUCACUGG GCCUCAGUGGUGGGCUCGCUGCAAGCAAAUGAACGUGCUGGACUCCUUCAUCA ACUACUAUGAUUCCGAGAAGCACGCCGAGAACGCCGUGAUUUUUCUGCAUGGU AACGCUGCCUCCAGCUACCUGUGGAGGCACGUCGUGCCUCACAUCGAGCCCGU GGCUAGAUGCAUCAUCCCUGAUCUGAUCGGAAUGGGUAAGUCCGGCAAGAGCG GGAAUGGCUCAUAUCGCCUCCUGGAUCACUACAAGUACCUCACCGCUUGGUUC GAGCUGCUGAACCUUCCAAAGAAAAUCAUCUUUGUGGGCCACGACUGGGGGGC UUGUCUGGCCUUUCACUACUCCUACGAGCACCAAGACAAGAUCAAGGCCAUCG UCCAUGCUGAGAGUGUCGUGGACGUGAUCGAGUCCUGGGACGAGUGGCCUGA CAUCGAGGAGGAUAUCGCCCUGAUCAAGAGCGAAGAGGGCGAGAAAAUGGUG CUUGAGAAUAACUUCUUCGUCGAGACCAUGCUCCCAAGCAAGAUCAUGCGGAA ACUGGAGCCUGAGGAGUUCGCUGCCUACCUGGAGCCAUUCAAGGAGAAGGGC GAGGUUAGACGGCCUACCCUCUCCUGGCCUCGCGAGAUCCCUCUCGUUAAGGG AGGCAAGCCCGACGUCGUCCAGAUUGUCCGCAACUACAACGCCUACCUUCGGG CCAGCGACGAUCUGCCUAAGAUGUUCAUCGAGUCCGACCCUGGGUUCUUUUCC AACGCUAUUGUCGAGGGAGCUAAGAAGUUCCCUAACACCGAGUUCGUGAAGGU GAAGGGCCUCCACUUCAGCCAGGAGGACGCUCCAGAUGAAAUGGGUAAGUACA UCAAGAGCUUCG | 7 |

**[Table 7]**

| Primer | Sequence | SEQ ID NO: |
|---|---|---|
| Rluc_WT_BamR01 | GAAGGATCCTTACAGGTCCTCCTCTGA GATCAGCTTCTGCTC | 8 |
| Rluc_WT_EcoF01 | GCAGAATTCACCATGGCTTCCAAGGTG TACGAC | 9 |
| Rluc_NheF01 | GACGCTAGCACCATGGCTTCCAAGGT GTACGAC | 10 |
| RL_W104X_RV | CAGCTCGAACTAAGCGGTGAG | 11 |
| Rluc_XhoR01 | GAACTCGAGTTACAGGTCCTCCTCTGA GATCAGCTTCTGCTC | 12 |
| RL_W104X_FW | CTCACCGCTTAGTTCGAGCTG | 13 |
| T7_Rluc_sRNA_F01 | CTAATACGACTCACTATAGGGCTGGAC TCCTTCATCAAC | 14 |
| Rluc_sRNA_R01 | CCAGTCGTGGCCCACAAAG | 15 |

**[Table 8]**

| | Sequence | SEQ ID NO: |
|---|---|---|
| Rluc_K41R_W104X | ATGGCTTCCAAGGTGTACGACCCCGAGCAACGCAAACGCATGATCACTGGGC CTCAGTGGTGGGCTCGCTGCAAGCAAATGAACGTGCTGGACTCCTTCATCAAC TACTATGATTCCGAGAGGCACGCCGAGAACGCCGTGATTTTTCTGCATGGTAA CGCTGCCTCCAGCTACCTGTGGAGGCACGTCGTGCCTCACATCGAGCCCGTG GCTAGATGCATCATCCCTGATCTGATCGGAATGGGTAAGTCCGGCAAGAGCG GGAATGGCTCATATCGCCTCCTGGATCACTACAAGTACCTCACCGCTTAGTTC GAGCTGCTGAACCTTCCAAAGAAAATCATCTTTGTGGGCCACGACTGGGGGG CTTGTCTGGCCTTTCACTACTCCTACGAGCACCAAGACAAGATCAAGGCCATC GTCCATGCTGAGAGTGTCGTGGACGTGATCGAGTCCTGGGACGAGTGGCCTG ACATCGAGGAGGATATCGCCCTGATCAAGAGCGAAGAGGGCGAGAAAATGG TGCTTGAGAATAACTTCTTCGTCGAGACCATGCTCCCAAGCAAGATCATGCGG AAACTGGAGCCTGAGGAGTTCGCTGCCTACCTGGAGCCATTCAAGGAGAAGG GCGAGGTTAGACGGCCTACCCTCTCCTGGCCTCGCGAGATCCCTCTCGTTAAG GGAGGCAAGCCCGACGTCGTCCAGATTGTCCGCAACTACAACGCCTACCTTC GGGCCAGCGACGATCTGCCTAAGATGTTCATCGAGTCCGACCCTGGGTTCTTT TCCAACGCTATTGTCGAGGGAGCTAAGAAGTTCCCTAACACCGAGTTCGTGAA GGTGAAGGGCCTCCACTTCAGCCAGGAGGACGCTCCAGATGAAATGGGTAA GTACATCAAGAGCTTCGTGGAGCGCGTGCTGAAGAACGAGCAGTAA | 16 |
| Rluc_sRNA | GGGCUGGACUCCUUCAUCAACUACUAUGAUUCCGAGAGGCACGCCGAGAA CGCCGUGAUUUUUCUGCAUGGUAACGCUGCCUCCAGCUACCUGUGGAGGC ACGUCGUGCCUCACAUCGAGCCCGUGGCUAGAUGCAUCAUCCCUGAUCUG AUCGGAAUGGGUAAGUCCGGCAAGAGCGGGAAUGGCUCAUAUCGCCUCCU GGAUCACUACAAGUACCUCACCGCUUAGUUCGAGCUGCUGAACCUUCCAAA GAAAAUCAUCUUUGUGGGCCACGACUGG | 17 |

### (B) Annealing Reaction

In an annealing buffer (10 mM Tri-HCl (pH 7.6), 150 mM NaCl), 0.3 µM Rluc_sRNA and a 0.9 µM compound of Examples and Reference Example (hereinafter, also referred to as gRNA) were heated at 80 °C for 3 minutes and cooled to 25 °C over 15 minutes.

### (C) In Vitro Editing Reaction

Assuming that Rluc_sRNA completely formed a complex with gRNA through the annealing reaction, the following Rluc_sRNA-gRNA complex concentration was calculated. The editing reaction was performed in an editing reaction buffer (20 mM HEPES-KOH (pH 7.5), 2 mM MgCl₂, 100 mM NaCl, 0.5 mM DTT, 0.01 % Triton X-100, 5 % glycerol) by adding 12.5 nM or 6.25 nM recombinant hADAR2 to a 5 nM Rluc_sRNA-gRNA complex and incubating the mixture at 37 °C for 1 hour.

### (D) Editing Analysis Method

Rluc_sRNA after the editing reaction was purified by phenol/chloroform extraction and ethanol precipitation, and cDNA was synthesized with Rluc_sRNA_R01 primer (SEQ ID NO: 18) by using Primescript Reverse Transcriptase II (TaKaRa). The cDNA was then amplified by PCR (denaturation: 98 °C, 10 seconds, annealing: 55 °C, 15 seconds, elongation: 68 °C, 20 seconds) using Rluc_sRNA_F01 primer (SEQ ID NO: 18) and Rluc_sRNA_R01 primer (SEQ ID NO: 15), and PrimeStar GXL DNA Polymerase (TaKaRa). For the sequence analysis of the obtained cDNA, a sequencing reaction was performed by using Rluc_sRNA_F01 primer (SEQ ID NO: 18) and Big Dye Terminator v3.1 Cycle Sequence Kit, and the sequence was analyzed by Applied Biosystem 3500 Genetic Analyzer (Genetic Science). From the chromatographic chart obtained by sequencing, an editing rate (%) was calculated from a peak height ratio (G/(G+A)) of the target site (A311).

**[Table 9]**

| Primer | Sequence | SEQ ID NO: |
|---|---|---|
| Rluc_sRNA_F01 | GCTGGACTCCTTCATCAAC | 18 |
| Rluc_sRNA_R01 | CCAGTCGTGGCCCACAAAG | 15 |

### (E) Results of Editing Analysis

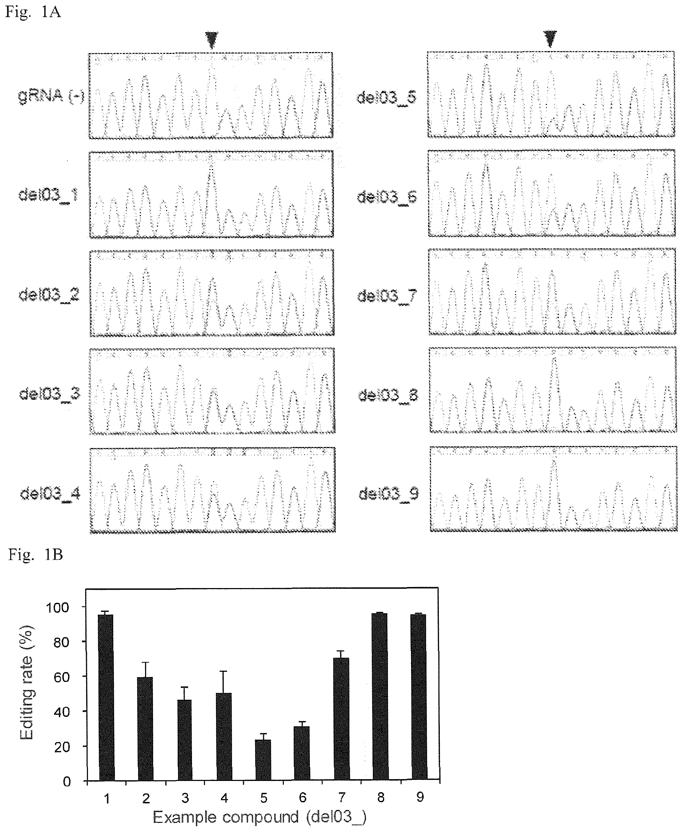

The editing rate (%) in the case of using the compound of Reference Example 1 and the compounds of Example is shown in Figs. 1A and 1B. The compound of Reference Example 1 exhibited an editing rate more than 90 %. Even when a modified nucleic acid was introduced as in the compounds of Examples (del03_02 to del03_07), the editing-inducing activity was exhibited in the same manner as the compound of Reference Example 1. The compound having a phosphorothioate bond introduced to C corresponding to the target editing site (del03_08) and the compound having the loop of the ADAR induction region replaced with a linker composed of hexaethylene glycol (del03_09) also exhibited the same level of the editing-inducing activity as the compound of Reference Example 1.

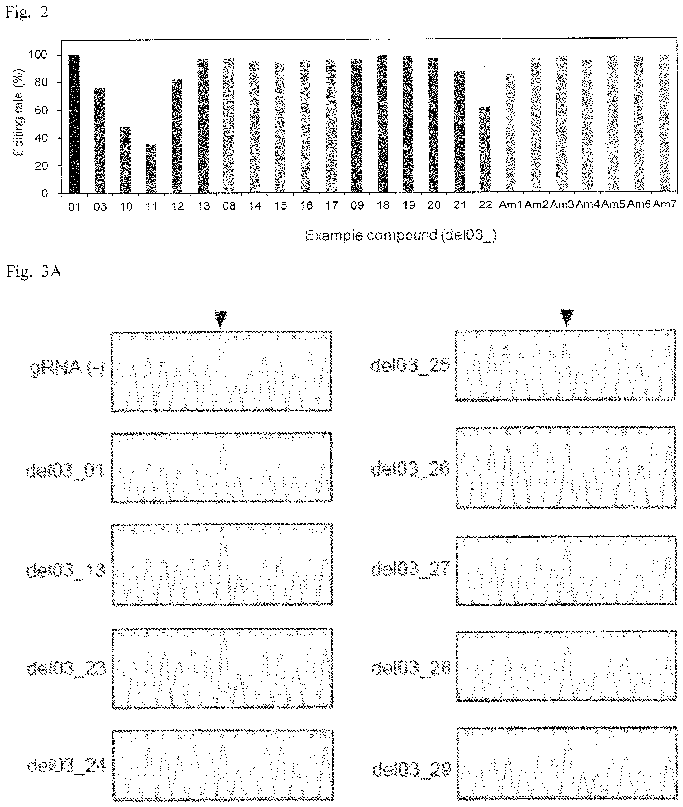

As shown in Fig. 2, even when a modified nucleic acid was introduced as in the compounds of Examples (del03_10 to del03_21), the editing-inducing activity was exhibited in the same manner as the compound of Reference Example 1.

The compound of Reference Example 2 (del03_22) has a stem-loop sequence of Z-type DNA and is acquired by adapting the gRNA design method described in WO2016/097212 in accordance with the Rluc_sRNA sequence. It can be seen that del03_22 has a weaker editing-inducing activity than the compound of Example (del03_21) having the same Z-type DNA stem sequence. On the other hand, even when a modified nucleic acid was introduced as in the compounds of Examples (del03_Am1 to del03_Am7), the same level of the editing-inducing activity as the compound of Reference Example 1 was exhibited.

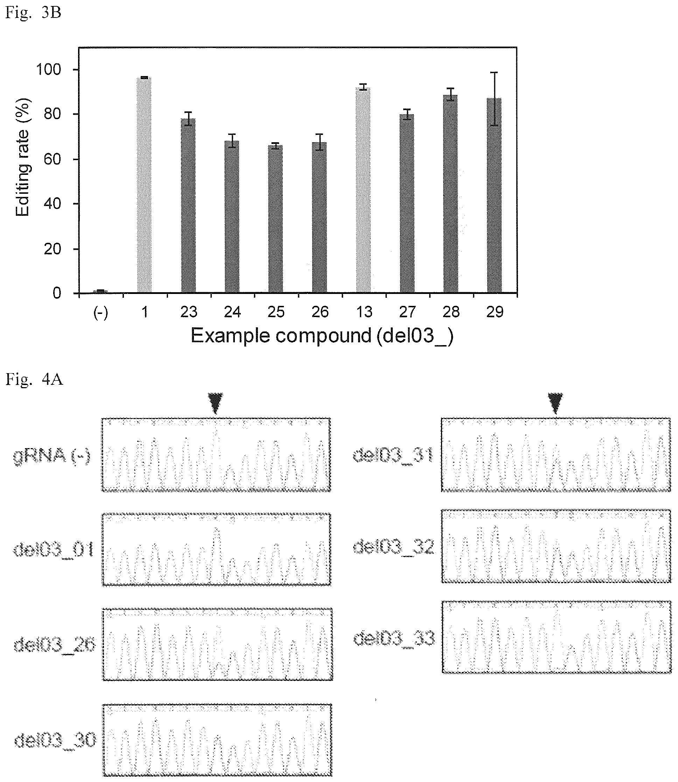

As shown in Figs. 3A and 3B, even when a modified nucleic acid was introduced as in the compounds of Examples (del03_23 to del03_29), the same editing-inducing activity as the compound of Reference Example 1 was exhibited.

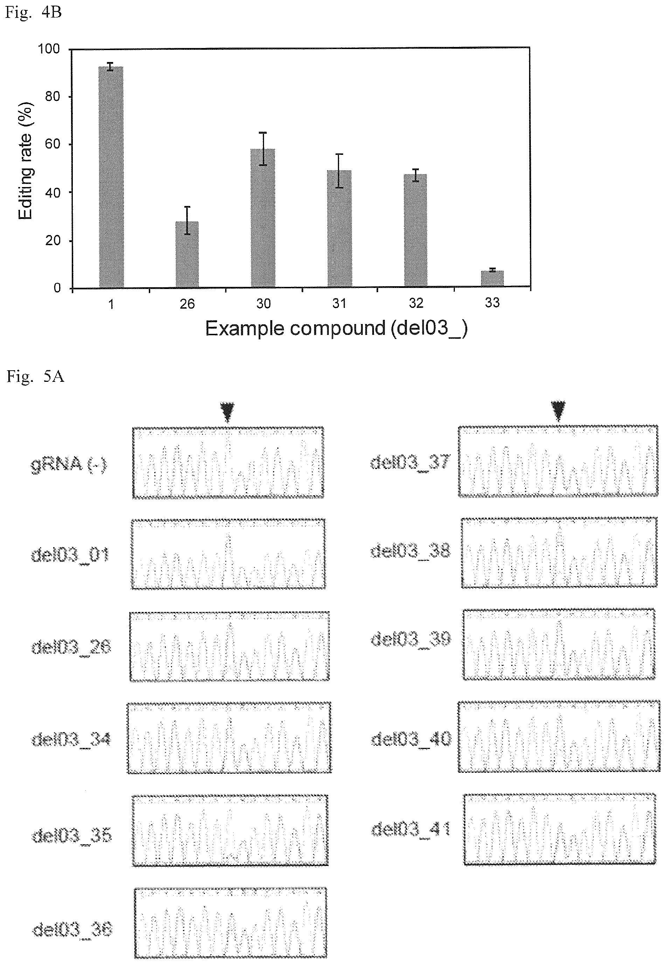

As shown in Figs. 4A and 4B, even when a modified nucleic acid was introduced as in the compounds of Examples (del03_26, del03_30 to del03_32), the same editing-inducing activity as the compound of Reference Example 1 was exhibited.

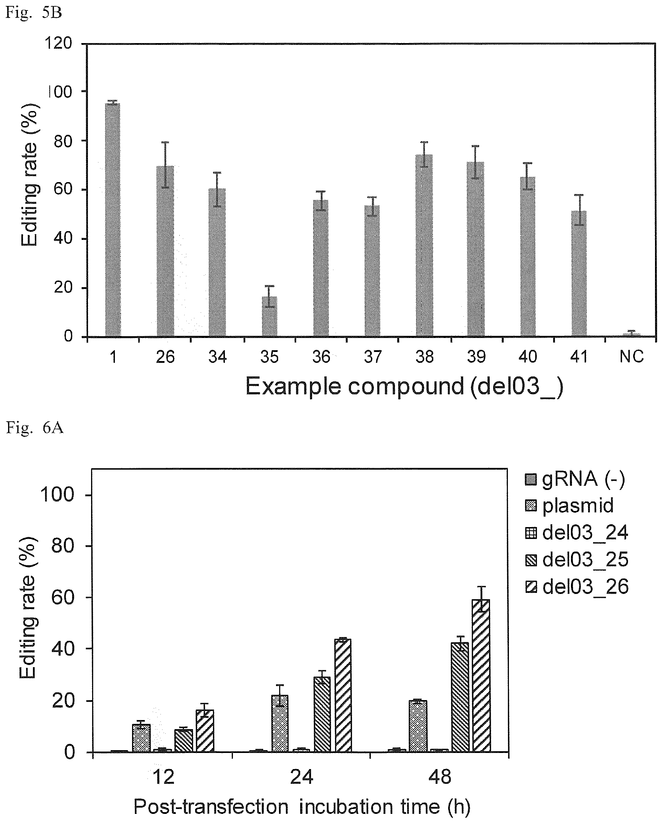

As shown in Figs. 5A and 5B, even when a modified nucleic acid was introduced as in the compounds of Examples (del03_26, del03_34 to del03_41), the editing-inducing activity was exhibited in the same manner as the compound of Reference Example 1.

### (Test Example 2)

### Evaluation of Editing Inducibility in Cultured Cells

### (A) Cell Culture

HeLa cells were subcultured on a 24-well plate at 5.0×10⁵ cells/well for 48 hours. Lipofectamine 3000 (Thermo) was used for transfecting 50 ng of psiCHECK2^{™}_Rluc_K41R_W104X, 350 ng of pcDNA3.1(-) Hygro_ADAR2, 10 nM of gRNA that is a compound of Example or Reference Example. As a control, 100 ng of a plasmid expressing gRNA (described in Reference Example 2 of Japanese Patent Application No. 2017-234341) was transfected and used.

### (B) Editing Analysis Method

Total RNA was extracted from cells cultured in the 24-well Plate by using Sepasol RNA I Super G (nacalai), subjected to a DNase treatment by using Recombinant DNase I (TaKaRa), then purified by phenol/chloroform extraction and ethanol precipitation. A reverse transcription reaction was performed by using PrimeScript II Reverse Transcriptase (TaKaRa), 0.5 µg of Total RNA, and 0.25 µM Oligo (dT) 17 (SEQ ID NO: 19) to amplify cDNA. PrimeStar GXL DNA polymerase (TaKaRa), Rluc_F01 primer (SEQ ID NO: 20), and 3'-Adp primer (SEQ ID NO: 21) were used to perform 1st PCR (30 cycles (denaturation: 98 °C, 10 seconds, annealing: 55 °C, 15 seconds, elongation: 68 °C, 60 seconds)). With the cDNA obtained by diluting the 1st PCR product 200 times as a template, 2nd PCR (30 cycles (denaturation: 98 °C, 10 seconds, annealing: 55 °C, 15 seconds, elongation: 68 °C, 60 seconds))) using PrimeStar GXL DNA polymerase (TaKaRa), Rluc_F01 primer (SEQ ID NO: 20), and Rluc_R01 primer (SEQ ID NO: 22) was performed to amplify the Rluc fragment. A sequencing reaction was performed by using Big Dye Terminator v3.1 Cycle Sequence Kit (Thermo Fisher Scientific) and 0.165 µM Rluc_sRNA_F01 primer (SEQ ID NO: 18), and the analysis was performed by Applied Biosystems 3500 Genetic Analyzer (Thermo Fisher Scientific). From the chromatographic chart obtained by sequencing, an editing rate (%) was calculated from a peak height ratio (G/(G+A)) of the target site (A311).

**[Table 10]**

| Primer | Sequence | SEQ ID NO: |
|---|---|---|
| Oligo(dT)17 | GGCCACGCGTCGACTAGTACTTTTTTT TTTTTTTTTT | 19 |
| Rluc_F01 | CTAATACGACTCACTATAGGGACCATG GCTTCCAAGGTGTAC | 20 |
| 3'-Adp | GGCCACGCGTCGACTAGTAC | 21 |
| Rluc_R01 | TTACTGCTCGTTCTTCAGCACG | 22 |
| Rluc_sRNA_F01 | GCTGGACTCCTTCATCAAC | 18 |

### (C) Luciferase Reporter Assay Method

Dual-Luciferase Reporter Assay System (Promega) was used. A cell extract was obtained by using 100 µL of Passive Lysis Buffer (Promega) for cells cultured on a 24-well Plate. After 60 seconds from addition of 100 µL of LARII to 20 µL of the obtained cell extract, the emission intensity of Firefly luciferase (Fluc) was measured by GloMax^{(R)} 20/20 Luminometer (Promega). Immediately after that, 100 µL of Stop & Glo Reagent was added, and 60 seconds later, the emission intensity of Renilla luciferase (Rluc) was measured. The emission intensity was normalized by Fluc.

### (D) Results of Editing Analysis and Luciferase Reporter Assay

The editing rate (%) in the case of using the compounds of Examples (del03_24 to del03_26) is shown in Fig. 6A. When the plasmid was transfected, the editing rate was about 10 to 20 %. When a modified nucleic acid with an increased number of phosphorothioate bonds as in the compounds of Examples (del03_25 and del03_26) was transfected, the edit-inducing activity was recognized, and the rate thereof was higher than when the plasmid was transfected.

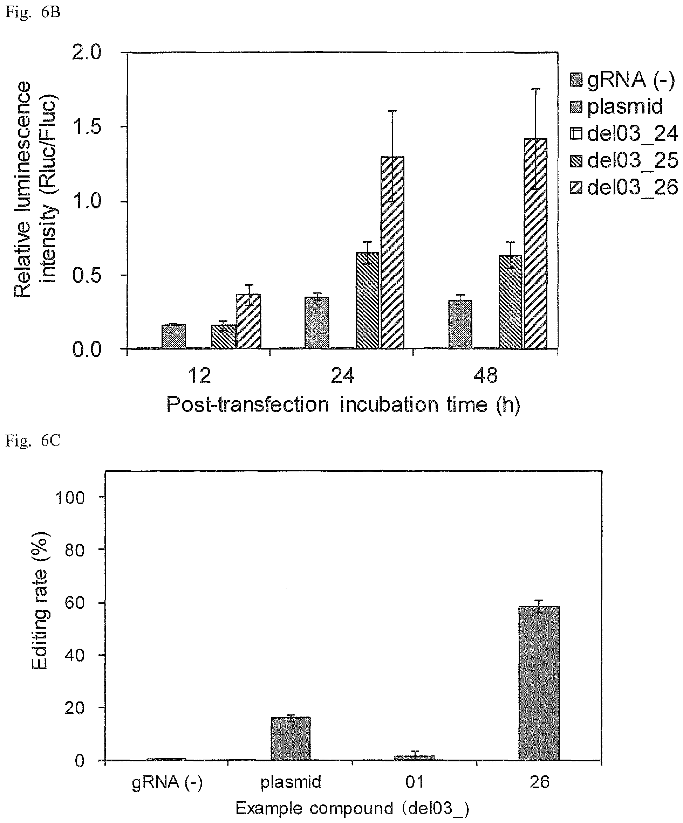

Results of a luciferase reporter assay using the compounds of Examples (del03_24 to del03_26) are shown in Fig. 6B. When the compounds of Examples (del03_25 and del03_26) were used, high luciferase activity equal to or higher than the case of transfecting the plasmid was recognized.

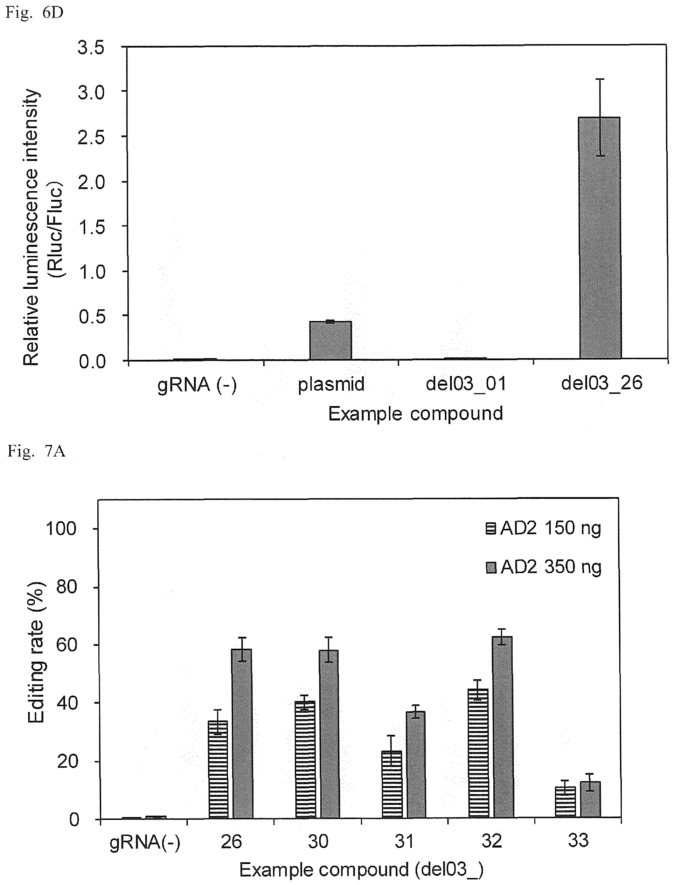

The editing rate (%) in the case of using the compound of Reference Example 1 (del03_01) and the compound of Example (del03_26) is shown in Fig. 6C. When the plasmid was transfected, the editing rate was about 20 %. When the compound of Reference Example 1 (del03_01) was transfected, no editing-inducing activity was recognized. However, when the compound of Example (del03_26) was transfected, high editing-inducing activity was recognized. The results of the luciferase reporter assay in the case of using the compound of Reference Example 1 (del03_01) and the compound of Example (del03_26) are shown in Fig. 6D. Although no luciferase activity was recognized when the compound of Reference Example (del03_01) was used, much higher luciferase activity was recognized when the compound of Example (del03_26) was used as compared to when the plasmid was transfected.

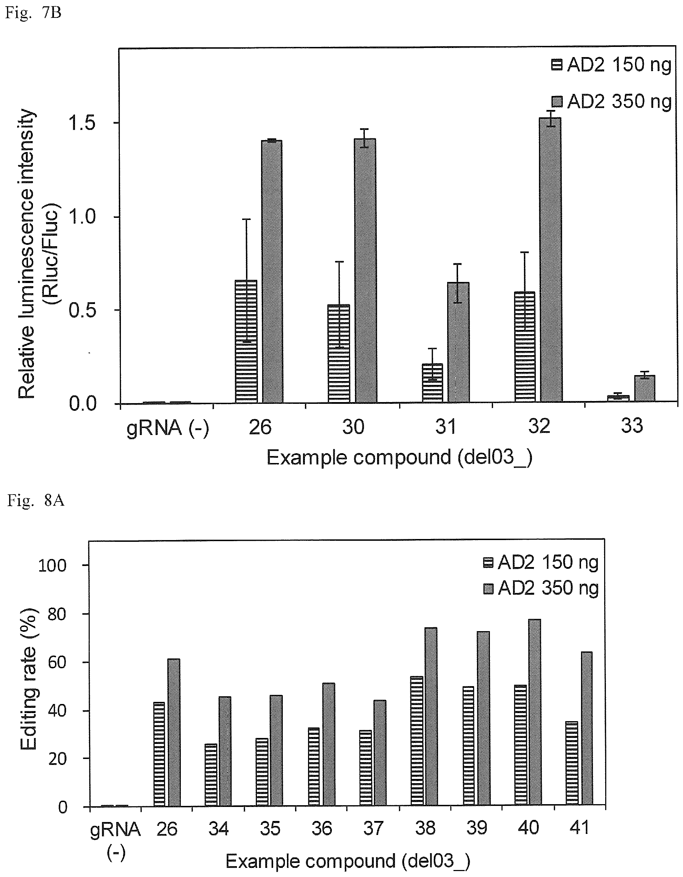

The editing rate (%) in the case of using the compounds of Examples (del03_26, del03_30 to del03_33) is shown in Fig. 7A. When the compounds of Examples (del03_30 to del03_32) were transfected, the same level of the editing-inducing activity as in the case of transfecting the compound of the example described above (del03_26) was recognized. The results of the luciferase reporter assay in the case of using the compounds of Examples (del03_26, del03_30 to del03_33) are shown in Fig. 7B. When the compounds of Examples (del03_30 to del03_32) were transfected, the same level of high luciferase activity as in the case of transfecting the compound of Example described above (del03_26) was observed.

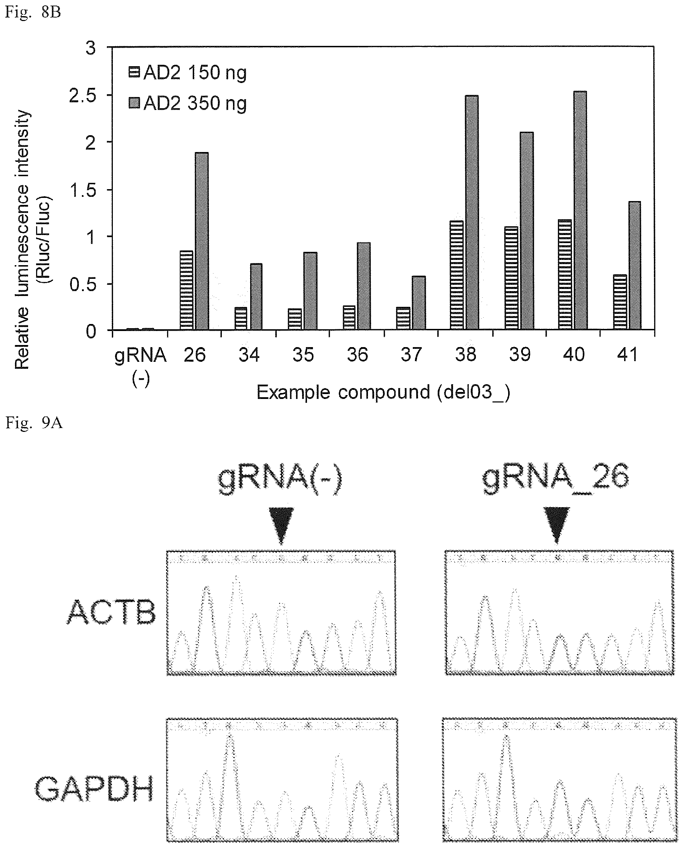

The editing rate (%) in the case of using the compounds of Examples (del03_26, del03_34 to del03_41) is shown in Fig. 8A. When the compounds of Examples (del03_34 to del03_41) were transfected, the editing-inducing activity equal to or higher than the case of transfecting the compound of Example described above (del03_26) was recognized. The results of the luciferase reporter assay in the case of using the compounds of Examples (del03_26, del03_30 to del03_33) are shown in Fig. 8B. When the compounds of Examples (del03_34 to del03_41) was transfected, the same level of high luciferase activity as in the case of transfecting the compound of the example described above (del03_26) was observed.

### (Test Example 3)

### Evaluation of Editing Inducibility for RNA Having β-Actin Gene Sequence

### (A) Synthesis of RNA

An annealing reaction (heated at 80 °C for 3 minutes and cooled to 25 °C over 15 minutes) was performed by using oligo DNAs of ACTB_FW (SEQ ID NO: 23) and ACTB_RV (SEQ ID NO: 24) and NEBuffer 2 (NEB) to prepare an insert. By using EcoRI (TaKaRa) and HindIII (TaKaRa), pUC19 was reacted at 37 °C for 1 hour and then purified by phenol/chloroform extraction and ethanol precipitation. The insert and the plasmid were added to achieve a molar ratio of 3:1, and a ligation reaction was performed by using DNA Ligation Kit <Mighty Mix> (TaKaRa). The obtained ligation sample was transformed into DH5α and cultured overnight at 37 °C by using an LB agar plate. Selected colonies were cultured overnight at 37 °C in the LB liquid medium, and the plasmid was extracted by using QIAprep Spin Miniprep Kit (QIAGEN). The sequencing reaction was performed for 100 ng of the obtained plasmid by using Big Dye Terminator v3.1 Cycle Sequence Kit (Thermo Fisher Scientific), 0.165 µM pUC19_seqFW primer (SEQ ID NO: 25), and 0.165 µM pUC19_seqRV primer (SEQ ID NO: 26), and the sequence analysis was performed by Applied Biosystems 3500 Genetic Analyzer (Thermo Fisher Scientific). With the correctly prepared plasmid used as a template, template DNA was prepared by PCR (denaturation: 98 °C, 10 seconds, annealing: 55 °C, 15 seconds, elongation: 68 °C, 20 seconds) using T7_pUC19_FW (SEQ ID NO: 27) and M13_RV primers (SEQ ID NO: 28), and PrimeStar GXL DNA Polymerase (TaKaRa) and purified by phenol/chloroform extraction and ethanol precipitation. In vitro transcription was performed by using AmpliScribe T7 Kit (Epicenter Biotechnology), and purification were performed by using a 5 % polyacrylamide gel containing 8M urea.

**[Table 11]**

| Oligo DNA | Sequence | SEQ ID NO: |
|---|---|---|
| ACTB_FW | AATTCCCCAAGTCCACACAGGGGAGGTGATAGCA TTGCTTTCGTGTAAATTATGTAA | 23 |
| ACTB_RV | AGCTTTACATAATTTACACGAAAGCAATGCTATCA CCTCCCCTGTGTGGACTTGGGG | 24 |

**[Table 12]**

| Primer | Sequence | SEQ ID NO: |
|---|---|---|
| pUC19_seqFW | CAACTGTTGGGAAGGGCGATC | 25 |
| pUC19_seqRV | CGACAGGTTTCCCGACTGGAAAG | 26 |
| T7_pUC19_FW | CTAATACGACTCACTATAGGGCCTCTT CGCTATTACGCCAG | 27 |
| M13_RV | GGAAACAGCTATGACCATGATTAC | 28 |
| T7proGGG | CTAATACGACTCACTATAGGG | 29 |

### (B) Evaluation of Editing Inducibility and Results

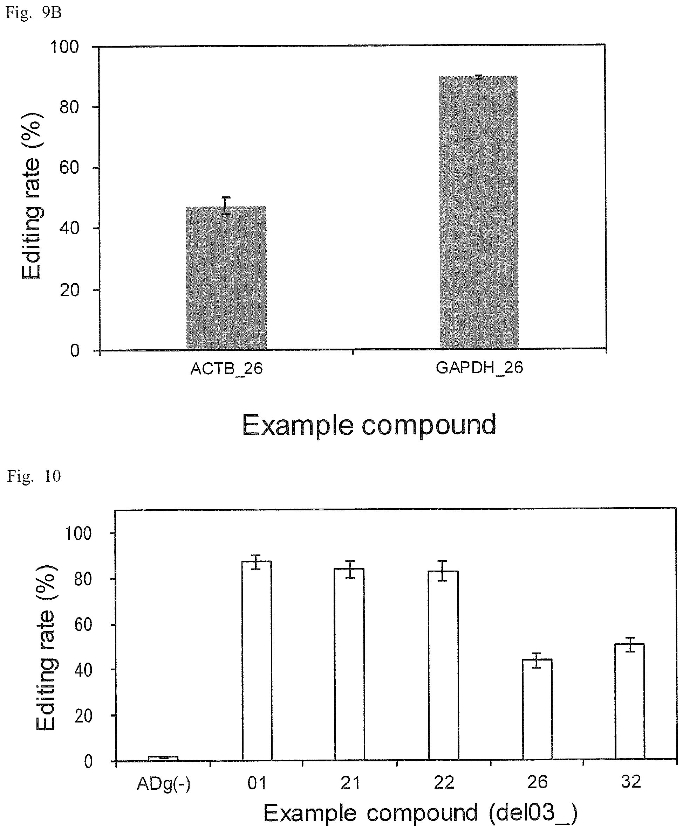

For the method of evaluating the editing inducibility of the compound of Example (ACTB_26), the same method as in (B) to (D) of Test Example 1 was used. However, the primers described below were used. The editing rate (%) in the case of using the compound of Example (ACTB_26) is shown in Figs. 9A and 9B. As a result, the compound of Example (ACTB_26) exhibited an editing rate of about 50 %.

**[Table 13]**

| Primer | Sequence | SEQ ID NO: |
|---|---|---|
| T7_pUC19_FW | CTAATACGACTCACTATAGGGCCTCTT CGCTATTACGCCAG | 27 |
| M13_RV | GGAAACAGCTATGACCATGATTAC | 28 |
| T7proGGG | CTAATACGACTCACTATAGGG | 29 |

### (Test Example 4) Evaluation of Editing Inducibility for RNA Having GAPDH Gene Sequence

### (A) Synthesis of RNA

The operation was performed in the same manner as the method described in (A) Synthesis of RNA of Test Example 3. However, the oligo DNAs described below were used.

**[Table 14]**

| Oligo DNA | Sequence | SEQ ID NO: |
|---|---|---|
| GAPDH_FW | AATTCTCTCCCCTCCTCACAGTTGCCATGTAGACC CCTTGAAGAGGGGAGGGGCCTA | 30 |
| GAPDH_RV | AGCTTAGGCCCCTCCCCTCTTCAAGGGGTCTACAT GGCAACTGTGAGGAGGGGAGAG | 31 |

### (B) Evaluation of Editing Inducibility and Results

For the method of evaluating the editing inducibility of the compound of Example (GAPDH_26), the same method as in "(B) Evaluation of Editing Inducibility and Results" in Test Example 3 was used. The editing rate (%) in the case of using the compound of Example (GAPDH_26) is shown in Figs. 9A and 9B. As a result, the compound of Example (GAPDH_26) exhibited an editing rate of about 90 %.

### (Test Example 5) Evaluation of Editing Inducibility for RNA Having GFAP gene sequence

### (A) Synthesis of Target RNA

The operation was performed in the same manner as the method described in (A) Synthesis of RNA of Test Example 3. However, the oligo DNAs described below were used.

**[Table 15]**

| Oligo DNA | Sequence | SEQ ID NO: |
|---|---|---|
| GFAP_FW | AATTCCTCACCGCAGCCCTGAAAGAGATCCACACG CAGTATGAGGCAATGGCGTCCA | 32 |
| GFAP_RV | AGCTTGGACGCCATTGCCTCATACTGCGTGTGGAT CTCTTTCAGGGCTGCGGTGAGG | 33 |

### (B) Synthesis of Target-Editing Guide RNA (gRNA)

For GFAP_del03 (SEQ ID NO: 38), an annealing reaction (in annealing buffer (10 mM Tri-HCl (pH 7.6), 150 mM NaCl), heating at 80 °C for 3 minutes and cooling to 25 °C over 15 minutes) was performed by using oligo DNAs of GFAP_del03_RV (SEQ ID NO: 34) and T7proGGG (SEQ ID NO: 29) to prepare a template DNA for in vitro transcription. For 5'AS_GFAP_gRNA (SEQ ID NO: 37), a reaction was performed at 25 °C for 30 minutes by using 5'AS_GFAP_FW (SEQ ID NO: 35), 5'AS_ADg_RV oligo DNA (SEQ ID NO: 36) and DNA Polymerase I, Large (Klenow) Fragment (NEB) to prepare template DNA. Subsequently, purification was performed by phenol/chloroform extraction and ethanol precipitation. In vitro transcription was performed by using 1.0 µg of each template DNA and AmpliScribe T7 Kit (Epicenter Biotechnology), and purification were performed by using an 8 % polyacrylamide gel containing 8M urea.

**[Table 16]**

| | Sequence | SEQ ID NO: |
|---|---|---|
| GFAP_del03_RV | AGGTGGCGAACCACCCTCCGCACGCAGTATG AGGCCTATAGTGAGTCGTATTAG | 34 |
| 5'AS_GFAP_FW | CTAATACGACTCACTATAGGCCTCATACTGCG TGCGGAGGGTGGAATAGTATAACAATATG | 35 |
| 5'AS_ADg_RV | AGGTGGGATACTATAACAACATTTAGCATATTG TTATACTATTCCACCC | 36 |

**[Table 17]**

| | Sequence | SEQ ID NO: |
|---|---|---|
| GFAP_del03 | GGCCUCAUACUGCGUGCGGAGGGUGGUUCG CCACCU | 38 |
| 5'AS_GFAP_gRNA | GGCCUCAUACUGCGUGCGGAGGGUGGAAUAG UAUAACAAUAUGCUAAAUGUUGUUAUAGUAUC CCACCU | 37 |

### (C) Evaluation of Editing Inducibility

For the method of evaluating the editing inducibility of the compound of Example (GFAP_26), the same method as in "(B) Evaluation of Editing Inducibility and Results" in Test Example 3 was used.

### (Test Example 6) Evaluation of Editing Inducibility for Rluc_sRNA by hADAR1 p110

### (A) Annealing Reaction

In the annealing buffer (10 mM Tri-HCl (pH 7.6), 150 mM NaCl), 0.3 µM Rluc_sRNA and a 0.9 µM compound of Examples and Reference Example (gRNA) were heated at 80 °C for 3 minutes and cooled to 25 °C over 15 minutes.

### (B) In Vitro Editing Reaction

Assuming that Rluc_sRNA completely formed a complex with gRNA through the annealing reaction, the following Rluc_sRNA-gRNA complex concentration was calculated. The editing reaction was performed in an editing reaction buffer (20 mM HEPES-KOH (pH 7.5), 2 mM MgCl₂, 100 mM NaCl, 0.5 mM DTT, 0.01 % Triton X-100, 5 % glycerol) by adding recombinant hADAR1 p110 (synthesized and purified using a yeast expression system. See Macbeth, MR. and Bass, BL. Methods Enzymol. 424, 319-331 (2007); Fukuda, M. et al. Sci. Rep. Srep41478 (2017)) to the 5 nM Rluc_sRNA-gRNA complex to a final concentration of 250 nM and incubating the mixture at 37 °C for 30 minutes.

### (C) Editing Analysis Method

Rluc_sRNA after the editing reaction was purified by phenol/chloroform extraction and ethanol precipitation, and cDNA was synthesized with the Rluc_sRNA_R01 primer by using Primescript Reverse Transcriptase II (TaKaRa). The cDNA was then amplified by PCR (denaturation: 98 °C, 10 seconds, annealing: 55 °C, 15 seconds, elongation: 68 °C, 20 seconds) using Rluc_sRNA_F01 primer (SEQ ID NO: 18), Rluc_sRNA_R01 primer (SEQ ID NO: 15), and PrimeStar GXL DNA Polymerase (TaKaRa). For the sequence analysis of the obtained cDNA, a sequencing reaction was performed by using Rluc_sRNA_F01 primer (SEQ ID NO: 18) and Big Dye Terminator v3.1 Cycle Sequence Kit, and the sequence was analyzed by Applied Biosystem 3500 Genetic Analyzer. From the chromatographic chart obtained by sequencing, an editing rate (%) was calculated from a peak height ratio (G/(G+A)) of the target site (A311)

### (D) Results of Editing Analysis

The editing rate (%) in the case of using the compounds of Examples (del03_21, del03_26, and del03_32) is shown in Fig. 10. In the figure, ADg(-) indicates the case where gRNA is not added. The compound of Example (del03_21) exhibited the same level of the editing-inducing activity by hADAR1 p110 as the compound of Reference Example 1 (del03_01) and the compound of Reference Example 2 (del03_22). Even when a modified nucleic acid was introduced as in the compounds of Examples (del03_26 and del03_32), the editing-inducing activity by hADAR1 p110 was exhibited.

### (Examples 51 to 66)

Oligonucleotide compounds of Examples 51 to 57 were obtained based on the oligonucleotide sequence of Reference Example 1 by introducing modified nucleotides as described below. Oligonucleotide compounds of Examples 58 to 63 have the first oligonucleotides (ASR) of various lengths corresponding to hGAPDH and are obtained by introducing modified nucleotides as described below. Oligonucleotide compounds of Examples 64 to 66 have the first oligonucleotides (ASR) having the sequence described below and are obtained by introducing a modified nucleotide as described below. These oligonucleotide compounds were synthesized by the phosphoramidite method as in Examples 1 to 50.

**[Table 18]**

| Example | Sequece name | Sequence (5'-3') | Molecular weight | SEQ ID NO: |
|---|---|---|---|---|
| 51 | del03_42 | C(L)^A(M)^G(L)^C(M)^A(L)^G(M)^C(L)^U(M)^C(L)^G(M)^A(L)^A(M)^C(M)^C^A( M)^A(L)^G(M)^G(M)^G(M)^G(M)^U(M)^G(M)^G(M)^18^C(M)^C(M)^A(M)^C(M)^ C(M)^U(M) | 10553.78 | - |
| 52 | del03_43 | C(L)^A(M)^G(L)^C(M)^A(L)^G(M)^C(L)^U(M)^C(L)^G(M)^A(L)^A(M)^C(M)^C^A( M)^A(M)^G(L)^G(M)^G(M)^G(M)^U(M)^G(M)^G(M)^18^C(M)^C(M)^A(M)^C(M)^ C(M)^U(M) | 10551.83 | - |
| 53 | del03_44 | C(L)^A(M)^G(L)^C(M)^A(L)^G(M)^C(L)^U(M)^C(L)^G(M)^A(L)^A(M)^C(M)^C^A( M)^A(L)^G(M)^C(L)^G(M)^G(M)^G(M)^G(M)^U(M)^G(M)^G(M)^18^C(M)^C(M)^A (M)^C(M)^C(M)^U(M) | 11271.32 | - |
| 54 | del03_45 | C(L)^A(M)^G(L)^C(M)^A(L)^G(M)^C(L)^U(M)^C(L)^G(M)^A(L)^A(M)^C(M)^C^A( M)^A(M)^G(L)^C(M)^G(L)^G(M)^G(M)^G(M)^U(M)^G(M)^G(M)^18^C(M)^C(M)^A (M)^C(M)^C(M)^U(M) | 11260.61 | - |
| 55 | det03_46 | U(F)^U(M)^C(F)^A(M)^G(F)^C(M)^A(F)^G(M)^C(F)^U(M)^C(F)^G(M)^A(F)^A(M)^ C(M)^C^A(M)^A(M)^G(M)^G(M)^C(M)^G(M)^C(M)^G(M)^C(M)^18^G(M)^C(M)^G (M)^C(M)^G(M)^C(M) | 11121.85 | - |
| 56 | del03_47 | U(F)^U(M)^C(F)^A(M)^G(F)^C(M)^A(F)^G(M)^C(F)^U(M)^C(F)^G(M)^A(F)^A(M)^ C(M)^C^A(M)^A(M)^G(M)^G(M)^C(M)^A(M)^C(M)^G(M)^C(M)^18^G(M)^C(M)^G (M)^U(M)^G(M)^C(M) | 11107.72 | - |
| 57 | del103_48 | U(F)^U(M)^C(F)^A(M)^G(F)^C(M)^A(F)^G(M)^C(F)^U(M)^C(F)^G(M)^A(F)^A(M)^ C(M)^C^A(M)^A(M)^G(M)^9^G(M)^C(M)^A(M)^C(M)^G(M)^C(M)^18^G(M)^C(M) ^G(M)^U(M)^G(M)^C(M) | 11335.5 | - |
| 58 | ASR | CCUCUUCAAGGGGUCCACAUG | - | 39 |
| | GAPDH_39 | C(F)^C(M)^U(F)^C(M)^U(F)^U(M)^C(F)^A(M)^A(F)^G(M)^G(F)^G(M)^G(F)^U(M) ^C(M)^C^A(M)^C(M)^A(M)^U(M)^G(M)^G(M)^G(M)^G(M)^U(M)^G(M)^G(M)^18^ C(M)^C(M)^A(M)^C(M)^C(M)^U(M) | 11778.2 | - |
| 59 | ASR | CCCCUCUUCAAGGGGUCCACAUG | - | 40 |
| | GAPDH_49 | C(F)^C(M)^C(F)^C(M)^U(F)^C(M)^U(F)^U(M)^C(F)^A(M)^A(F)^G(M)^G(F)^G(M)^ G(F)^U(M)^C(M)^C^A(M)^C(M)^A(M)^U(M)^G(M)^G(M)^G(M)^G(M)^U(M)^G(M) ^G(M)^18^C(M)^C(M)^A(M)^C(M)^C(M)^U(M) | 12437.59 | - |
| 60 | ASR | CUCCCCUCUUCAAGGGGUCCACAUG | - | 41 |
| | GAPDH_50 | C(F)^U(M)^C(F)^C(M)^C(F)^C(M)^U(F)^C(M)^U(F)^U(M)^C(F)^A(M)^A(F)^G(M)^ G(F)^G(M)^G(F)^U(M)^C(M)^C^A(M)^C(M)^A(M)^U(M)^G(M)^G(M)^G(M)^G(M)^ U(M)^G(M)^G(M)^18^C(M)^C(M)^A(M)^C(M)^C(M)^U(M) | 13095.07 | - |
| 61 | ASR | CCCUCCCCUCUUCAAGGGGUCCACAUG | - | 42 |
| | GAPDH_51 | C(F)^C(M)^C(F)^U(M)^C(F)^C(M)^C(F)^C(M)^U(F)^C(M)^U(F)^U(M)^C(F)^A(M)^ A(F)^G(M)^G(F)^G(M)^G(F)^U(M)^C(M)^C^A(M)^C(M)^A(M)^U(M)^G(M)^G(M)^ G(M)^G(M)^U(M)^G(M)^G(M)^18^C(M)^C(M)^A(M)^C(M)^C(M)^U(M) | 13754.36 | - |
| 62 | ASR | CCUCUUCAAGGGGUCCACA | - | 43 |
| | GAPDH_52 | C(F)^C(L)^U(F)^C(L)^U(F)^T(L)^C(F)^A(L)^A(F)^G(L)^G(F)^G(M)^G(F)^U(M)^C( M)^C^A(M)^C(M)^A(M)^G(M)^G(M)^G(M)^U(M)^G(M)^G(M)^18^C(M)^C(M)^A( M)^C(M)^C(M)^U(M) | 11094.60 | - |
| 63 | ASR | CCUCUUCAAGGGGUCCACAUG | - | 44 |
| | GAPDH_53 | C(F)^C(L)^U(F)^C(L)^U(F)^T(L)^C(F)^A(L)^A(F)^G(L)^G(F)^G(M)^G(F)^U(M)^C( M)^C^A(M)^C(M)^A(M)^U(M)^G(M)^G(M)^G(M)^G(M)^U(M)^G(M)^G(M)^18^C( M)^C(M)^A(M)^C(M)^C(M)^U(M) | 11806.00 | - |

"Molecular weight" in the table indicates an actually measured value according to negative ion ESI mass spectrometry. In the "sequence" in the table, uppercase letters denote RNA, lowercase letters denote DNA, N(M) denotes 2'-O-methylation of D-ribofuranose, N(F) denotes 2'-deoxy-2'-fluoration of D-ribofuranose, N(L) denotes 2'-O,4'-C-methylenation of D-ribofuranose, and N(E) denotes 2'-O,4'-C-ethylenation of D-ribofuranose. Linkers represented by -O(CH₂CH₂O)₃- and -O(CH₂CH₂O)₆- are denoted by "9" and "18", respectively. Nucleoside units bonded by -P(=S)(OH)- are denoted by "^". Unless otherwise specified, nucleoside units, or a nucleoside unit and a linker, are bonded by -P(=O)(OH)-. The 5'- and 3'-ends of the oligonucleotide are hydroxyl groups in which a hydrogen atom is bonded to an oxygen atom in a chemical formula.

### (Test Example 7) Evaluation of Editing Inducibility for Rluc_sRNA (2)

### (A) Annealing Reaction, In Vitro Editing Reaction, and Editing Analysis Method

The reactions and the method were performed in the same manner as in Test Example 1 or Test Example 6. In the case of hADAR2, recombinant hADAR2 was added to the 5 nM Rluc_sRNA-gRNA complex to 12.5 nM and incubated at 37 °C for 1 hour. In the case of hADAR1 p110, recombinant hADAR1 p110 was added to the 5 nM Rluc_sRNA-gRNA complex to 250 nM and incubated at 37 °C for 30 minutes.

### (B) Results of Editing Analysis

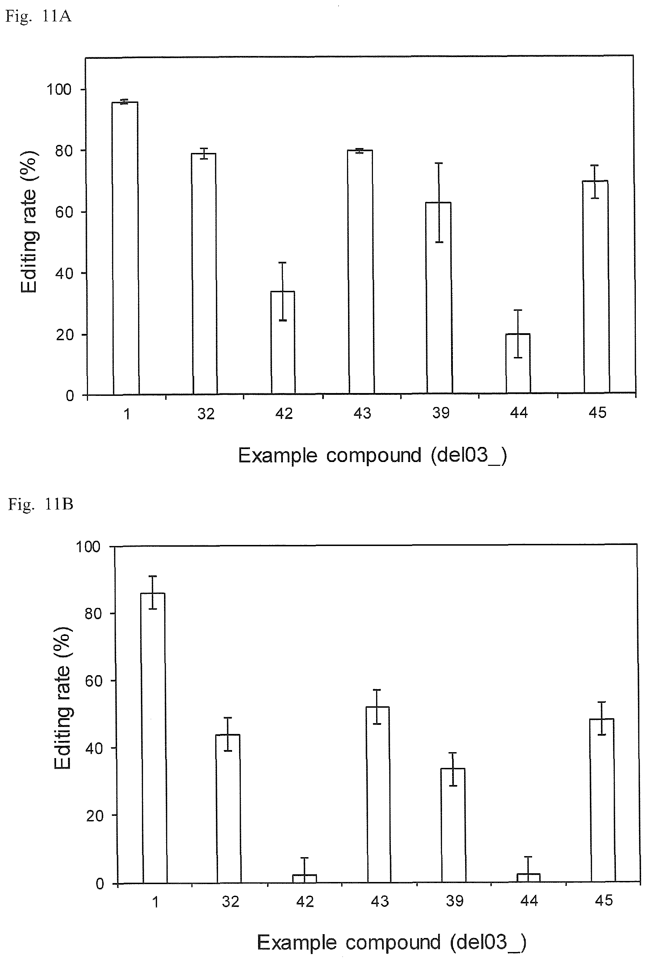

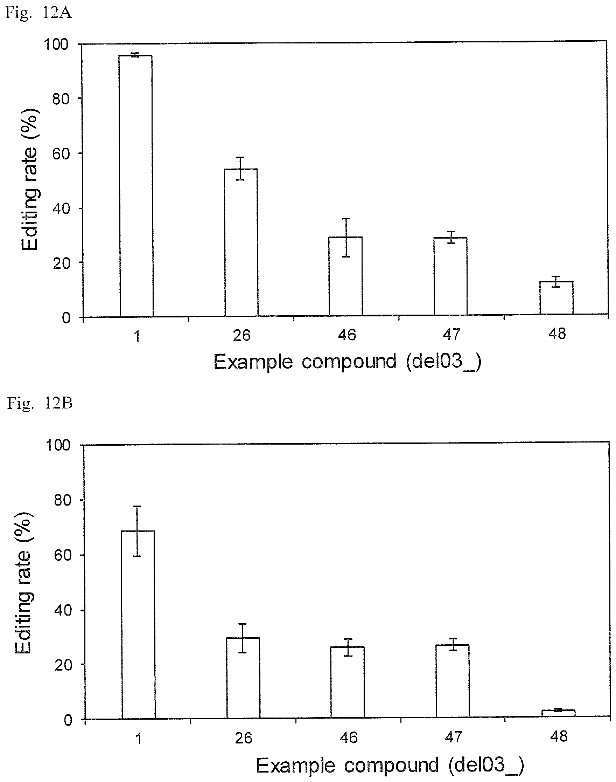

Figs. 11A and 12A show the results of hADAR2, and Figs. 11B and 12B show the results of hADAR1 p110. As shown in Fig. 11A, even when a modified nucleic acid was introduced as in the compounds of Examples (del03_32, 39, 42, 43, 44, and 45), the editing-inducing activity by ADAR2 was exhibited as in the compound of Reference Example 1. As shown in Fig. 11B, even when a modified nucleic acid was introduced as in the compounds of Examples (del03_32, 39, 43, and 45), the editing-inducing activity by ADAR1 p110 was exhibited as in the compound of Reference Example 1. On the other hand, when a modified nucleic acid was introduced as in the compounds of Examples (del03_42 and 44), the editing-inducing activity by ADAR1 p110 was significantly reduced as compared to the compound of Reference Example 1. From the above, it was clarified that the compounds of Examples (del03_42 and 44) selectively act on ADAR2.

As shown in Fig. 12A, even when a modified nucleic acid was introduced as in the compounds of Examples (del03_26 and del03_46 to del03_48), the editing-inducing activity by ADAR2 was exhibited as in the compound of Reference Example 1. As shown in Fig. 12B, even when a modified nucleic acid was introduced as in the compounds of Examples (del03_26, del03_46, and del03_47), the edit-inducing activity by ADAR1 p110 was exhibited as in the compound of Reference Example 1. On the other hand, when a modified nucleic acid was introduced as in the compound of Example (del03_48), the editing-inducing activity by ADAR1 p110 was significantly reduced as compared to the compound of Reference Example 1. From the above, it was clarified that the compound of the example (del03_48) having the linker between the first oligonucleotide and the second oligonucleotide selectively acts on ADAR2.

### (Test Example 8) Evaluation of Editing Inducibility in Cultured Cells (2) (A) Cell Culture

HeLa cells were subcultured on a 24-well plate at 5.0×10⁵ cells/well for 48 hours. Lipofectamine 3000 (Thermo) was used for transfecting 50 ng of psiCHECK2^{™}_Rluc_K41R_W104X, a plasmid expressing 350 ng of ADAR (pcDNA3.1(-) Hygro_ADAR2, pcDNA3.1(-) Hygro_ADAR1 p110, or pcDNA3.1(-) Hygro_ADAR1 p150), and 20 nM of gRNA that is a compound of Example or Reference Example. As a control, pSuper_neo was transfected and used instead of gRNA that is a compound of Example or Reference Example.

### (B) Editing Analysis Method and Luciferase Reporter Assay Method

The same methods as in Test Example 2 were performed.

### (C) Results of Editing Analysis and Luciferase Reporter Assay

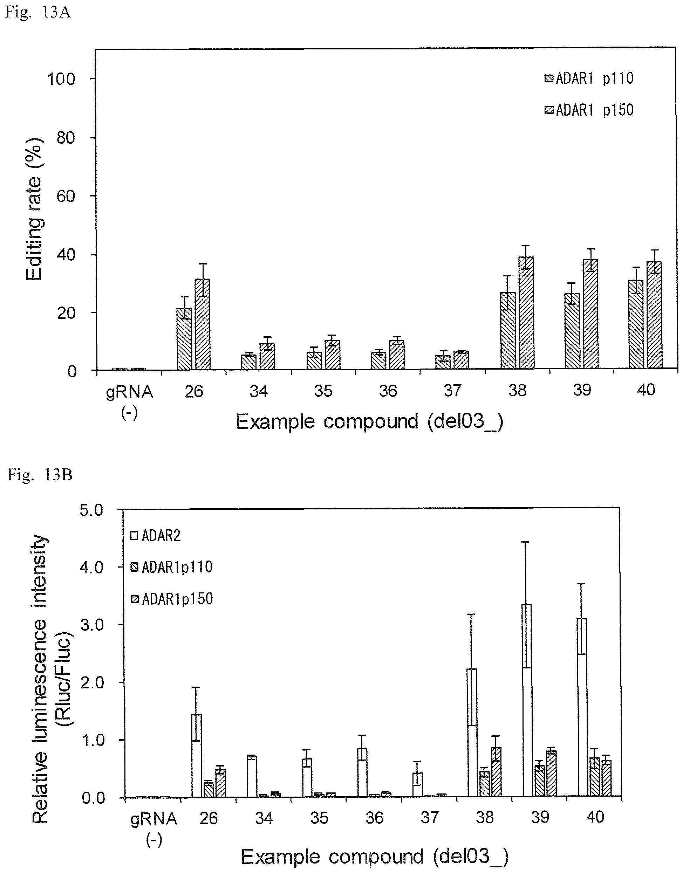

The editing rate (%) in the case of using the compounds of Examples (del03_26 and del03_34 to del03_40) is shown in Fig. 13A. When the plasmid expressing ADAR1 p110 or ADAR1 pl50 was transfected, the editing rate was about 10 % to 40 %, which was clearly higher than when only the ADAR1 p110 or ADAR1 p150 plasmid was transfected.

The results of the luciferase reporter assay in the case of using the compounds of Examples (del03_26 and del03_34 to del03_40) are shown in Fig. 13B. When the compounds of Examples (del03_26, and del03_38 to del03_40) were used, the luciferase activity recognized was clearly higher than when only the ADAR2, ADAR1 p110, or ADAR1 p150 plasmid was transfected. When the compounds of Examples (del03_34 to del03_37) were used, the luciferase activity recognized was clearly higher than when only the ADAR2 plasmid was transfected. However, the luciferase activity in the case of transfecting the ADAR1 p110 or ADAR1 p150 plasmid was very weak. From the above, it was clarified that the compounds of Examples (del03_34 to del03_37) having the linker between the first oligonucleotide and the second oligonucleotide are specific to ADAR2.

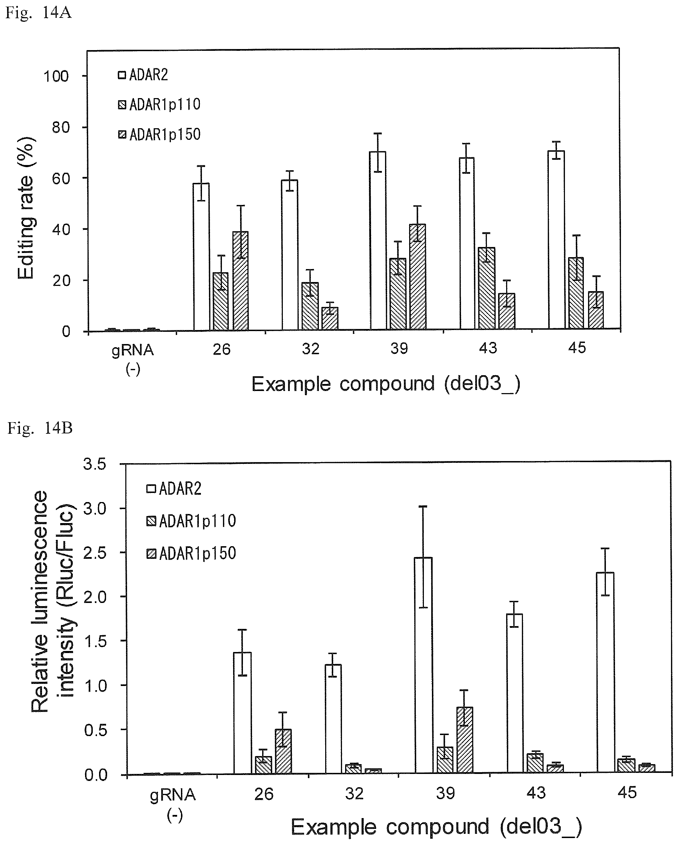

The editing rate (%) in the case of using the compounds of Examples (del03_26 and del03_32, 39, 43, and 45) is shown in Fig. 14A. When the plasmid expressing ADAR2 was transfected, the editing rate was about 60 %, which was clearly higher than when only the ADAR2 plasmid was transfected. When the plasmid expressing ADAR1 p110 or ADAR1 p150 was transfected, the editing rate was about 10 % to 40 %, which was clearly higher than when only the ADAR1 p110 or ADAR1 p150 plasmid was transfected.

The results of the luciferase reporter assay in the case of using the compounds of Examples (del03_26 and del03_32, 39, 43, and 45) are shown in Fig. 14B. When the compounds of Examples (del03_26 and de103_32, 39, 43, and 45) were used, the luciferase activity recognized was clearly higher than when only the ADAR2, ADAR1 p110, or ADAR1 p150 plasmid was transfected.

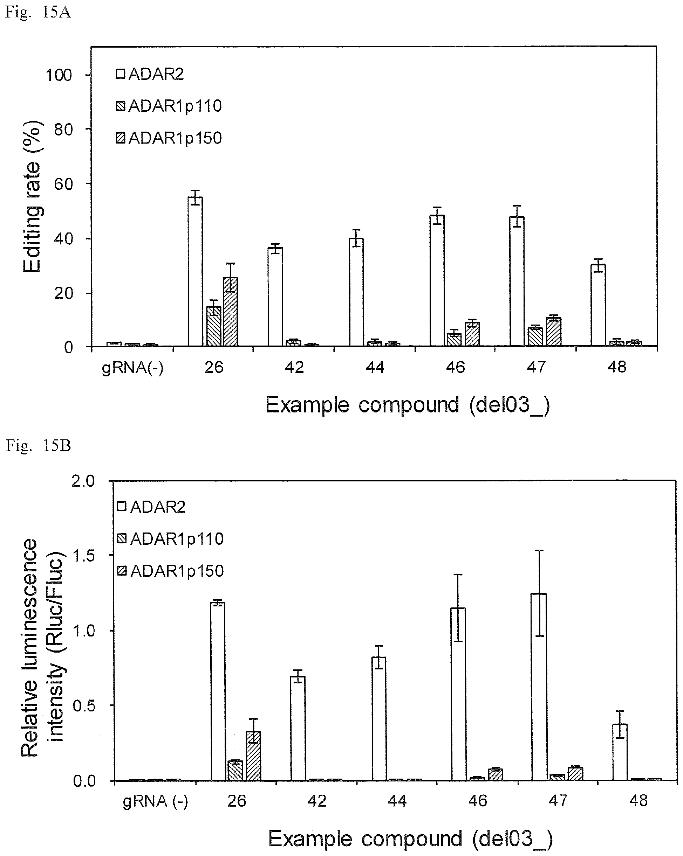

The editing rate (%) in the case of using the compounds of Examples (del03_26 and del03_42, 44, 46, 47, and 48) is shown in Fig. 15A. When the plasmid expressing ADAR2 was transfected, the editing rate was about 30 % to 60 %, which was clearly higher than when only the plasmid expressing ADAR2 was transfected. When the plasmid expressing ADAR1 p110 or ADAR1 p150 was transfected, the editing rate by using the compounds of Examples (del03_26, 46, and 47) was about 10 % to 30 %, which was clearly higher than when only the ADAR1 p110 or p150 plasmid was transfected. On the other hand, when the plasmid expressing ADAR1 p110 or ADAR1 p150 was transfected, the rate by using the compounds of Examples (del03_42, 44, and 48) was the same level as when only the ADAR1 p110 or ADAR1 p150 plasmid was transfected, and almost no editing activity was recognized.

The results of the luciferase reporter assay in the case of using the compounds of Examples (del03_26 and del03_42, 44, 46, 47, and 48) are shown in Fig. 15B. When the compounds of Examples (del03_26, and del03_46 and 47) were used, the luciferase activity recognized was clearly higher than when only the ADAR2, ADAR1 p110, or p150 plasmids were transfected. On the other hand, when the plasmid expressing ADAR1 p110 or ADAR1 p150 was transfected, the rate by using the compounds of Examples (del03_42, 44, and 48) was the same level as when only the ADAR1 p110 or ADAR1 p150 plasmid was transfected, and almost no luciferase activity was recognized. From the above, it was clear that the compounds of Examples (del03_42,44) and the compound of Example having the linker between the first oligonucleotide and the second oligonucleotide (del03_48) selectively act on ADAR2.

### (Test Example 9) Evaluation of Editing Inducibility for RNA Having GAPDH Gene Sequence (2)

### (A) Synthesis of RNA and Evaluation of Editing Inducibility

The synthesis and the evaluation were performed as in Test Example 4 except that hADAR1 p110 described in Test Example 6 was used in addition to hADAR2. In the case of hADAR2, recombinant hADAR2 was added to a 5 nM Rluc_sRNA-gRNA complex to 12.5 nM and incubated at 37 °C for 1 hour. In the case of hADAR1 p110, recombinant hADAR1 p110 was added to the 5 nM Rluc_sRNA-gRNA complex to 250 nM and incubated at 37 °C for 30 minutes.

### (B) Results of Evaluation of Editing Inducibility

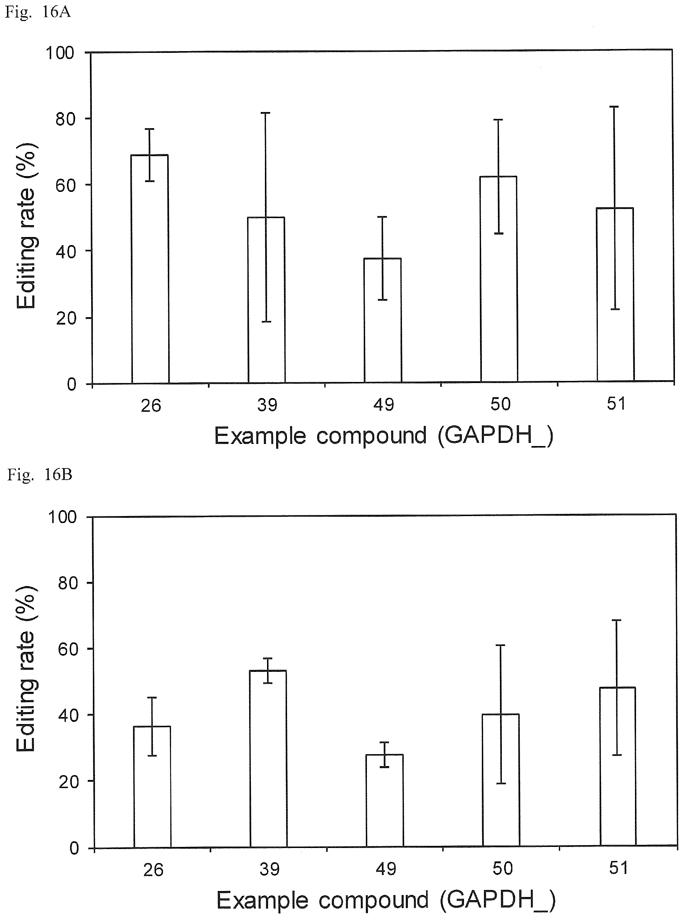

For the method of evaluating the editing inducibility of the compounds of Examples (GAPDH_26 and GAPDH_49 to 51), the same method as in (B) Evaluation of Editing Inducibility and Results described in Test Example 4 was used. The editing rate (%) in the case of using the compounds of Examples (GAPDH_26 and GAPDH_49 to 51) are shown in FIGS. 16A and 16B. As a result, the compounds of Examples (GAPDH_26 and GAPDH_49 to 51) exhibited a high editing rate when hADAR2 and hADAR1 p110 were used.

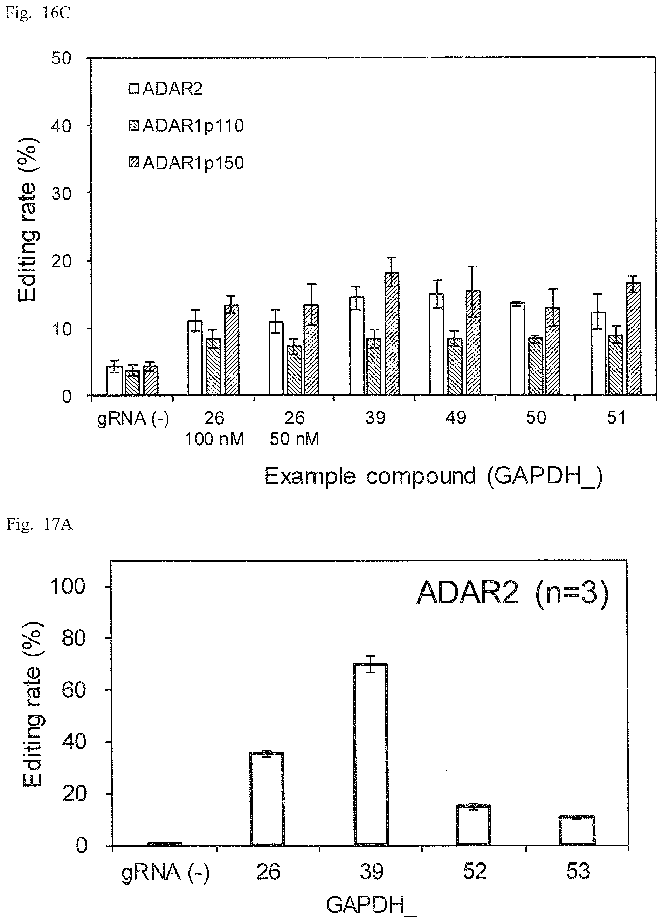

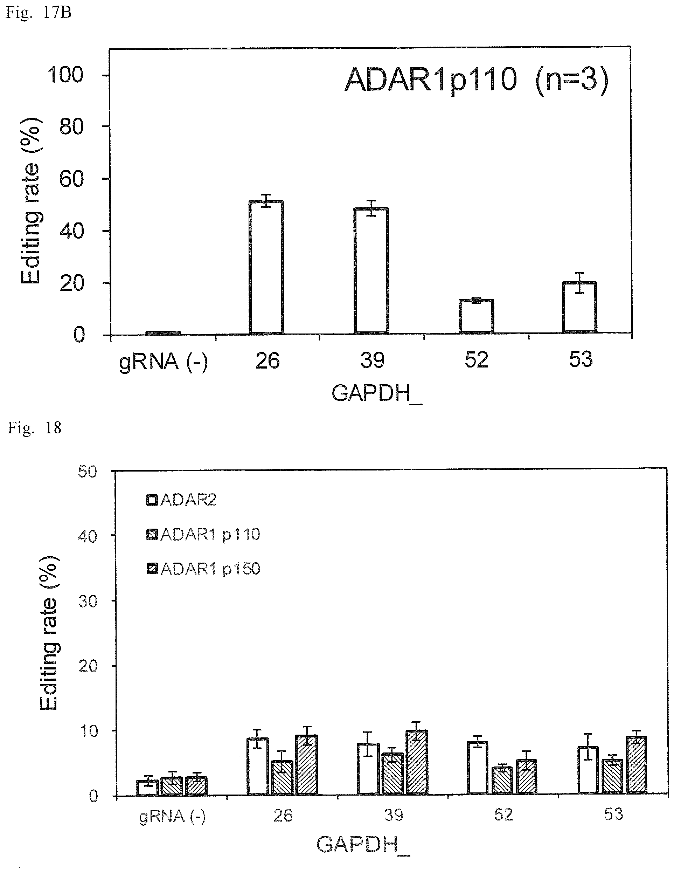

For the method of evaluating the editing inducibility of the compounds of Examples (GAPDH_26 and GAPDH_39, 52, and 53), the same method as in (B) Evaluation of Editing Inducibility and Results described in Test Example 4 was used. The editing rate (%) in the case of using the compounds of Examples (GAPDH_26 and GAPDH_39, 52, and 53) are shown in FIGS. 17A and 17B. As a result, the compounds of Examples (GAPDH_26 and GAPDH_39, 52, and 53) showed a higher editing rate when hADAR2 and hADAR1 p110 were used as compared to when gRNA was not added.

### (Test Example 10) Evaluation of Editing Inducibility for RNA Having GAPDH Gene Sequence in Cultured Cells

### (A) Cell Culture

HEK293 cells were subcultured on a 24-well plate at 5.0×10⁴ cells/well for 48 hours. Lipofectamine 3000 (Thermo) was used for transfecting a plasmid expressing 500 ng of ADAR (pcDNA3.1(-) Hygro_ADAR2, pcDNA3.1(-) Hygro_ADAR1 p110, or pcDNA3.1(-) Hygro_ADAR1 p150), and 50 nM of gRNA that is a compound of Example or Reference Example. As a control, cultured cells without transfection of gRNA that is a compound of Example or Reference Example were used.

### (B) Evaluation of Editing Inducibility and Results

For the method of evaluating the editing inducibility of the compounds of Examples (GAPDH 26 and GAPDH_49 to 51), the same method as in "(B) Evaluation of Editing Inducibility and Results" in Test Example 4 was used. The editing rate (%) in the case of using the compounds of Examples (GAPDH_26 and GAPDH49 to 51) is shown in Fig. 16C. As a result, the compounds of Examples (GAPDH_26 and GAPDH_49 to 51) exhibited a high editing rate when hADAR2, hADAR1 p11O, and hADAR1 p150 were used.

For the method of evaluating the editing inducibility of the compounds of Examples (GAPDH_26 and GAPDH_39, 52, and 53), the same method as in "(B) Evaluation of Editing Inducibility and Results" in Test Example 4 was used. The editing rate (%) in the case of using the compounds of Examples (GAPDH_26 and GAPDH_39, 52, and 53) is shown in Fig. 18. As a result, the compounds of Examples (GAPDH_26 and GAPDH_39, 52, and 53) exhibited a higher editing rate when hADAR2, hADAR1 p110, and hADAR1 p150 were used as compared to when gRNA was not added.

## Claims

1. An oligonucleotide, or a pharmaceutically acceptable salt thereof, comprising:
a first oligonucleotide identifying a target RNA;
a second oligonucleotide linked to the 3'-side of the first oligonucleotide;
a third oligonucleotide capable of forming a complementary pair with the second oligonucleotide; and
a first linking portion linking the second oligonucleotide and the third oligonucleotide, wherein
the first oligonucleotide is composed of a target-corresponding nucleotide residue corresponding to an adenosine residue in the target RNA,
an oligonucleotide of 10 to 30 residues linked to the 5'-side of the target-corresponding nucleotide residue and having a base sequence complementary to the target RNA, and
an oligonucleotide of 3 to 6 residues linked to the 3'-side of the target-corresponding nucleotide residue and having a base sequence complementary to the target RNA, wherein
the number of residues of the second oligonucleotide is 5 to 8, wherein
the number of residues of the third oligonucleotide is 5 to 8, wherein
at least one residue selected from a counter region composed of the target-corresponding nucleotide residue and two respective residues on the 3'- and 5'-sides thereof is a nucleotide residue other than a natural ribonucleotide residue, and wherein
the oligonucleotide or a pharmaceutically acceptable salt thereof induces site-specific editing for the target RNA.

2. The oligonucleotide, or a pharmaceutically acceptable salt thereof, according to claim 1, wherein the first linking portion contains at least one selected from the group consisting of an oligonucleotide of 4 or 5 residues and a polyalkyleneoxy group consisting of 1 to 8 alkyleneoxy units.

3. The oligonucleotide, or a pharmaceutically acceptable salt thereof, according to claim 1 or 2, comprising a second linking portion containing an alkyleneoxy unit between the first oligonucleotide and the second oligonucleotide.

4. The oligonucleotide, or a pharmaceutically acceptable salt thereof, according to any one of claims 1 to 3, wherein the first oligonucleotide contains a phosphorothioate bond.

5. The oligonucleotide, or a pharmaceutically acceptable salt thereof, according to any one of claims 1 to 4, wherein the first oligonucleotide contains at least one modified nucleotide residue selected from the group consisting of a 2'-O-alkylribonucleotide residue, a 2'-deoxy-2'-fluororibonucleotide residue, a bridged nucleotide residue, and a 2'-deoxyribonucleotide residue.

6. The oligonucleotide, or a pharmaceutically acceptable salt thereof, according to any one of claims 1 to 5, wherein the counter region contains a phosphorothioate bond.

7. The oligonucleotide, or a pharmaceutically acceptable salt thereof, according to any one of claims 1 to 6, wherein the target-corresponding nucleotide residue contains a phosphorothioate bond.

8. The oligonucleotide, or a pharmaceutically acceptable salt thereof, according to any one of claims 1 to 7, wherein at least one of the two respective residues on the 3'- and 5'-sides of the target-corresponding nucleotide residue is at least one modified nucleotide residue selected from the group consisting of a 2'-O-alkylribonucleotide residue and a 2'-deoxy-2'-fluororibonucleotide residue.

9. The oligonucleotide, or a pharmaceutically acceptable salt thereof, according to any one of claims 1 to 8, wherein at least one of the second oligonucleotide and the third oligonucleotide contains at least one modified nucleotide residue selected from the group consisting of a 2'-O-alkylribonucleotide residue, a 2'-deoxy-2'-fluororibonucleotide residue, and a 2'-deoxyribonucleotide residue.

10. The oligonucleotide, or a pharmaceutically acceptable salt thereof, according to any one of claims 1 to 9, wherein the oligonucleotide linked to the 5'-side of the target-corresponding nucleotide residue has a base sequence in which 2'-deoxy-2'-fluoronucleotide residues and 2'-O-alkylribonucleotide residues are alternately linked.

11. The oligonucleotide, or a pharmaceutically acceptable salt thereof, according to claim 10, wherein in the oligonucleotide linked to the 5'-side of the target-corresponding nucleotide residue, a third nucleotide residue counted in the 5'-direction from the target-corresponding nucleotide is a 2'-deoxy-2'-fluoronucleotide residue.

12. The oligonucleotide, or a pharmaceutically acceptable salt thereof, according to any one of claims 1 to 9, wherein the oligonucleotide linked to the 5'-side of the target-corresponding nucleotide residue has a base sequence in which bridged nucleotide residues and 2'-O-alkylribonucleotide residues are alternately linked.

13. The oligonucleotide, or a pharmaceutically acceptable salt thereof, according to claim 12, wherein in the oligonucleotide linked to the 5'-side of the target-corresponding nucleotide residue, a third nucleotide residue counted in the 5'-direction from the target-corresponding nucleotide is a bridged nucleotide residue.

14. The oligonucleotide, or a pharmaceutically acceptable salt thereof, according to any one of claims 1 to 9, wherein the oligonucleotide linked to the 5'-side of the target-corresponding nucleotide residue has a base sequence in which 2'-deoxy-2'-fluoronucleotide residues and bridged nucleotide residues are alternately linked.

15. The oligonucleotide, or a pharmaceutically acceptable salt thereof, according to claim 14, wherein in the oligonucleotide linked to the 5'-side of the target-corresponding nucleotide residue, a third nucleotide residue counted in the 5'-direction from the target-corresponding nucleotide is a 2'-deoxy-2'-fluoronucleotide residue.

16. The oligonucleotide, or a pharmaceutically acceptable salt thereof, according to any one of claims 1 to 15, wherein in the first oligonucleotide, the oligonucleotide linked to the 3'-side of the target-corresponding nucleotide residue has a base sequence in which the 2'-O-alkylribonucleotide residues are linked.

17. The oligonucleotide, or a pharmaceutically acceptable salt thereof, according to any one of claims 1 to 15, wherein in the first oligonucleotide, the oligonucleotide linked to the 3'-side of the target-corresponding nucleotide residue has a base sequence in which 2'-O-alkylribonucleotide residues and bridged nucleotide residues are alternately linked.

18. The oligonucleotide, or a pharmaceutically acceptable salt thereof, according to any one of claims 1 to 17, wherein in the first oligonucleotide, the oligonucleotide linked to the 3'-side of the target-corresponding nucleotide residue consists of 4 to 6 residues.

19. The oligonucleotide, or a pharmaceutically acceptable salt thereof, according to any one of claims 1 to 18, wherein the second oligonucleotide and the third oligonucleotide have a base sequence in which 2'-O-alkylribonucleotide residues are linked.

20. The oligonucleotide, or a pharmaceutically acceptable salt thereof, according to any one of claims 1 to 19, wherein each of the first oligonucleotide, the second oligonucleotide, and the third oligonucleotide has nucleotide residues linked by a phosphorothioate bond.

21. The oligonucleotide, or a pharmaceutically acceptable salt thereof, according to any one of claims 1 to 20, wherein the site-specific editing is caused by an enzyme reaction by adenosine deaminase.

22. A medicine containing the oligonucleotide, or a pharmaceutically acceptable salt thereof, according to any one of claims 1 to 21.

23. A therapeutic agent for a hereditary disease containing the oligonucleotide, or a pharmaceutically acceptable salt thereof, according to any one of claims 1 to 21.

24. A pharmaceutical composition containing the oligonucleotide, or a pharmaceutically acceptable salt thereof, according to any one of claims 1 to 21 as an active ingredient.

25. The pharmaceutical composition according to claim 24 for prevention or treatment of a hereditary disease.

26. The pharmaceutical composition according to claim 25, wherein the hereditary disease is a disease treatable by converting an adenosine residue in a target RNA into an inosine residue.

27. The pharmaceutical composition according to claim 25, wherein the hereditary disease is a hereditary disease caused by a mutation of a guanosine residue to an adenosine residue in a gene.

28. A use of the oligonucleotide, or a pharmaceutically acceptable salt thereof, according to any one of claims 1 to 21 for producing a medicine for prevention or treatment of a disease.

29. A use of the oligonucleotide, or a pharmaceutically acceptable salt thereof, according to any one of claims 1 to 21 for use in prevention or treatment of a disease.

30. A method for prevention or treatment of a disease by administering a pharmacologically effective amount of the oligonucleotide according to any one of claims 1 to 29 or a pharmacologically acceptable salt thereof to a warm-blooded animal.

31. The method according to claim 30, wherein the disease is a hereditary disease.

32. The method according to claim 31, wherein the hereditary disease is a disease treatable by converting an adenosine residue in a target RNA into an inosine residue.

33. The method according to claim 31, wherein the hereditary disease is a hereditary disease caused by a mutation of a guanosine residue to an adenosine residue in a gene.

34. The method according to any one of claims 30 to 33, wherein the warm-blooded animal is a human.
